# EUROPEAN PATENT SPECIFICATION

(11) **EP 1 727 906 B1**
(45) Date of publication and mention of the grant of the patent: **22.02.2012**
(21) Application number: 05716067.3
(22) Date of filing: 15.03.2005
(51) Int. Cl.: C12N 15/82

(54) **IMPROVED CONSTRUCTS FOR MARKER EXCISION BASED ON DUAL-FUNCTION SELECTION MARKER**
VERBESSERTES KONSTRUKT FÜR MARKER AUSSCHNEIDEN GESTÜTZT AUF EINEN DOPPELFUNKTION SELEKTIONSMARKER
CONSTRUCTS AMÉLIORÉS POUR L'EXCISION DE MARQUEUR BASÉS SUR UN MARQUEUR DE SÉLECTION AYANT UNE DOUBLE FONCTION

(30) Priority: 17.03.2004 EP 04006358
(43) Date of publication of application: 06.12.2006
(73) Proprietor: BASF Plant Science GmbH, 67056 Ludwigshafen (DE); SWETREE TECHNOLOGIES AB, 907 19 Umea (SE)
(72) Inventor: HILLEBRAND, Helke, 68159 Mannheim (DE); EBNETH, Marcus, 10965 Berlin (DE); NÄSHOLM, Torgny, S-91331 Holmsund (SE); ERIKSON, Oskar, S-91332 Holmsund (SE); HERTZBERG, Magnus, S-90339 Umea (SE)
(74) Representative: Popp, Andreas
(86) International application number: PCT/EP2005/002734
(87) International publication number: WO 2005/090581

(56) References cited:
- WO-A-03/004659
- WO-A-03/060133
- ERIKSON OSKAR ET AL: "A conditional marker gene allowing both positive and negative selection in plants." NATURE BIOTECHNOLOGY. APR 2004, vol. 22, no. 4, April 2004 (2004-04), pages 455-458, XP002292867 ISSN: 1087-0156
- DATABASE UNIPROT D-amino acid oxidase. Candida Boidinii. 1 March 2001 (2001-03-01), "DAO1" XP002292868 retrieved from EBI Database accession no. Q9HGY3
- DATABASE UNIPROT putative D-amino acid oxidase. S. coelicolor 1 November 1999 (1999-11-01), XP002292869 retrieved from EBI Database accession no. Q9X7P6
- DATABASE UNIPROT SPCC1450.07c protein. Schizosaccharomices pombe 1 November 1999 (1999-11-01), XP002292870 retrieved from EBI Database accession no. Q9Y7N4
- DATABASE UNIPROT D-amino acid oxidase. Trigonopsis variabilis 1 November 1997 (1997-11-01), XP002292871 retrieved from EBI Database accession no. OXDA_TRIVR
- DATABASE UNIPROT D-amino acid oxidase. Fusarium solani 1 March 1992 (1992-03-01), XP002292872 retrieved from EBI Database accession no. OXDA_FUSSO
- DATABASE UNIPROT Putative D-amino acid oxidase. C. elegans 1 November 1997 (1997-11-01), XP002292881 retrieved from EBI Database accession no. OXDA_CAEEL
- DATABASE UNIPROT D-amino acid oxidase. Rhodosporidium toruloides 1 November 1995 (1995-11-01), XP002292873 retrieved from EBI Database accession no. OXDA_RHOTO

## Description

### FIELD OF THE INVENTION

The invention relates to methods for eliminating maker sequences from the genome of plants, based on dual-function selection marker which - depending on the employed compound - can act as both negative and counter-selection marker.

### BACKGROUND OF THE INVENTION

An aim of plant biotechnology is the generation of plants with advantageous novel characteristics, for example for increasing agricultural productivity, improving the quality in foodstuffs or for the production of certain chemicals or pharmaceuticals (Dunwell JM (2000) J Exp Bot 51:487-96).

There are various methods described in art for inserting heterogenous DNA sequences into the genome of a host cell or organism. Because of the low insertion-frequency, it is generally required to employ selection marker to select for cells or organisms which have successfully incorporated the transgenic construct. Selectable markers enable transgenic cells or organisms (e.g., plants or plant cells) to be identified after transformation. They can be divided into positive selection marker (conferring a selective advantage), negative selection marker (compensating a selection disadvantage), and counter-selection marker (conferring a selection disadvantage), respectively.

Negative selection markers are most often employed in methods for producing transgenic cells or organisms. Such negative selection markers confer for example a resistance to a biocidal compound such as a metabolic inhibitor (*e.g.*, 2-deoxyglucose-6-phosphate, WO 98/45456), antibiotics (*e.g.*, kanamycin, G 418, bleomycin or hygromycin) or herbicides (*e.g.*, phosphinothricin or glyphosate). Examples - especially suitable for plant transformation - are:
- Phosphinothricin acetyltransferases (PAT; also named Bialophos ^{®}resistance; bar; de Block 1987; EP 0 333 033; US 4,975,374)
- 5-enolpyruvylshikimate-3-phosphate synthase (EPSPS) conferring resistance to Glyphosate^{®} (N-(phosphonomethyl)glycine) (Shah 1986)
- Glyphosate^{®} degrading enzymes (Glyphosate^{®} oxidoreductase; gox),
- Dalapon^{®} inactivating dehalogenases (deh)
- sulfonylurea- and imidazolinone-inactivating acetolactate synthases (for example mutated ALS variants with, for example, the S4 and/or Hra mutation
- Bromoxynil^{®} degrading nitrilases (bxn)
- Kanamycin- or. G418- resistance genes (NPTII; NPTI) coding e.g., for neomycin phosphotransferases (Fraley 1983)
- 2-Desoxyglucose-6-phosphate phosphatase (DOG^{R}1-Gene product; WO 98/45456; EP 0 807 836) conferring resistance against 2-desoxyglucose (Randez-Gil 1995).
- hygromycin phosphotransferase (HPT), which mediates resistance to hygromycin (Vanden Elzen 1985).
- dihydrofolate reductase (Eichholtz 1987)
- D-amino acid metabolizing enzyme (e.g., D-amino acid dehydratases or oxidases; WO 03/060133)

Additional negative selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).

Additional selection markers are those which do not result in detoxification of a biocidal compound but confer an advantage by increased or improved regeneration, growth, propagation, multiplication as the like of the cell or organism comprising such kind of "positive selection marker". Examples are isopentenyltransferase (a key enzyme of the cytokinin biosynthesis facilitating regeneration of transformed plant cells by selection on cytokinin-free medium; Ebinuma 2000a; Ebinuma 2000b). Additional positive selection markers, which confer a growth advantage to a transformed plant cells in comparison with a non-transformed one, are described *e.g.*, in EP-A 0 601 092. Growth stimulation selection markers may include (but shall not be limited to) β-Glucuronidase (in combination with *e.g.*, a cytokinin glucuronide), mannose-6-phosphate isomerase (in combination with mannose), UDP-galactose-4-epimerase (in combination with *e.g.*, galactose).

The selectable marker gene is useful during the transformation process to select for, and identify, transformed organisms, but typically provides no useful function once the transformed organism has been identified and contributes substantially to the lack of acceptance of these "gene food" products among consumers (Kuiper HA et al. (2001) Plant J. 27, 503-528), and few markers are available that are not based on these mechanisms (Hare P & Chua NH (2002) Nat. Biotechnol. 20, 575-580). Thus, there is a demand for new markers for both research and commercial crop production. Alternatively, there are multiple attempts to develop techniques by means of which marker DNA can be excised from plant genome (Ow DW and Medberry SL (1995) Crit Rev in Plant Sci 14:239-261; Gleave AP et al. (1999) Plant Mol. Biol. 40, 223-23).

The person skilled in the art is familiar with a variety of systems for the site-directed removal of recombinantly introduced nucleic acid sequences. They are mainly based on the use of sequence specific recombinases. Various sequence-specific recombination systems are described, such as the Cre/lox system of the bacteriophage P1 (Dale EC and Ow DW (1991) Proc Natl Acad Sci USA 88:10558-10562; Russell SH et al. (1992) Mol Gene Genet 234: 49-59; Osbome BI et al. (1995) Plant J. 7, 687-701), the yeast FLP/FRT system (Kilby NJ et al. (1995) Plant J 8:637-652; Lyznik LA et al. (1996) Nucleic Acids Res 24:3784-3789), the Mu phage Gin recombinase, the E. coli Pin recombinase or the R/RS system of the plasmid pSR1 (Onouchi H et al.(1995) Mol Gen Genet 247:653-660; Sugita Ket al. (2000) Plant J. 22:461-469).

Zubko et al. (Nature Biotech (April 2000) 18(4):442-445) describe a system for the deletion of nucleic acid sequences, where the sequence to be deleted is flanked by two 352 bp attP recognition sequences from the bacteriophage Lambda. Deletion of the flanked region takes place independently of the expression of helper proteins in two out of eleven transgenic tobacco lines by spontaneous intrachromosomal recombination between the attP recognition regions.

WO 02/29071 discloses a method for conditional excision of transgenic sequences from the genome of a transgenic organism. Excision occurs directly by action of an enzyme (e.g., a recombinase or a endonuclease). Self-excising constructs based on a site-specific recombinase are described in WO97/037012 and WO02/10415.

WO 03/004659 describes a recombination system based on homologous recombination between two homologous sequences induced by action of a sequence specific double-strand break inducing enzyme, preferably a meganuclease (homing-endonuclease).

Since also the marker excision efficiency is most often low, the systems are most often combined with counter-selection marker. These are sequences encoding for enzymes which are able to convert a non-toxic compound into a toxic compound. In consequence, only cells will survive treatment with said non-toxic compound which are lacking said counter-selection marker, thereby allowing for selection of cells which have successfully undergone sequence (e.g., marker) deletion. Typical counter-selection markers known in the art are for example
a) cytosine deaminases (CodA) in combination with 5-fluorocytosine (5-FC) (WO 93/01281; US 5,358,866; Gleave AP et al. (1999) Plant Mol Biol 40(2):223-35; Perera RJ et al. (1993) Plant Mol Biol.23(4):793-799; Stougaard J (1993) Plant J 3:755-761); EP-A1 595 837; Mullen CA et al. (1992) Proc Natl Acad Sci USA 89(1):33-37; Kobayashi T et al. (1995) Jpn J Genet 70(3):409-422; Schlaman HRM & Hooykaas PFF (1997) Plant J 11:1377-1385; Xiaohui Wang H et al. (2001) Gene 272(1-2): 249-255; Koprek T et al. (1999) Plant J 19(6):719-726; Gleave AP et al. (1999) Plant Mol Biol 40(2):223-235; Gallego ME (1999) Plant Mol Biol 39(1):83-93; Salomon S & Puchta H (1998) EMBO J 17(20):6086-6095; Thykjaer T et al. (1997) Plant Mol Biol 35(4):523-530; Serino G (1997) Plant J 12(3):697-701; Risseeuw E (1997) Plant J 11(4):717-728; Blanc V et al. (1996) Biochimie 78(6):511-517; Corneille S et al. (2001) Plant J 27:171-178).
b) Cytochrome P-450 enzymes in combination with the sulfonylurea pro-herbicide R7402 (2-methylethyl-2-3-dihydro-N-[(4,6-dimethoxypyrimidine-2-yl)aminocarbonyl]-1,2-benzoisothiazol-7-sulfonamid-1,1-dioxide) (O'Keefe DP et al. (1994) Plant Physiol 105:473-4.82; Tissier AF et al. (1999) Plant Cell 11:1841-1852; Koprek T et al. (1999) Plant J 19(6):719-726; O'Keefe DP (1991) Biochemistry 30(2):447-55).
c) Indoleacetic acid hydrolases like e.g., the tms2 gene product from Agrobacterium tumefaciens in combination with naphthalacetamide (NAM) (Fedoroff NV & Smith DL (1993) Plant J 3:273-289; Upadhyaya NM et al. (2000) Plant Mol Biol Rep 18:227-223; Depicker AG et al. (1988) Plant Cell rep 104:1067-1071; Karlin-Neumannn GA et al. (1991) Plant Cell 3:573-582; Sundaresan V etal. (1995) Gene Develop 9:1797-1810; Cecchini E et al. (1998) Mutat Res 401(1-2):199-206; Zubko E et al. (2000) Nat Biotechnol 18:442-445).
d) Haloalkane dehalogenases (dhlA gene product) from Xanthobacter autotropicus GJ10 in combination with 1 ,2-dichloroethane (DCE) (Naested H et al. (1999) Plant J 18(5)571-576; Janssen DB et al. (1994) Annu Rev Microbiol 48: 163-191; Janssen DB (1989) J Bacteriol 171 (12):6791-9).
e) Thymidine kinases (TK), e.g., from Type 1 Herpes Simplex virus (TK HSV-1), in combination with acyclovir, ganciclovir or 1,2-deoxy-2-fluoro-b-D-arabinofuranosil-5-iodouracile (FIAU) (Czako M & Marton L (1994) Plant Physiol 104:1067-1071; Wigler M et al. (1977) Cell 11(1):223-232; McKnight SL et al. (1980) Nucl Acids Res 8(24):5949-5964; McKnight SL et al. (1980) Nucl Acids Res 8(24):5931-5948; Preston et al. (1981) J Virol 38(2):593-605; Wagner et al. (1981) Proc Natl Acad Sci USA 78(3):1441-1445; St. Clair et al. (1987) Antimicrob Agents Chemother 31(6):844-849).

Several other counter-selection systems are known in the art (see for example international application WO 04/013333; p.13 to 20 for a summary; hereby incorporated by reference).

However, the selection systems directed to the production of marker-free cells or organisms described in the art so far requires two separate selection-marker:
1. first, a negative selection marker (e.g., conferring resistance against a herbicide or a antibiotic), which allows for selection of cells which have incorporated the transformation construct, and
2. second, a counter-selection marker (see above) which allows for selection of cells which have successfully undergone deletion/excision of the marker sequences.

WO 03/060133 is describing enzymes like the D-amino acid oxidase from Rhodotorula gracilis. The toxic effect of certain amino acids can - depending on the amino acid - be lowered or increased by metabolization by e.g., a D-amino acid oxidase.

There is so far no combined negative/counter-selection systems described in the art which are based on dual-function marker making subsequent use of both its properties as a negative selection marker and a counter-selection marker. There is furthermore an unsatisfied demand - especially in the plant biotechnology area - for fast transformation systems leading to marker-free plants. It is therefore an objective of the present invention to provide an efficient negative/counter-selection system which allows for fast generation of marker-free transgenic plant cells and plants and which is based on a single dual-function marker. This objective has been achieved by the present invention.

### BRIEF DESCRIPTION OF THE INVENTION

The invention the embodiments characterized in any one of claims 1 to 11.

### GENERAL DEFINITIONS

The teachings, methods, sequences etc. employed and described in the international patent applications WO 03/004659, WO 04/013333 and WO 03/060133 refer to gene excision and negative selection techniques.

To facilitate understanding of the invention, a number of terms are defined below. It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a" and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range; it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

"Agronomically valuable trait" include any phenotype in a plant organism that is useful or advantageous for food production or food products, including plant parts and plant products. Non-food agricultural products such as paper, etc. are also included. A partial list of agronomically valuable traits includes pest resistance, vigor, development time (time to harvest), enhanced nutrient content, novel growth patterns, flavors or colors, salt, heat, drought and cold tolerance, and the like. Preferably, agronomically valuable traits do not include selectable marker genes (e. g., genes encoding herbicide or antibiotic resistance used only to facilitate detection or selection of transformed cells), hormone biosynthesis genes leading to the production of a plant hormone (e.g., auxins, gibberllins, cytokinins, abscisic acid and ethylene that are used only for selection), or reporter genes (e.g. luciferase, glucuronidase, chloramphenicol acetyl transferase (CAT, etc.). Such agronomically valuable important traits may include improvement of pest resistance (e.g., Melchers et al. (2000) Curr Opin Plant Biol 3(2):147-52), vigor, development time (time to harvest), enhanced nutrient content, novel growth patterns, flavors or colors, salt, heat, drought, and cold tolerance (e.g., Sakamoto et al. (2000) J Exp Bot 51(342):81-8; Saijo et al. (2000) Plant J 23(3): 319-327; Yeo et al. (2000) Mol Cells 10(3):263-8; Cushman et al. (2000) Curr Opin Plant Biol 3(2):117-24), and the like. Those of skill will recognize that there are numerous polynucleotides from which to choose to confer these and other agronomically valuable traits.

As used herein, the term "amino acid sequence" refers to a list of abbreviations, letters, characters or words representing amino acid residues. Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes. The abbreviations used herein are conventional one letter codes for the amino acids: A, alanine; B, asparagine or aspartic acid; C, cysteine; D aspartic acid; E, glutamate, glutamic acid; F, phenylalanine; G, glycine; H histidine; I isoleucine; K, lysine; L, leucline; M, methionine; N, asparagine; P, proline; Q, glutamine; R, arginine ; S, serine; T, threonine; V, valine; W, tryptophan; Y, tyrosine; Z, glutamine or glutamic acid (see L. Stryer, Biochemistry, 1988, W. H. Freeman and Company, New York. The letter "x" as used herein within an amino acid sequence can stand for any amino acid residue.

The term "nucleotide sequence of interest" refers to any nucleotide sequence, the manipulation of which may be deemed desirable for any reason (*e.g.*, confer improved qualities), by one of ordinary skill in the art. Such nucleotide sequences include, but are not limited to, coding sequences of structural genes (*e.g.*, reporter genes, selection marker genes, oncogenes, drug resistance genes, growth factors, *etc.*), and non-coding regulatory sequences which do not encode an mRNA or protein product, (*e.g.*, promoter sequence, polyadenylation sequence, termination sequence, enhancer sequence, *etc*.). A nucleic acid sequence of interest may preferably encode for an agronomically valuable trait.

The term "nucleic acid" refers to deoxyribonucleotides or ribonucleotides and polymers or hybrids thereof in either single-or double-stranded, sense or antisense form. Unless otherwise indicated, a particular nucleic acid sequence also implicitly encompasses conservatively modified variants thereof (*e.g.*, degenerate codon substitutions) and complementary sequences, as well as the sequence explicitly indicated. The term "nucleic acid" is used interchangeably herein with "gene", "cDNA, "mRNA", "oligonucleotide," and "polynucleotide".

The phrase "nucleic acid sequence" refers to a single or double-stranded polymer of deoxyribonucleotide or ribonucleotide bases read from the 5'- to the 3'-end. It includes chromosomal DNA, self-replicating plasmids, infectious polymers of DNA or RNA and DNA or RNA that performs a primarily structural role. "Nucleic acid sequence" also refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acrid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid which is transcribed and translated in a sequence-specific manner to produce into a particular polypeptide or protein when placed under the control of appropriate regulatory sequences. The coding region is said to encode such a polypeptide or protein.

A "polynucleotide construct" refers to a nucleic acid at least partly created by recombinant methods. The term "DNA construct" is referring to a polynucleotide construct consisting of deoxyribonucleotides. The construct may be single- or - preferably - double stranded. The construct may be circular or linear.

The skilled worker is familiar with a variety of ways to obtain one of a DNA construct. Constructs can be prepared by means of customary recombination and cloning techniques as are described, for example, in T. Maniatis, E.F. Fritsch and J. Sambrook, Molecular Cloning: A Laboratory Manual, Cold Spring Harbor Laboratory, Cold Spring Harbor, NY (1989), in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley Interscience (1987).

The term "sense" is understood to mean a nucleic acid having a sequence which is homologous or identical to a target sequence, for example a sequence which binds to a protein transcription factor and which is involved in the expression of a given gene. According to a preferred embodiment, the nucleic acid comprises a gene of interest and elements allowing the expression of the said gene of interest.

The term "antisense" is understood to mean a nucleic acid having a sequence complementary to a target sequence, for example a messenger RNA (mRNA) sequence the blocking of whose expression is sought to be initiated by hybridization with the target sequence.

As used herein, the terms "complementary" or "complementarity" are used in reference to nucleotide sequences related by the base-pairing rules. For example, the sequence 5'-AGT-3' is complementary to the sequence 5'-ACT-3'. Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. A "complement" of a nucleic acid sequence as used herein refers to a nucleotide sequence whose nucleic acids show total complementarity to the nucleic acids of the nucleic acid sequence.

The term "genome" or "genomic DNA" is referring to the heritable genetic information of a host organism. Said genomic DNA comprises the DNA of the nucleus (also referred to as chromosomal DNA) but also the DNA of the plastids (e.g., chloroplasts) and other cellular organelles (e.g., mitochondria). Preferably the terms genome or genomic DNA is referring to the chromosomal DNA of the nucleus.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like *e.g.*, polymerase chain reaction (PCR) analysis, Southern blot analysis, fluorescence *in situ* hybridization (FISH), and *in situ* PCR.

The term "gene" refers to a coding region operably joined to appropriate regulatory sequences capable of regulating the expression of the polypeptide in some manner. A gene includes untranslated regulatory regions of DNA (*e.g.*, promoters, enhancers, repressors, *etc*.) preceding (upstream) and following (downstream) the coding region (open reading frame, ORF) as well as, where applicable, intervening sequences (*i.e.*, introns) between individual coding regions (*i.e.*, exons). The term "structural gene" as used herein is intended to mean a DNA sequence that is transcribed into mRNA which is then translated into a sequence of amino acids characteristic of a specific polypeptide.

As used herein the term "coding region" when used in reference to a structural gene refers to the nucleotide sequences which encode the amino acids found in the nascent polypeptide as a result of translation of a RNA molecule. The coding region is bounded, in eukaryotes, on the 5'-side by the nucleotide triplet "ATG" which encodes the initiator methionine and on the 3'-side by one of the three triplets which specify stop codons (*i.e.*, TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, posttranscriptional cleavage and polyadenylation.

The term "expression construct" or "expression construct" as used herein is intended to mean the combination of any nucleic acid sequence to be expressed in operable linkage with a promoter sequence and - optionally - additional elements (like *e.g.*, terminator and/or polyadenylation sequences) which facilitate expression of said nucleic acid sequence.

The term "promoter," "promoter element," or "promoter sequence" as used herein, refers to a DNA sequence which when ligated to a nucleotide sequence of interest is capable of controlling the transcription of the nucleotide sequence of interest into mRNA.

A promoter is typically, though not necessarily, located 5' (*i.e.*, upstream) of a nucleotide sequence of interest (*e.g.*, proximal to the transcriptional start site of a structural gene) whose transcription into mRNA it controls, and provides a site for specific binding by RNA polymerase and other transcription factors for initiation of transcription. A polynucleotide sequence is "heterologous to" an organism or a second polynucleotide sequence if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is not naturally associated with the promoter (e. g. a genetically engineered coding sequence or an allele from a different ecotype or variety). Suitable promoters can be derived from plants or plant pathogens like e.g., plant viruses.

Promoters may be tissue specific or cell specific. The term "tissue specific" as it applies to a promoter refers to a promoter that is capable of directing selective expression of a nucleotide sequence of interest to a specific type of tissue (*e.g.*, petals) in the relative absence of expression of the same nucleotide sequence of interest in a different type of tissue (*e.g.*, roots). Tissue specificity of a promoter may be evaluated by, for example, operably linking a reporter gene to the promoter sequence to generate a reporter construct, introducing the reporter construct into the genome of a plant such that the reporter construct is integrated into every tissue of the resulting transgenic plant, and detecting the expression of the reporter gene (*e.g.*, detecting mRNA, protein, or the activity of a protein encoded by the reporter gene) in different tissues of the transgenic plant. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, *e.g.*, GUS activity staining (as described for example in Example 7) or immunohistochemical staining. Briefly, tissue sections are embedded in paraffin, and paraffin sections are reacted with a primary antibody which is specific for the polypeptide product encoded by the nucleotide sequence of interest whose expression is controlled by the promoter. A labeled (*e.g.*, peroxidase conjugated) secondary antibody which is specific for the primary antibody is allowed to bind to the sectioned tissue, and specific binding detected (*e.g.*, with avidin/biotin) by microscopy. Promoters may be constitutive or regulatable. The term "constitutive" when made in reference to a promoter means that the promoter is capable of directing transcription of an operably linked nucleic acid sequence in the absence of a stimulus (*e.g.*, heat shock, chemicals, light, *etc.*). Typically, constitutive promoters are capable of directing expression of a transgene in substantially any cell and any tissue. In contrast, a "regulatable" promoter is one which is capable of directing a level of transcription of an operably linked nuclei acid sequence in the presence of a stimulus (*e.g.*, heat shock, chemicals, light, *etc.*) which is different from the level of transcription of the operably linked nucleic acid sequence in the absence of the stimulus.

Where expression of a gene in all tissues of a transgenic plant or other organism is desired, one can use a "constitutive" promoter, which is generally active under most environmental conditions and states of development or cell differentiation (Benfey et al. (1989) EMBO J. 8:2195-2202). The promoter controlling expression of the trait gene and/or selection marker can be constitutive. Suitable constitutive promoters for use in plants include, for example, the cauliflower mosaic virus (CaMV) 35S transcription initiation region (Franck et al. (1980) Cell 21:285-294; Odell et al.(1985) Nature 313:810-812; Shewmaker et al. (1985) Virology 140:281-288; Gardner et al. 1986, Plant Mol. Biol. 6, 221-228), the 19S transcription initiation region (US 5,352,605 and WO 84/02913), and region VI promoters, the 1'-or 2'-promoter derived from T-DNA of Agrobacterium tumefaciens, and other promoters active in plant cells that are known to those of skill in the art. Other suitable promoters include the full-length transcript promoter from Figwort mosaic virus, actin promoters, histone promoters, tubulin promoters, or the mannopine synthase promoter (MAS). Other constitutive plant promoters include various ubiquitin or polyubiquitin promoters derived from, inter alia, Arabidopsis (Sun and Callis (1997) Plant J 11(5): 1017-1027), the mas, Mac or DoubleMac promoters (US 5,106,739; Comai et al. (1990) Plant Mol Biol 15:373-381), the ubiquitin promoter (Holtorf S et al. (1995) Plant Mol Biol 29:637-649) and other transcription initiation regions from various plant genes known to those of skill in the art. Useful promoters for plants also include those obtained from Ti-or Ri-plasmids, from plant cells, plant viruses or other organisms whose promoters are found to be functional in plants. Bacterial promoters that function in plants, and thus are suitable for use in the methods of the invention include the octopine synthetase promoter, the nopaline synthase promoter, and the mannopine synthetase promoter. Suitable endogenous plant promoters include the ribulose-1,6-biphosphate (RUBP) carboxylase small subunit (ssu) promoter, the α-conglycinin promoter, the phaseolin promoter, the ADH promoter, and heat-shock promoters. Further preferred constitutive promoters are the nitrilase promoter from Arabidopsis thaliana (WO 03/008596) and the Pisum sativum ptxA promoter (e.g., as incorporated in the construct described by SEQ ID NO: 15; base pair 6479 - 7341, complementary orientation).

Of course, promoters can regulate expression all of the time in only one or some tissues. Alternatively, a promoter can regulate expression in all tissues but only at a specific developmental time point. As noted above, the excision promoter (i. e., the promoter that is linked to the sequence-specific DNA cleaving polynucleotide) is generally not constitutive, but instead is active for only part of the life cycle or at least one tissue of the transgenic organism. One can use a promoter that directs expression of a gene of interest in a specific tissue or is otherwise under more precise environmental or developmental control. Examples of environmental conditions that may affect transcription by inducible promoters include pathogen attack, anaerobic conditions, ethylene or the presence of light. Promoters under developmental control include promoters that initiate transcription only in certain tissues or organs, such as leaves, roots, fruit, seeds, or flowers, or parts thereof. The operation of a promoter may also vary depending on its location in the genome. Thus, an inducible promoter may become fully or partially constitutive in certain locations.

Examples of tissue-specific plant promoters under developmental control include promoters that initiate transcription only in certain tissues, such as fruit, seeds, flowers, anthers, ovaries, pollen, the meristem, flowers, leaves, stems, roots and seeds. The tissue-specific ES promoter from tomato is particularly useful for directing gene expression so that a desired gene product is located in fruits. See, e. g., Lincoln et al. (1988) Proc Natl Acad Sci USA 84:2793-2797; Deikman et al. (1988) EMBO J 7:3315-3320 ; Deikman et al. (1992) Plant Physiol 100:2013-2017. Other suitable seed specific promoters include those derived from the following genes: MAC1 from maize (Sheridan et al. (1996) Genetics 142:1009-1020, Cat3 from maize (GenBank No. L05934, Ableretal. (1993) Plant Mol Biol 22:10131-1038, the gene encoding oleosin 18kD from maize (GenBank No. J05212, Lee et al. (1994) Plant Mol Biol 26:1981-1987), viviparous-1 from Arabidopsis (Genbank No. U93215), the gene encoding oleosin from Arabidopsis (Genbank No. Z17657), Atmycl from Arabidopsis (Urao et al. (1996) Plant Mol Biol 32:571-576, the 2s seed storage protein gene family from Arabidopsis (Conceicao et al. (1994) Plant 5:493-505) the gene encoding oleosin 20kD from Brassica napus (GenBank No. M63985), napin from Brassica napus (GenBank No. J02798, Josefsson et al. (1987) J. Biol. Chem. 262:12196-12201), the napin gene family (e.g., from Brassica napus ; Sjodahl et al. (1995) Planta 197:264-271, US 5,608,152; Stalberg K, et al. (1996) L. Planta 199: 515-519), the gene encoding the 2S storage protein from Brassica napus (Dasgupta et al. (1993) Gene 133: 301-302), the genes encoding oleosin A (Genbank No. U09118) and oleosin B (Genbank No. U09119) from soybean, the gene encoding low molecular weight sulphur rich protein from soybean (Choi et al. (1995) Mol Gen Genet 246:266-268), the phaseolin gene (US 5,504,200, Bustos MM et al., Plant Cell. 1989;1(9):839-53), the 2S albumin gene (Joseffson LG et al.(1987) J Biol Chem 262: 12196-12201), the legumin gene (Shirsat A et al. (1989) Mol Gen Genet. 215(2):326-331), the USP (unknown seed protein) gene (Bäumlein H et al. (1991) Mol Gen Genetics 225(3):459-67), the sucrose binding protein gene (WO 00/26388), the legumin B4 gene (LeB4; Bäumlein H et al. (1991) Mol Gen Genet 225:121-128; Baeumlein et al. (1992) Plant J 2(2):233-239; Fiedler U et al. (1995) Biotechnology (NY) 13(10):1090-1093), the Ins Arabidopsis oleosin gene (WO9845461), the Brassica Bce4 gene (WO 91/13980), genes encoding the "high-molecular-weight glutenin" (HMWG), gliadin, branching enzyme, ADP-glucose pyrophosphatase (AGPase) or starch synthase. Furthermore preferred promoters are those which enable seed-specific expression in monocots such as maize, barley, wheat, rye, rice and the like. Promoters which may advantageously be employed are the promoter of the Ipt2 or Ipt1 gene (WO 95/15389, WO 95/23230) or the promoters described in WO 99/16890 (promoters of the hordein gene, the glutelin gene, the oryzin gene, the prolamine gene, the gliadin gene, the zein gene, the kasirin gene or the secalin gene).

Further suitable promoters are, for example, specific promoters for tubers, storage roots or roots such as, for example, the class I patatin promoter (B33), the potato cathepsin D inhibitor promoter, the starch synthase (GBSS1) promoter or the sporamin promoter, and fruit-specific promoters such as, for example, the tomato fruit-specific promoter(EP-A 409 625).

Promoters which are furthermore suitable are those which ensure leaf-specific expression. Promoters which may be mentioned are the potato cytosolic FBPase promoter (WO 98/18940), the Rubisco (ribulose-1,5-bisphosphate carboxylase) SSU (small subunit) promoter or the potato ST-LSI promoter (Stockhaus et al. (1989) EMBO J 8(9):2445-2451). Other preferred promoters are those which govern expression in seeds and plant embryos.

Further suitable promoters are, for example, fruit-maturation-specific promoters such as, for example, the tomato fruit-maturation-specific promoter (WO 94/21794), flower-specific promoters such as, for example, the phytoene synthase promoter (WO 92/16635) or the promoter of the P-rr gene (WO 98/22593) or another node-specific promoter as described in EP-A 249676 may be used advantageously. The promoter may also be a pith-specific promoter, such as the promoter isolated from a plant TrpA gene as described in WO 93/07278. A development-regulated promoter is, inter alia, described by Baerson et al. (Baerson SR, Lamppa GK (1993) Plant Mol Biol 22(2):255-67).

Other preferred promoters are promoters induced by biotic or abiotic stress, such as, for example, the pathogen-inducible promoter of the PRP1 gene (Ward et al., Plant Mol Biol 1993, 22: 361-366), the tomato heat-inducible hsp80 promoter (US 5,187,267), the potato chill-inducible alpha-amylase promoter (WO 96/12814) or the wound-induced pinII promoter (EP375091).

Promoters may also encompass further promoters, promoter elements or minimal promoters capable of modifying the expression-specific characteristics. Thus, for example, the tissue-specific expression may take place in addition as a function of certain stress factors, owing to genetic control sequences. Such elements are, for example, described for water stress, abscisic acid (Lam E and Chua NH (1991) J Biol Chem 266(26):17131 -17135) and heat stress (Schoffl F et al. (1989) Molecular & General Genetics 217(2-3):246-53).

The term "operable linkage" or "operably linked" is to be understood as meaning, for examples, the sequential arrangement of a regulatory element (*e.g.* a promoter) with a nucleic acid sequence to be expressed and, if appropriate, further regulatory elements (such as *e.g.*, a terminator) in such a way that each of the regulatory elements can fulfill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. Operable linkage, and an expression construct, can be generated by means of customary recombination and cloning techniques as described (*e.g.*, in Maniatis 1989; Silhavy 1984; Ausubel 1987; Gelvin 1990). However, further sequences which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of promoter and nucleic acid sequence to be expressed, can exist in a vector-integrated form and be inserted into a plant genome, for example by transformation.

The terms "polypeptide", "peptide", "oligopeptide", "polypeptide", "gene product", "expression product" and "protein" are used interchangeably herein to refer to a polymer or oligomer of consecutive amino acid residues.

Preferably, the term "isolated" when used in relation to a nucleic acid, as in "an isolated nucleic acid sequence" refers to a nucleic acid sequence that is identified and separated from at least one contaminant nucleic acid with which it is ordinarily associated in its natural source. Isolated nucleic acid is nucleic acid present in a form or setting that is different from that in which it is found in nature. In contrast, non-isolated nucleic acids are nucleic acids such as DNA and RNA which are found in the state they exist in nature. For example, a given DNA sequence (*e.g.*, a gene) is found on the host cell chromosome in proximity to neighboring genes; RNA sequences, such as a specific mRNA sequence encoding a specific protein, are found in the cell as a mixture with numerous other mRNAs which encode a multitude of proteins. However, an isolated nucleic acid sequence comprising SEQ ID NO:1 includes, by way of example, such nucleic acid sequences in cells which ordinarily contain SEQ ID NO:1 where the nucleic acid sequence is in a chromosomal or extrachromosomal location different from that of natural cells, or is otherwise flanked by a different nucleic acid sequence than that found in nature. The isolated nucleic acid sequence may be present in single-stranded or double-stranded form. When an isolated nucleic acid sequence is to be utilized to express a protein, the nucleic acid sequence will contain at a minimum at least a portion of the sense or coding strand (i.e., the nucleic acid sequence may be single-stranded). Alternatively, it may contain both the sense and anti-sense strands (*i.e.*, the nucleic acid sequence may be double-stranded).

As used herein, the term "purified" refers to molecules, either nucleic or amino acid sequences, that are removed from their natural environment, isolated or separated. An "isolated nucleic acid sequence" is therefore a purified nucleic acid sequence. "Substantially purified" molecules are at least 60% free, preferably at least 75% free, and more preferably at least 90% free from other components with which they are naturally associated.

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

"Transgene", "transgenic" or "recombinant" refers to an polynucleotide manipulated by man or a copy or complement of a polynucleotide manipulated by man. For instance, a transgenic expression cassette comprising a promoter operably linked to a second polynucleotide may include a promoter that is heterologous to the second polynucleotide as the result of manipulation by man (e.g., by methods described in Sambrook et al., Molecular Cloning-A Laboratory Manual, Cold Spring Harbor Laboratory, Cold Spring Harbor, New York, (1989) or Current Protocols in Molecular Biology Volumes 1-3, John Wiley & Sons, Inc. (1994-1998)) of an isolated nucleic acid comprising the expression cassette. In another example, a recombinant expression cassette may comprise polynucleotides combined in such a way that the polynucleotides are extremely unlikely to be found in nature. For instance, restriction sites or plasmid vector sequences manipulated by man may flank or separate the promoter from the second polynucleotide. One of skill will recognize that polynucleotides can be manipulated in many ways and are not limited to the examples above.

The term "transgenic" or "recombinant" when used in reference to a cell refers to a cell which contains a transgene, or whose genome has been altered by the introduction of a transgene. The term "transgenic" when used in reference to a tissue or to a plant refers to a tissue or plant, respectively, which comprises one or more cells that contain a transgene, or whose genome has been altered by the introduction of a transgene. Transgenic cells, tissues and plants may be produced by several methods including the introduction of a "transgene" comprising nucleic acid (usually DNA) into a target cell or integration of the transgene into a chromosome of a target cell by way of human intervention, such as by the methods described herein.

The term "transgene" as used herein refers to any nucleic acid sequence which is introduced into the genome of a cell by experimental manipulations. A transgene may be an "endogenous DNA sequence," or a "heterologous DNA sequence" (*i.e.*, "foreign DNA"). The term "endogenous DNA sequence" refers to a nucleotide sequence which is naturally found in the cell into which it is introduced so long as it does not contain some modification (*e.g.*, a point mutation, the presence of a selectable marker gene, etc.) relative to the naturally-occurring sequence. The term "heterologous DNA sequence" refers to a nucleotide sequence which is ligated to, or is manipulated to become ligated to, a nucleic acid sequence to which it is not ligated in nature, or to which it is ligated at a different location in nature. Heterologous DNA is not endogenous to the cell into which it is introduced, but has been obtained from another cell. Heterologous DNA also includes an endogenous DNA sequence which contains some modification. Generally, although not necessarily, heterologous DNA encodes RNA and proteins that are not normally produced by the cell into which it is expressed. Examples of heterologous DNA include reporter genes, transcriptional and translational regulatory sequences, selectable marker proteins (*e.g.*, proteins which confer drug resistance), etc. Preferably, the term "transgenic" or "recombinant" with respect to a regulatory sequence (*e.g.*, a promoter of the invention) means that said regulatory sequence is covalently joined and adjacent to a nucleic acid to which it is not adjacent in its natural environment.

The term "foreign gene" refers to any nucleic acid (*e.g.*, gene sequence) which is introduced into the genome of a cell by experimental manipulations and may include gene sequences found in that cell so long as the introduced gene contains some modification (*e.g.*, a point mutation, the presence of a selectable marker gene, *etc.*) relative to the naturally-occurring gene.

Preferably, the term "transgene" or "transgenic" with respect to, for example, a nucleic acid sequence (or an organism, expression construct or vector comprising said nucleic acid sequence) refers to all those constructs originating by experimental manipulations in which either
a) said nucleic acid sequence, or
b) a genetic control sequence linked operably to said nucleic acid sequence a), for example a promoter, or
c) (a) and (b)
is not located in its natural genetic environment or has been modified by experimental manipulation, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the nucleic acid sequence is preferably retained, at least in part. The environment flanks the nucleic acid sequence at least at one side and has a sequence of at least 50 bp, preferably at least 500 bp, especially preferably at least 1000 bp, very especially preferably at least 5000 bp, in length. A naturally occurring expression construct - for example the naturally occurring combination of a promoter with the corresponding gene - becomes a transgenic expression construct when it is modified by non-natural, synthetic "artificial" methods such as, for example, mutagenization. Such methods have been described (US 5,565,350; WO 00/15815).

"Recombinant" polypeptides or proteins refer to polypeptides or proteins produced by recombinant DNA techniques, *i.e.*, produced from cells transformed by an exogenous recombinant DNA construct encoding the desired polypeptide or protein. Recombinant nucleic acids and polypeptide may also comprise molecules which as such does not exist in nature but are modified, changed, mutated or otherwise manipulated by man.

The term "genetically-modified organism" or "GMO" refers to any organism that comprises transgene DNA. Exemplary organisms include plants, animals and microorganisms.

The terms "heterologous nucleic acid sequence" or "heterologous DNA" are used interchangeably to refer to a nucleotide sequence which is ligated to a nucleic acid sequence to which it is not ligated in nature, or to which it is ligated at a different location in nature. Heterologous DNA is not endogenous to the cell into which it is introduced, but has been obtained from another cell. Generally, although not necessarily, such heterologous DNA encodes RNA and proteins that are not normally produced by the cell into which it is expressed.

The term "cell" or "plant cell" as used herein refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronize or not synchronized. A plant cell within the meaning of this invention may be isolated (e.g., in suspension culture) or comprised in a plant tissue, plant organ or plant at any developmental stage.

The term "organ" with respect to a plant (or "plant organ") means parts of a plant and may include (but shall not limited to) for example roots, fruits, shoots, stem, leaves, anthers, sepals, petals, pollen, seeds, *etc.*

The term "tissue" with respect to a plant (or "plant tissue") means arrangement of multiple plant cells including differentiated and undifferentiated tissues of plants. Plant tissues may constitute part of a plant organ (*e.g.*, the epidermis of a plant leaf) but may also constitute tumor tissues (e.g., callus tissue) and various types of cells in culture (*e.g.*, single cells, protoplasts, embryos, calli, protocorm-like bodies, *etc.*). Plant tissue may be *in planta*, in organ culture, tissue culture, or cell culture.

The term "plant" as used herein refers to a plurality of plant cells which are largely differentiated into a structure that is present at any stage of a plant's development. Such structures include one or more plant organs including, but are not limited to, fruit, shoot, stem, leaf, flower petal, *etc.*

The term "expression" refers to the biosynthesis of a gene product. For example, in the case of a structural gene, expression involves transcription of the structural gene into mRNA and - optionally - the subsequent translation of mRNA into one or more polypeptides.

The term "transformation" as used herein refers to the introduction of genetic material (*e.g.*, a transgene) into a cell. Transformation of a cell may be stable or transient. The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA) which detects the presence of a polypeptide encoded by one or more of the transgenes. Alternatively, transient transformation may be detected by detecting the activity of the protein (*e.g.*, β-glucuronidase) encoded by the transgene (*e.g.*, the uid A gene) as demonstrated herein [*e.g.*, histochemical assay of GUS enzyme activity by staining with X-gluc which gives a blue precipitate in the presence of the GUS enzyme; and a chemiluminescent assay of GUS enzyme activity using the GUS-Light kit (Tropix)]. The term "transient transformant" refers to a cell which has transiently incorporated one or more transgenes. In contrast, the term "stable transformation" or "stably transformed" refers to the introduction and integration of one or more transgenes into the genome of a cell, preferably resulting in chromosomal integration and stable heritability through meiosis. Stable transformation of a cell may be detected by Southern blot hybridization of genomic DNA of the cell with nucleic acid sequences which are capable of binding to one or more of the transgenes. Alternatively, stable transformation of a cell may also be detected by the polymerase chain reaction of genomic DNA of the cell to amplify transgene sequences. The term "stable transformant" refers to a cell which has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene. Transformation also includes introduction of genetic material into plant cells in the form of plant viral vectors involving epichromosomal replication and gene expression which may exhibit variable properties with respect to meiotic stability.

The terms "infecting" and "infection" with a bacterium refer to co-incubation of a target biological sample, (*e.g.*, cell, tissue, *etc.*) with the bacterium under conditions such that nucleic acid sequences contained within the bacterium are introduced into one or more cells of the target biological sample.

The term "*Agrobacterium*" refers to a soil-borne, Gram-negative, rod-shaped phytopathogenic bacterium which causes crown gall. The term "*Agrobacterium*" includes, but is not limited to, the strains *Agrobacterium tumefaciens,* (which typically causes crown gall in infected plants), and *Agrobacterium rhizogenes* (which causes hairy root disease in infected host plants). Infection of a plant cell with *Agrobacterium* generally results in the production of opines (*e.g.*, nopaline, agropine, octopine *etc.*) by the infected cell. Thus, *Agrobacterium* strains which cause production of nopaline (*e.g.*, strain LBA4301, C58, A208) are referred to as "nopaline-type" *Agrobacteria; Agrobacterium* strains which cause production of octopine (*e.g.*, strain LBA4404, Ach5, B6) are referred to as "octopine-type" *Agrobacteria;* and *Agrobacterium* strains which cause production of agropine (*e.g.*, strain EHA105, EHA101, A281) are referred to as "agropine-type" *Agrobacteria.*

The terms "bombarding, "bombardment," and "biolistic bombardment" refer to the process of accelerating particles towards a target biological sample (*e.g.*, cell, tissue, *etc*.) to effect wounding of the cell membrane of a cell in the target biological sample and/or entry of the particles into the target biological sample. Methods for biolistic bombardment are known in the art (*e.g.*, US 5,584,807, the contents of which are herein incorporated by reference), and are commercially available (*e.g.*, the helium gas-driven microprojectile accelerator (PDS-1000/He) (BioRad).

The term "microwounding" when made in reference to plant tissue refers to the introduction of microscopic wounds in that tissue. Microwounding may be achieved by, for example, particle bombardment as described herein.

The terms "homology" or "identity" when used in relation to nucleic acids refers to a degree of complementarity. Homology or identity between two nucleic acids is understood as meaning the identity of the nucleic acid sequence over in each case the entire length of the sequence, which is calculated by comparison with the aid of the program algorithm GAP (Wisconsin Package Version 10.0, University of Wisconsin, Genetics Computer Group (GCG), Madison, USA) with the parameters being set as follows:

| | |
|---|---|
| Gap Weight: 12 | Length Weight: 4 |
| Average Match: 2,912 | Average Mismatch:-2,003 |

For example, a sequence with at least 95% homology (or identity) to the sequence SEQ ID NO. 1 at the nucleic acid level is understood as meaning the sequence which, upon comparison with the sequence SEQ ID NO. 1 by the above program algorithm with the above parameter set, has at least 95% homology. There may be partial homology (*i.e.*, partial identity of less then 100%) or complete homology (*i.e.*, complete identity of 100%).

Alternatively, a partially complementary sequence is understood to be one that at least partially inhibits a completely complementary sequence from hybridizing to a target nucleic acid and is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe (*i.e.*, an oligonucleotide which is capable of hybridizing to another oligonucleotide of interest) will compete for and inhibit the binding (*i.e.*, the hybridization) of a completely homologous sequence to a target under conditions of low stringency. This is not to say that conditions of low stringency are such that non-specific binding is permitted; low stringency conditions require that the binding of two sequences to one another be a specific (*i.e.*, selective) interaction. The absence of non-specific binding may be tested by the use of a second target which lacks even a partial degree of complementarity (*e.g.*, less than about 30% identity); in the absence of non-specific binding the probe will not hybridize to the second non-complementary target.

When used in reference to a double-stranded nucleic acid sequence such as a cDNA or genomic clone, the term "substantially homologous" refers to any probe which can hybridize to either or both strands of the double-stranded nucleic acid sequence under conditions of low stringency as described infra. When used in reference to a single-stranded nucleic acid sequence, the term "substantially homologous" refers to any probe which can hybridize to the single-stranded nucleic acid sequence under conditions of low stringency as described infra.

The term "hybridization" as used herein includes "any process by which a strand of nucleic acid joins with a complementary strand through base pairing." (Coombs 1994). Hybridization and the strength of hybridization (*i.e.*, the strength of the association between the nucleic acids) is impacted by such factors as the degree of complementarity between the nucleic acids, stringency of the conditions involved, the Tm of the formed hybrid, and the G:C ratio within the nucleic acids.

As used herein, the term "Tm" is used in reference to the "melting temperature." The melting temperature is the temperature at which a population of double-stranded nucleic acid molecules becomes half dissociated into single strands. The equation for calculating the Tm of nucleic acids is well known in the art. As indicated by standard references, a simple estimate of the Tm value may be calculated by the equation: Tm=81.5+0.41 (% G+C), when a nucleic acid is in aqueous solution at 1 M NaCl [see e.g., Anderson and Young, Quantitative Filter Hybridization, in Nucleic Acid Hybridization (1985)]. Other references include more sophisticated computations which take structural as well as sequence characteristics into account for the calculation of Tm.

Low stringency conditions when used in reference to nucleic acid hybridization comprise conditions equivalent to binding or hybridization at 68°C. in a solution consisting of 5x SSPE (43.8 g/L NaCl, 6.9 g/L NaH₂PO₄.H₂O and 1.85 g/L EDTA, pH adjusted to 7.4 with NaOH), 1% SDS, 5x Denhardt's reagent [50x Denhardt's contains the following per 500 mL: 5 g Ficoll (Type 400, Pharmacia), 5 g BSA (Fraction V; Sigma)] and 100 µg/mL denatured salmon sperm DNA followed by washing in a solution comprising 0.2x SSPE, and 0.1% SDS at room temperature when a DNA probe of about 100 to about 1000 nucleotides in length is employed.

High stringency conditions when used in reference to nucleic acid hybridization comprise conditions equivalent to binding or hybridization at 68° C. in a solution consisting of 5x SSPE, 1% SDS, 5x Denhardt's reagent and 100 µg/mL denatured salmon sperm DNA followed by washing in a solution comprising 0.1x SSPE, and 0.1% SDS at 68° C. when a probe of about 100 to about 1000 nucleotides in length is employed.

The term "equivalent" when made in reference to a hybridization condition as it relates to a hybridization condition of interest means that the hybridization condition and the hybridization condition of interest result in hybridization of nucleic acid sequences which have the same range of percent (%) homology. For example, if a hybridization condition of interest results in hybridization of a first nucleic acid sequence with other nucleic acid sequences that have from 80% to 90% homology to the first nucleic acid sequence, then another hybridization condition is said to be equivalent to the hybridization condition of interest if this other hybridization condition also results in hybridization of the first nucleic acid sequence with the other nucleic acid sequences that have from 80% to 90% homology to the first nucleic acid sequence.

When used in reference to nucleic acid hybridization the art knows well that numerous equivalent conditions may be employed to comprise either low or high stringency conditions; factors such as the length and nature (DNA, RNA, base composition) of the probe and nature of the target (DNA, RNA, base composition, present in solution or immobilized, etc.) and the concentration of the salts and other components (e.g., the presence or absence of formamide, dextran sulfate, polyethylene glycol) are considered and the hybridization solution may be varied to generate conditions of either low or high stringency hybridization different from, but equivalent to, the above-listed conditions. Those skilled in the art know that whereas higher stringencies may be preferred to reduce or eliminate non-specific binding, lower stringencies may be preferred to detect a larger number of nucleic acid sequences having different homologies.

### DETAILED DESCRIPTION OF THE INVENTION

Accordingly, a first embodiment of the invention relates to a method for producing a transgenic plant comprising:
i) transforming a plant cell with a first expression cassette comprising a nucleic acid sequence encoding a D-amino acid oxidase operably linked with a promoter allowing expression in plant cells or plants, in combination with at least one second expression cassette suitable for conferring to said plant an agronomically valuable trait, and
ii) providing at least one first compound X, wherein compound X is either D-clorine or D-serine, which is phytotoxic against plant cells not functionally expressing said D-amino acid oxidase, wherein said compound X can be metabolized by said D-amino acid oxidase into one or more compound(s) Y which are non-phytotoxic or less phytotoxic than compound X, and
iii) treating plants in coil comprising transformed plant cells of step i) with said first compound X in a phyto-for three consecutive days, wherein if compound X is D-alanine the concentration is 5 to 100 mM, and if compound X is D-serine the concentration is 5 to 80 mM; or treating seeds comprising transformed plant cells of step i) with said first compound X in a phytotoxic concentration for five days during germination on media, wherein if compound X is D-alanine the concentration is 0,1 mM to 100 mM and if compound X is D-serine the concentration is 0,1 to 10 mM
   toxic concentration and selecting plant cells comprising in their genome both said first and said second expression cassette, wherein said first expression cassette is conferring resistance to said transformed plant cells against said compound X by expression of said D-amino acid oxidase, and
iv) providing at least one second compound M, which is non-phytotoxic or moderately phytotoxic against plant cells not functionally expressing said D-amino acid oxidase, wherein said compound M can be metabolized by said D-amino acid oxidase into one or more compound(s) N which are phytotoxic or more phytotoxic than compound M, and
v) separation of said first expression cassette and said second expression cassette and treating resulting said plant cells with said second compound M in a concentration toxic to plant cells still comprising said first expression cassette, and selecting plant cells comprising said second expression cassette but lacking said first expression cassette,
whereby M is comprising a structure selected from the groups consisting of D-valine, D-leucine, D-lysine, and D-proline, or D-isoleucine applied in the cell culture medium in concentrations of about 10 mM to about 30 mM or D-asparagine or D-glutamine applied via the cell culture medium in concentrations of about 3 mM to about 20 mM.

The term "combination" or "combined" with respect to the relation between the first and the second expression cassette is to be understood in the broad sense and is intended to mean any mode operation which is linking the functionality of the two expression cassettes. The first and the second expression cassette may be comprised in one DNA construct but may also be separate molecules.

Correspondingly the term "breaking the combination" is to be understood in the broad sense to comprise any method which leads to separation of the two expression cassettes. The person skilled in the art is aware of various means to separate sequences comprised in a genome, including but not limited to segregation, sequence excision or deletion etc.

The plant cell to be transformed can be an isolated cell but also part of a plant tissue, culture, organ or an entire plant at any developmental stage. Furthermore, the plant cell placed under negative selection or counter selection can be isolated cells but also part of a plant tissue, culture, organ or an entire plant at any developmental stage. Between the described steps of the method of the invention additional steps may be comprised. For example, between negative selection and counter selection there might be additional steps of e.g., induction of excision and/or plant regeneration.

As mentioned above, the first and the second expression cassette may not be combined on one DNA construct but may be employed in combination in form of - for example - a co-transformation approach wherein the two separate molecules are transformed together into the plant cells. In a scenario like this the combination of the first and the second expression cassette can be broken e.g by segregation (for example following reproduction of resulting plantlets). In this scenario the multiplicity of resulting segregated transgenic plantlets can be easily screened for lack of the first expression cassette by employment of compound M, which can be applied, e.g., by spraying.

However, the first and the second expression cassette may be combined on one DNA construct. Here the combination can be broken for example by means of sequence specific sequence deletion or excision e.g., by employing a sequence-specific recombinase or by induced sequence specific homologous recombination.

Accordingly, a second embodiment of the invention relates to the method for producing a transgenic plant described above comprising:
i) transforming a plant cell with a first DNA construct comprising
   a) a first expression cassette comprising a nucleic acid sequence encoding a D-amino acid oxidase operably linked with a promoter allowing expression in plant cells or plants, wherein said first expression cassette is flanked by sequences which allow for specific deletion of said first expression cassette, and
   b) at least one second expression cassette suitable for conferring to said plant an agronomically valuable trait, wherein said second expression cassette is not localized between said sequences which allow for specific deletion of said first expression cassette, and
ii) providing at least one first compound X, which is phytotoxic against plant cells not functionally expressing said D-amino acid oxidase, wherein said compound X can be metabolized by said D-amino acid oxidase into one or more compound(s) Y which are non-phytotoxic or less phytotoxic than compound X, and
iii) treating said transformed plant cells of step i) with said first compound X in a phytotoxic concentration and selecting plant cells comprising in their genome said first DNA construct, conferring resistance to said transformed plant cells against said compound X by expression of said D-amino acid oxidase, and
iv) providing at least one second compound M, which is non-phytotoxic or moderately phytotoxic against plant cells not functionally expressing said D-amino acid oxidase, wherein said compound M can be metabolized by said D-amino acid oxidase into one or more compound(s) N which are phytotoxic or more phytotoxic than compound M, and
v) inducing deletion of said first expression cassette from the genome of said transformed plant cells and treating said plant cells with said second compound M in a concentration toxic to plant cells still comprising said first expression cassette, thereby selecting plant cells comprising said second expression cassette but lacking said first expression cassette.

In a preferred embodiment the method of the invention further comprises the step of regeneration of a fertile plant. The method may further include sexually or asexually propagating or growing off-spring or a descendant of the plant regenerated from said plant cell.

This invention discloses the subsequent use of a the marker gene *dao1* encoding a D-amino acid oxidase (DAAO, EC 1.4.3.3) for both negative selection and counter-selection, depending on the substrate. DAAO catalyzes the oxidative deamination of a range of D-amino acids (Alonso J et al. (1998) Microbiol. 144, 1095-1101). Thus, the D-amino acid oxidase constitutes a dual-function marker. The marker has been successfully established in *Arabidopsis thaliana*, and proven to be versatile, rapidly yielding unambiguous results, and allowing selection immediately after germination (WO 03/ 060133)

Many prokaryotes and eukaryotes metabolize D-amino acids (Pilone MS (2000) Cell. Mol. Life. Sci. 57, 1732-174), but current information suggests that D-amino acid metabolism is severely restricted in plants.

However, studies of amino acid transporters in plants have shown that several of these proteins may mediate the transport of both L- and D-enantiomers of amino acids, although the latter usually at lower rates (Frommer WB et al. (1995) Proc. Natl. Acad. Sci. USA 92, 12036-12040; Boorer KJ et al. (1996) J. Biol. Chem. 271, 2213-22203). These findings imply that plants absorb D-amino acids but metabolize few if any D-amino acids. D-amino acid catabolism follows several routes, one of the most common being oxidative deamination (Pilone MS (2000) Cell. Mo/. Life. Sci. 57, 1732-1742). The natural occurrence of D-amino acids in plants is generally low, with measurable levels of D-alanine, D-serine, D-glutamine and D-asparagine but no detectable levels of D-valine and D-isoleucine (Bruckner H & Westhauser T (2003) Amino acids 24, 43-55). Hence, the amount and nature of substrates that DAAO may engage under natural conditions would not cause negative effects on plants.

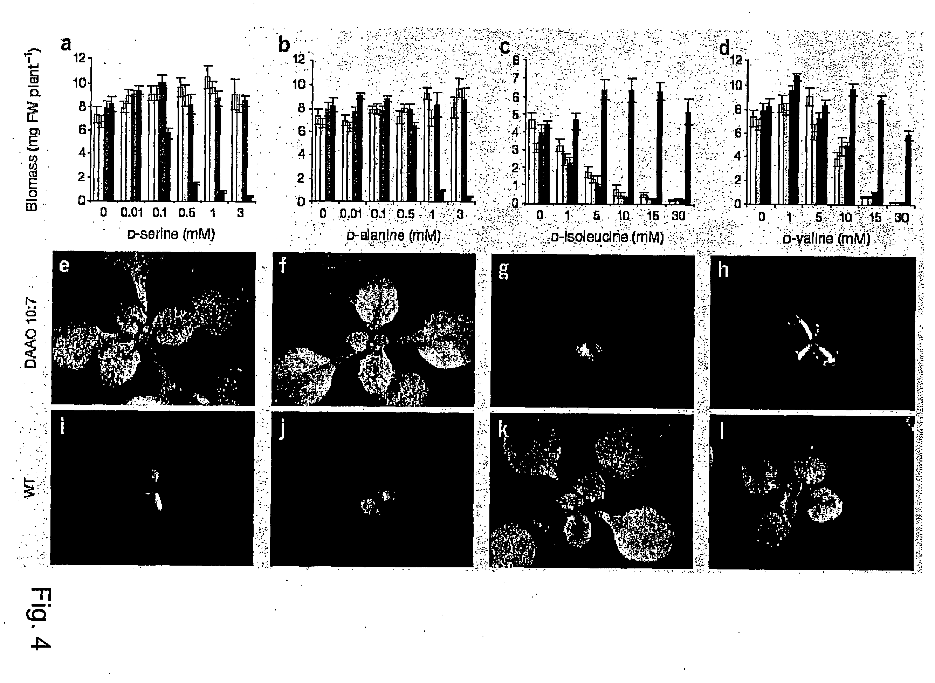

Within this invention it is demonstrated that the toxicity of D-amino acids like e.g., D-serine and D-alanine could be alleviated by the insertion of a gene encoding an enzyme that metabolizes D-amino acids. Wild-type *A. thaliana* were transformed with the dao1 gene from the yeast *Rhodotorula gracilis* under the control of the constitutive promoter CaMV 35S promoter. Exposure of this transgenic plant to D-alanine or D-serine showed that it could detoxify both of these D-amino acids (Fig. 4a,b).

In another preferred embodiment the second (non-phytotoxic, but metabolizable into phytotoxic) compound M is preferably comprising a D-amino acid structure selected from the group consisting of D-isoleucine, D-valine, D-asparagine. D-leucine, D-lysine, D-proline, and D-glutamine; more preferably D-isoleucine, D-valine, and derivatives thereof. Most preferably, M is comprising and/or consisting of D-isoleucine, D-valine, or derivatives thereof.

In contrast to D-amino acids like D-serine and D-alanine, other D-amino acids like D-valine and D-isoleucine, which are not toxic to wild-type plants, have a strong negative influence on the growth of plants expressing DAAO (Fig. 4c,d). The findings that DAAO expression mitigated the toxicity of D-serine and D-alanine, but induced metabolic changes that made D-isoleucine and D-valine toxic, demonstrate that the enzyme could provide a substrate-dependent, dual-function, selectable marker in plants. Selection is based on differences in the toxicity of different D-amino acids and their metabolites to plants. Thus, D-alanine and D-serine are toxic to plants, but are metabolized by DAAO into nontoxic products, whereas D-isoleucine and D-valine have low toxicity, but are metabolized by DAAO into the toxic keto acids 3-methyl-2-oxopentanoate and 3-methyl-2-oxobutanoate, respectively. Hence, both positive and negative selection is possible with the same marker gene, which is therefore considered a dual-function marker.

Another subject matter of the invention relates to DNA constructs which are suitable for employing in the method of the invention. A DNA construct suitable for use in the method of the invention is preferably comprising
a) a first expression cassette comprising a nucleic acid sequence encoding a D-amino acid oxidase operably linked with a promoter allowing expression in plant cells or plants, wherein said first expression cassette is flanked by sequences which allow for specific deletion of said first expression cassette, and
b) at least one second expression cassette suitable for conferring to said plant an agronomically valuable trait, wherein said second expression cassette is not localized between said sequences which allow for specific deletion of said first expression cassette.

### 1. The Dual-Function Marker of the Invention

The term D-amino acid oxidase (abbreviated DAAO, DAMOX, or DAO) is referring to the enzyme coverting a D-amino acid into a 2-oxo acid, by - preferably - employing Oxygen (O₂) as a substrate and producing hydrogen peroxide (H₂O₂) as a co-product (Dixon M & Kleppe K. Biochim. Biophys. Acta 96 (1965) 357-367; Dixon M & Kleppe K Biochim. Biophys. Acta 96 (1965) 368-382; Dixon M & Kleppe Biochim. Biophys. Acta 96 (1965) 383-389; Massey V et al. Biochim. Biophys. Acta 48 (1961) 1-9. Meister A & Wellner D Flavoprotein amino acid oxidase. In: Boyer, P.D., Lardy, H. and Myrbäck, K. (Eds.), The Enzymes, 2nd ed., vol. 7, Academic Press, New York, 1963, p. 609-648.)

DAAO can be described by the Nomenclature Committee of the International Union of Biochemistry and Molecular Biology (IUBMB) with the EC (Enzyme Commission) number EC 1.4.3.3. Generally an DAAO enzyme of the EC 1.4.3.3. class is an FAD flavoenzyme that catalyzes the oxidation of neutral and basic D-amino acids into their corresponding keto acids. DAAOs have been characterized and sequenced in fungi and vertebrates where they are known to be located in the peroxisomes. The term D-amino oxidase further comprises D-aspartate oxidases (EC 1.4.3.1) (DASOX) (Negri A et al. (1992) J Biol Chem. 267:11865-11871), which are enzymes structurally related to DAAO catalyzing the same reaction but active only toward dicarboxylic D-amino acids. Within this invention DAAO of the EC 1.4.3.3. class are preferred.

In DAAO, a conserved histidine has been shown (Miyano M et al. (1991) J Biochem 109:171-177) to be important for the enzyme's catalytic activity. In a preferred embodiment of the invention a DAAO is referring to a protein comprising the following consensus motive:

[LIVM]-[LIVM]-H*-[NHA]-Y-G-x-[GSA]-[GSA]-x-G-x₅-G-x-A

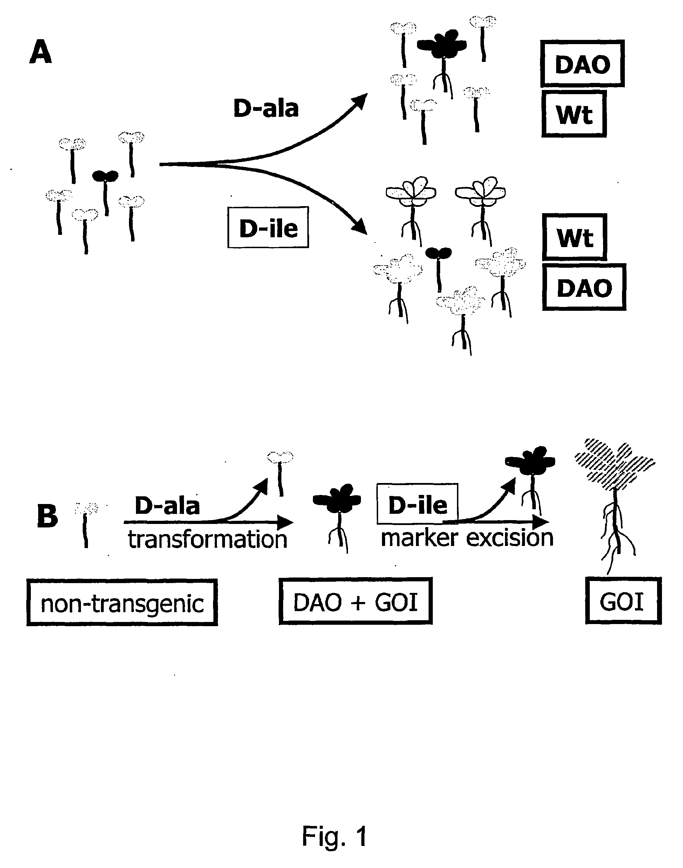

wherein amino acid residues given in brackets represent alternative residues for the respective position, x represents any amino acid residue, and indices numbers indicate the respective number of consecutive amino acid residues. The abbreviation for the individual amino acid residues have their standard IUPAC meaning as defined above. A Clustal multiple alignment of the characteristic active site from various D-amino acids is shown in Fig. 1. Further potential DAAO enzymes comprising said motif are described in table below:

**Tab.1: Suitable D-amino acid oxidases from various organism. Acc.-No. refers to protein sequence from SwisProt database.**

| Acc.-No. | Gene Name | Description | Source Organism | Length |
|---|---|---|---|---|
| Q19564 | F18E3.7 | Putative D-amino acid oxidase (EC 1.4.3.3) (DAMOX) (DAO) (DAAO) | Caenorhabditis elegans | 334 |
| P24552 | | D-amino acid oxidase (EC 1.4.3.3) (DAMOX) (DAO) (DAAO) | Fusarium solani (subsp. pisi) (Nectria haematococca) | 361 |
| P14920 | DAO, DAMOX | D-amino acid oxidase (EC 1.4.3.3) (DAMOX) (DAO) (DAAO) | Homo sapiens (Human) | 347 |
| P18894 | DAO, DAO1 | D-amino acid oxidase (EC 1.4.3.3) (DAMOX) (DAO) (DAAO) | Mus musculus (Mouse) | 346 |
| P00371 | DAO | D-amino acid.oxidase (EC 1.4.3.3) (DAMOX) (DAO) (DAAO) | Sus scrofa (Pig) | 347 |
| P22942 | DAO | D-amino acid oxidase (EC 1.4.3.3) (DAMOX) (DAO) (DAAO) | Oryctolagus cuniculus (Rabbit) | 347 |
| 035078 | DAO | D-amino acid oxidase (EC 1.4.3.3) (DAMOX) (DAO) (DAAO) | Rattus norvegicus (Rat) | 346 |
| P80324 | DAO1 | D-amino acid oxidase (EC 1.4.3.3) (DAMOX) (DAO) (DAAO) | Rhodosporidium toruloides (Yeast) (Rhodotorula gracilis) | 368 |
| U60066 | DAO | D-amino acid oxidase (EC 1.4.3.3) (DAMOX) (DAO) (DAAO) | Rhodosporidium toruloides, strain TCC 26217 | 368 |
| Q99042 | DAO1 | D-amino acid oxidase (EC 1.4.3.3) (DAMOX) (DAO) (DAAO) | Trigonopsis variabilis (Yeast) | 356 |
| P31228 | DDO | D-aspartate oxidase (EC 1.4.3.1) (DASOX) (DDO) | Bos taurus (Bovine) | 341 |
| Q99489 | DDO | D-aspartate oxidase (EC 1.4.3.1) (DASOX) (DDO) | Homo sapiens (Human) | 341 |
| Q9C1L2 | NCU06558.1 | (AF309689) putative D-amino acid oxidase G6G8.6 (Hypothetical protein) | Neurospora crassa | 362 |
| Q7SFW4 | NCU03131.1 | Hypothetical protein | Neurospora crassa | 390 |
| Q8N552 | | Similar to D-aspartate oxidase | Homo sapiens (Human) | 369 |
| Q7Z312 | DKFZP686F04272 | Hypothetical protein DKFZp686F04272 | Homo sapiens (Human) | 330 |
| Q9VM80 | CG11236 | CG11236 protein (GH12548p) | Drosophila melanogaster (Fruit fly) | 341 |
| 001739 | F20H11.5 | F20H11.5 protein | Caenorhabditis elegans | 383 |
| 045307 | C47A10.5 | C47A10.5 protein | Caenorhabditis elegans | 343 |
| Q8SZN5 | CG12338 | RE73481p | Drosophila melanogaster (Fruit fly) | 335 |
| Q9V5P1 | CG12338 | CG12338 protein (RE49860p) | Drosophila melanogaster (Fruit fly) | 335 |
| Q86JV2 | | Similar to Bos taurus (Bovine). D-aspartate oxidase (EC 1.4.3.1) (DASOX) (DDO) | Dictyostelium discoideum (Slime mold) | 599 |
| Q95XG9 | Y69A2AR.5 | Hypothetical protein | Caenorhabditis elegans | 322 |
| Q7Q7G4 | AGCG53627 | AgCP5709 (Fragment) | Anopheles gambiae str. PEST | 344 |
| Q7PWY8 | AGCG53442 | AgCP12432 (Fragment) | Anopheles gambiae str. PEST | 355 |
| Q7PWX4 | AGCG45272 | AgCP12797 (Fragment) | Anopheles gambiae str. PEST | 373 |
| Q8PG95 | XAC3721 | D-amino acid oxidase | Xanthomonas axonopodis (pv. citri) | 404 |
| Q8P4M9 | XCC3678 | D-amino acid oxidase | Xanthomonas campestris (pv. campestris) | 405 |
| Q9X7P6 | SC06740, SC5F2A.23C | Putative D-amino acid oxidase | Streptomyces coelicolor | 320 |
| Q82MI8 | DAO, SAV1672 | Putative D-amino acid oxidase | Streptomyces avermitilis | 317 |
| Q8VCW7 | DAO1 | D-amino acid oxidase | Mus musculus (Mouse) | 345 |
| Q9Z302 | | D-amino acid oxidase D-amino | Cricetulus griseus (Chinese hamster) | 346 |
| Q9Z1M5 | | D-amino acid oxidase | Cavia porcellus (Guinea pig) | 347 |
| Q922Z0 | | Similar to D-aspartate oxidase | Mus musculus (Mouse) | 341 |
| Q8R2R2 | | Hypothetical protein | Mus musculus (Mouse) | 341 |
| P31228 | | D-aspartate oxidase | B.taurus | 341 |

D-Amino acid oxidase (EC-number 1.4.3.3) can be isolated from various organisms, including but not limited to pig, human, rat, yeast, bacteria or fungi. Example organisms are Candida tropicalis, Trigonopsis variabilis, Neurospora crassa, Chlorella vulgaris, and Rhodotorula gracilis. A suitable D-amino acid metabolising polypeptide may be an eukaryotic enzyme, for example from a yeast (e.g. *Rhodotorula gracilis),* fungus, or animal or it may be a prokaryotic enzyme, for example, from a bacterium such as *Escherichia coli.* Examples of suitable polypeptides which metabolise D-amino acids are shown in Table 1 and Table 2.

**Tab.2: Suitable D-amino acid oxidases from various organism. Acc.-No. refers to protein sequence from SwisProt database.**

| | |
|---|---|
| Q19564 | Caenorhabditis elegans. F18E3.7. |
| P24552 | Fusarii solani (subsp. pisi) (Nectria haematococca). |
| JX0152 | Fusarium solani |
| P14920 | Homo sapiens (Human) |
| P18894 | Mus musculus (mouse) |
| P00371 | Sus scrofa (pig) |
| P22942 | Oryctolagus cuniculus (Rabbit) |
| 035078 | Rattus norvegicus (Rat) |
| P80324 | Rhodosporidium toruloides (Yeast) (Rhodotorula gracilis) |
| Q99042 | Trigonopsis variabilis |
| Q9Y7N4 | Schizosaccharomyces pombe (Fission yeast) SPCC1450 |
| 001739 | Caenorhabditis elegans.F20H11.5 |
| Q28382 | Sus scrofa (Pig). |
| 033145 | Mycobacterium leprae |
| Q9X7P6 | Streptomyces coelicolor.SCSF2A.23C |
| Q9JXF8 | Neisseria meningitidis (serogroup B). |
| Q9Z302 | Cricetulus griseus (Chinese hamster) |
| Q921M5 | D-AMINO ACID OXIDASE. Cavia parcellus (Guinea pig) |

Preferably the D-amino acid oxidase is selected from the enzymes encoded by a nucleic acid sequence or a corresponding amino acid sequences selected from the following table 3:

**Tab.3: Suitable D-amino acid oxidases from various organism. Acc.-No. refers to protein sequence from GenBank database.**

| GenBanc Acc.-No | Organism | SEQ ID |
|---|---|---|
| U60066 | Rhodosporidium toruloides (Yeast) | SEQ ID NO: 1, 2 |
| Z71657 | Rhodotorula gracilis | |
| A56901 | Rhodotorula gracilis | |
| AF003339 | Rhodosporidium toruloides | |
| AF003340 | Rhodosporidium toruloides | |
| U53139 | Caenorhabditis elegans | SEQ ID NO: 3, 4 |
| D00809 | Nectria haematococca | SEQ ID NO: 5, 6 |
| Z50019. | Trigonopsis variabilis | SEQ ID NO: 7, 8 |
| NC_003421 | Schizosaccharomyces pombe (fission yeast) | SEQ ID NO: 9, 10 |
| AL939129. | Streptomyces coelicolor A3(2) | SEQ ID NO: 11, 12 |
| AB042032 | Candida boidinii | SEQ ID NO: 13, 14 |

DAAO is a well-characterized enzyme, and both its crystal structure and its catalytic mechanism have been determined by high-resolution X-ray spectroscopy (Umhau S. et al. (2000) Proc. Natl. Acad. Sci. USA 97, 12463-12468). It is a flavoenzyme located in the peroxisome, and its recognized function in animals is detoxification of D-amino acids (Pilone MS (2000) Cell. Mol. Life. Sci. 57, 1732-174). In addition, it enables yeasts to use D-amino acids for growth (Yurimoto H et al. (2000) Yeast 16, 1217-1227). As demonstrated above, DAAO from several different species have been characterized and shown to differ slightly in substrate affinities (Gabler M et al. (2000) Enzyme Microb. Techno. 27, 605-611), but in general they display broad substrate specificity, oxidatively deaminating all D-amino acids (except D-glutamate and D-aspartate for EC 1.4.3.3. calss DAAO enzymes; Pilone MS (2000) Cell. Mol. Life. Sci. 57, 1732-174).

DAAO activity is found in many eukaryotes (Pilone MS (2000) Cell. Mol. Life. Sci. 57, 1732-174), but there is no report of DAAO activity in plants. The low capacity for D-amino acid metabolism in plants has major consequences for the way plants respond to D-amino acids. For instance, the results provided herein demonstrate that growth of *A. thaliana* in response to D-serine and/or D-alanine is inhibited even at quite low concentrations (Fig. 1a,b). On the other hand, some D-amino acids, like D-valine and D-isoleucine, have minor effects on plant growth (Fig. 1c,d) per se, but can be converted into toxic metabolites by action of a DAAO.

In an preferred embodiment D-amino acid oxidase expressed form the DNA-construct of the invention has preferably enzymatic activity against at least one of the amino acids selected from the group consisting of D-alanine, D-serine, D-isoleucine, D-valine, and derivatives thereof. Preferably said D-amino acid oxidase is selected from the group of amino acid sequences comprising
a) the sequences described by SEQ ID NO: 2, 4, 6, 8, 10, 12, and 14, and

Preferably said D-amino acid oxidase is selected from the group of amino acid sequences comprising
a) the sequences described by SEQ ID NO: 2, 4, 6, 8, 10, 12, and 14, and
b) the sequences having a sequence homology of at least 40%, preferably 60%, more preferably 80%, most preferably 95% with a sequence as described by SEQ ID NO: 2, 4, 6, 8, 10, 12, and 14, and
c) the sequences hybridizing under low or high stringency conditions - preferably under high stringency conditions - with a sequence as described by SEQ ID NO: 2, 4, 6, 8, 10, 12, and 14.

Suitable D-amino acid oxidases also include fragments, mutants, derivatives, variants and alleles of the polypeptides exemplified above. Suitable fragments, mutants, derivatives, variants and alleles are those which retain the functional characteristics of the D-amino acid oxidase as defined above. Changes to a sequence, to produce a mutant, variant or derivative, may be by one or more of addition, insertion, deletion or substitution of one or more nucleotides in the nucleic acid, leading to the addition, insertion, deletion or substitution of one or more amino acids in the encoded polypeptide. Of course, changes to the nucleic acid that make no difference to the encoded amino acid sequence are included.

The D-amino acid oxidase of the invention may be expressed in the cytosol, peroxisome, or other intracellular compartment of the plant cell. Compartmentalisation of the D-amino acid metabolising polypeptide may be achieved by fusing the nucleic acid sequence encoding the DAAO polypeptide to a sequence encoding a transit peptide to generate a fusion protein. Gene products expressed without such transit peptides generally accumulate in the cytosol. The localisation of expressed DAAO in the peroxisome produces H₂0₂ that can be metabolised by the H₂0₂ degrading enzyme catalase. Higher levels of D-amino acids may therefore be required to produce damaging levels of H₂0₂. Expression of DAAO in the cytosol, where levels of catalase activity are lower, reduces the amount of D-amino acid required to produce damaging levels H₂0₂. Expres7sion of DAAO in the cytosol may be achieved by removing peroxisome targeting signals or transit peptides from the encoding nucleic acid sequence. For example, the *dao1* gene (EC: 1.4.3.3: GenBank Acc.-No.: U60066) from the yeast *Rhodotorula gracilis (Rhodosporidium toruloides)* was cloned as described (WO 03/060133). The last nine nucleotides encode the signal peptide SKL, which guides the protein to the peroxisome sub-cellular organelle. Although no significant differences were observed between cytosolic and peroxisomal expressed DAAO, the peroxisomal construction was found to be marginally more effective than the cytosolic version in respect of inhibiting the germination of the DAAO transgenic plants on 30 mM D-Asn. However, both I.1 The compounds X and M

The term "Compound X" means one or more chemical substances (i.e. one chemical compound or a mixture of two or more compound) which is phytotoxic against plant cells not functionally expressing the D-amino acid oxidase expressed from the first expression cassette of the invention, and which can be metabolized by said D-amino acid oxidase into one or more compound(s) Y which are non-phytotoxic or less phytotoxic than compound X.

The term "phytotoxic", "phytotoxicity" or "phytotoxic effect" as used herein is intended to mean any measurable, negative effect on the physiology of a plant or plant cell resulting in symptoms including (but not limited to) for example reduced or impaired growth, reduced or impaired photosynthesis, reduced or impaired cell division, reduced or impaired regeneration (e.g., of a mature plant from a cell culture, callus, or shoot etc.), reduced or impaired fertility etc. Phytotoxicity may further include effects like e.g., necrosis or apoptosis. In an preferred embodiment results in an reduction of growth or regenerability of at least 50%, preferably at least 80%, more preferably at least 90% in comparison with a plant which was not treated with said phytotoxic compound.

The phytotoxic compound X is metabolized by said D-amino acid oxidase into one or more compound(s) Y which are non-phytotoxic or less phytotoxic than compound X. In an improved embodiment the toxicity (as for example assessed by one of the physiological indicators exemplified above like e.g., growth or regenerability) of the phytotoxic compound is reduced by the conversion to at least 50%, preferably at least 80%, more preferably at least 90% of the original phytotoxicity imposed by compound X. More preferred this reduction results in an phytotoxic effect on plants (or plant cells) functionally expressing said D-amino acid oxidase and treated with said compound X in comparison with plants (or plant cells; regardless whether expressing said D-amino acid oxidase or not) not treated with said compound X of not more then 30%, preferably not more then 15%, more preferably not more then 10 %, most preferably no statistically significant difference in physiology can be observed.

The term "Compound M" means one or more chemical substances (i.e. one chemical compound or a mixture of two or more compounds) which is non-phytotoxic or moderately phytotoxic against plant cells not functionally expressing said D-amino acid oxidase, and which can be metabolized by said D-amino acid oxidase into one or more compound(s) N which are phytotoxic or more phytotoxic than compound M.

The term "phytotoxic", "phytotoxicity" or "phytotoxic effect" has the same definition as given above.

The term "non-phytotoxic" means that no statistically significant difference in physiology can be observed between plant cells or plants (not comprising a functional D-amino

The term "non-phytotoxic" means that no statistically significant difference in physiology can be observed between plant cells or plants (not comprising a functional D-amino acid oxidase) and the same plant cells or plants treated with compound M or untreated plants.

The term "moderate phytotoxic" means an reduction of an physiological indicator (as exemplified above like e.g., growth or regenerability) for treated plant cells or plants - not comprising a functional D-amino acid oxidase - in comparison with untreated plants or plant cells (regardless whether expressing said D-amino acid oxidase or not) not irreversibly effecting growth and/or performance of said treated plants or plant cells (but using the compound in a concentration sufficient to allow for distinguishing and/or separating transgenic plants (i.e., comprising said dual function marker) from non-transgenic plants (i.e., not comprising said marker)). Preferably, the reduction of an physiological indicator for said treated plant cells is not more then 30%, preferably not more then 15%, more preferably not more then 10 %.

The phytotoxic compound M is metabolized by said D-amino acid oxidase into one or more compound(s) N which are phytotoxic or more phytotoxic than compound M. In an improved embodiment the toxicity (as for example assessed by one of the physiological indicators exemplified above like e.g., growth or regenerability) of the compound M is increased in a way that one or more physiological indicator (as exemplified above like e.g., growth or regenerability) are reduced by at least 20%, preferably at least 40%, more preferably at least 60%, most preferably at least 90%. The phytotoxic effect of compound N in comparison to compound M is increased by at least 100% (i.e. twice), preferably at least 500% (i.e. 5-times), more preferably at least 1000% (i.e. 10 times).

The fact that compound X and M are D-amino acids does not rule out the presence of L-amino acid structures or L-amino acids. For some applications it may be preferred (e.g., for cost reasons) to apply a racemic mixture of D- and L-amino acids (or a mixture with enriched content of D-amino acids). Preferably, the ratio of the D-amino acid to the corresponding L-enantiomer is at least 1:1, preferably 2:1, more preferably 5:1, most preferably 10:1 or 100:1.

The preferred compound may be used in isolated form or in combination with other substances. For the purpose of application, the compound X or M are advantageously used together with the adjuvants conventionally employed in the art of formulation, and are therefore formulated in known manner, e.g. into emulsifiable concentrates, coatable pastes, directly sprayable or dilutable solutions, dilute emulsions, wettable powders, soluble powders, dusts, granulates, and also encapsulations in e.g. polymer substances. As with the nature of the compositions to be used, the methods of application, such as spraying, atomising, dusting, scattering, coating or pouring, are chosen in accordance with the intended objectives and the prevailing circumstances.

The formulations, i.e. the compositions, preparations or mixtures containing compound X or M (active ingredient), and, where appropriate, a solid or liquid adjuvant, are prepared in known manner, e.g. by homogeneously mixing and/or grinding the active ingredients with extenders, e.g. solvents, solid carriers and, where appropriate, surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or dimethylformamide, as well as vegetable oils or epoxidised vegetable oils, such as epoxidised coconut oil or soybean oil; or - preferably - water.

The solid carriers used e.g. for dusts and dispersible powders are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable non-sorbent carriers are, for example, calcite or sand. In addition, a great number of pre-granulated materials of inorganic or organic nature can be used, e.g. especially dolomite or pulverised plant residues.

Depending on the nature of the compound X or M to be formulated suitable surface-active compounds are nonionic, cationic and/or anionic surfactants having good emulsifying, dispersing and wetting properties. The term "surfactants" will also be understood as comprising mixtures of surfactants.

Both so-called water-soluble soaps and also water-solubfe synthetic surface-active compounds are suitable anionic surfactants. Suitable soaps are the alkali metal salts, alkaline earth metal salts or unsubstituted or substituted ammonium salts of higher fatty acids (C₁₀-C₂₂), e.g. the sodium or potassium salts of oleic or stearic acid or of natural fatty acid mixtures which can be obtained e.g. from coconut oil or tallow oil. Fatty acid methyltaurin salts may also be mentioned as surfactants.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammonium salts and contain a C.sub.8 -C.sub.22 alkyl radical which also includes the alkyl moiety of acyl radicals, e.g. the sodium or calcium salt of lignosulfonic acid, of dodecylsulfate or of a mixture of fatty alcohol sulfates obtained from natural fatty acids. These compounds also comprise the salts of sulfated and sulfonated fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzenesulfonic acid, dibutylnaphtha-lenesulfonic acid, or of a condensate of naphthalenesulfonic acid and formaldehyde. Also suitable are corresponding phosphates, e.g. salts of the phosphoric acid ester of an adduct of p-nonylphenol with 4 to 14 moles.of ethylene oxide, or phospholipids.

Non-ionic surfactants are preferably polyglycol ether derivatives of aliphatic or cycloaliphatic alcohols, saturated or unsaturated fatty acids and alkylphenols, said derivatives contains 3 to 30 glycol ether groups and 8 to 20 carbon atoms in the (aliphatic) hydrocarbon moiety and 6 to 18 carbon atoms in the alkyl moiety of the alkylphenols. Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit. Representative examples of non-ionic surfactants are nonylphenolpoly-ethoxyethanols, castor oil polyglycol ethers, polypropylene/polyethylene oxide adducts, tributylphenoxy-polyethoxyethanol, polyethylene glycol and octylphenoxy-polyethoxyethanol. Fatty acid esters of polyoxyethylene sorbitan, e.g. polyoxyethylene sorbitan trioleate, are also suitable.

Cationic surfactants are preferably quaternary ammonium salts which contain, as N-substituent, at least one C₈-C₂₂ alkyl radical and, as further substituents, unsubstituted or halogenated lower alkyl, benzyl or hydroxy-lower alkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described e.g. in the following publications: "McCutcheon's Detergents and Emulsifiers Annual" MC Publishing Corp., Ridgewood, N.J., 1981. Stache, H., "Tensid-Taschenbuch", Carl Hanser Verlag Munich/Vienna 1981.

The compositions usually contain 0.1 to 99% by weight, preferably 0.1 to 95% by weight, of a compound X or M, 1 to 99.9% by weight, preferably 5 to 99.8% by weight, of a solid or liquid adjuvant and 0 to 25% by weight, preferably 0.1 to 25% by weight, of a surfactant.

The compositions may also contain further ingredients such as stabilizers, antifoams, viscosity regulators, binders, tackifiers as well as fertilizers or other active ingredients for obtaining special effects.

Various methods and techniques are suitable for employing compound X or M or compositions containing them for treating plant cells or plants. Such method may include
i) Incorporation into liquid or solidified media or substrates utilized during transformation, regeneration or growth of plant cells, plant material or plants.
ii) Seed dressing
iii) Application by spraying (e.g. from a tank mixture utilizing a liquid formulation)
ii) Seed dressing
iii) Application by spraying (e.g. from a tank mixture utilizing a liquid formulation)

### I.1.1 Compound X

Preferably compound X is comprising a substance comprising a structure selected from the group of consisting of D-tryptophane, D-histidine, D-arginine, D-threonine, D-methionine, D-serine, and D-alanine, more preferably a structure selected from the group consisting of D-serine, and D-alanine. Most preferably compound X is comprising a substance comprising the structure of D-alanine.

Preferably compound X is comprising a substance selected from the group of consisting of D-tryptophane, D-histidine, D-arginine, D-threonine, D-methionine, D-serine, and D-alanine, more preferably selected from the group consisting of D-serine, and D-alanine. Most preferably compound X is comprising D-alanine

The use of D-alanine has the advantage that racemic mixtures of D- and L-alanine can be applied without disturbing or detrimental effects of the L-enantiomer. Therefore, in an improved embodiment an racemic mixture of D/L-alanine is employed as compound X.

Also disclosed are D-amino acid structure comprising herbicidal compounds. Such compounds are for example described in US 5,059,239, and may include (but shall not be limited to) N-benzoyl-N-(3-chloro-4-fluorophenyl)-DL-alanine, N-benzoyl-N-(3-chloro-4-fluorophenyl)-DL-alanine methyl ester, N-benzoyl-N-(3-chloro-4-fluorophenyl)-DL-alanine ethyl ester, N-benzoyl-N-(3-chloro-4-fluorophenyl)-*D*-alanine, N-benzoyl-N-(3-chloro-4-fluorophenyl)-*D*-alanine methyl ester, or N-benzoyi-N-(3-chloro-4-fluorophenyl)-*D*-alanine isopropyl ester.

When applied via the cell culture medium (e.g., incorporated into agar-solidified MS media plates), D-alanine can be employed in concentrations of about 0.1 mM to about 100 mM, preferably about 0.3 mM to about 30 mM, more preferably about 1 mM to about 5 mM.

When applied via the cell culture medium (e.g., incorporated into agar-solidified MS media plates), D-serine can be employed in concentrations of about 0.1 to about 10 mM, preferably about 0.3 to 4 mM, more preferably about 0.5 mM to about 1.5 mM.

### I.1.2 Compound M

Preferably compound M is comprising a substance comprising a structure selected from the group of consisting of D-isoleucine, D-valine, D-asparagine, D-leucine, D-lysine, D-proline, and D-glutamine, more preferably a structure selected from the group consisting of D-isoleucine, and D-valine. Most preferably compound M is comprising a substance comprising the structure of D-isoleucine.

Preferably compound M is comprising a substance selected from the group of consisting of D-isoleucine, D-valine, D-asparagine, D-leucine, D-lysine, D-proline, and D-glutamine, more preferably selected from the group consisting of D-isoleucine, and D-valine. Most preferably compound M is comprising D-isoleucine.

When applied via the cell culture medium (e.g., incorporated into agar-solidified MS media plates), D-isoleucine can be employed in concentrations of about 0.1 mM to about 100 mM, preferably about 1 mM to about 50 mM, more preferably about 10 mM to about 30 mM.

When applied via the cell culture medium (e.g., incorporated into agar-solidified MS media plates), D-valine can be employed in concentrations of about 1 to about 100 mM, preferably about 5 to 50 mM, more preferably about 15 mM to about 30 mM.

When applied via the cell culture medium (e.g., incorporated into agar-solidifed MS media plates), D-asparagine or D-glutamine can be employed in concentrations of about 0-5 to about 100 mM, preferably about 1 to 50 mM, more preferably about 3 mM to about 20 mM. to about 20 mM.

### I.1.3 Mode of Application

As described above, the selection can be done by treating plants comprising transformed plant cells in soil for three consecutive days, or by treating seeds comprising transformed plant cells for five days during germination on media. Surprisingly, the D-amino acid compounds are able to exhibit their growth modulating properties not only during cell culture but also later when applied on plants via spraying. When applied via spraying, D-alanine may be applied in concentrations of about 5 to about 100 mM, preferably from about 10 to about 80 mM, more preferably from about 40 to about 60 mM. When applied via spraying, D-serine may be applied in concentrations of about 5 to about 80 mM, preferably from about 10 to about 60 mM, more preferably from about 20 to about 40 mM.

### II. The Marker Excision Feature of the Invention

It is one essential feature of the invention that the dual-function marker of the invention is specifically deleted after its use. Preferably, deletion of the first expression cassette encoding for said dual-function marker can be realized by various means known in the art, including but not limited to one or more of the following methods:
a) recombination induced by a sequence specific recombinase, wherein said first expression cassette is flanked by corresponding recombination sites in a way that re-
b) homologous recombination between homology sequences A and A' flanking said first expression cassette, preferably induced by a sequence-specific double-strand break between said homology sequences caused by a sequence specific endonuclease, wherein said homology sequences A and A' have sufficient length and homology in order to ensure homologous recombination between A and A', and having an orientation which - upon recombination between A and A' - will lead to excision of said first expression cassette from the genome of said plant (for specific embodiments see III.2 below).

Accordingly, for ensuring marker deletion / excision the expression cassette for the D-amino acid oxidase (the first expression construct) comprised in the DNA construct of the invention is flanked by sequences which allow for specific deletion of said expression cassette. Said sequences may be recombination sites for a sequence specific recombinase, which are placed in a way the recombination induced between said flanking recombination sites results in deletion of the said first expression cassette from the genome. There are various recombination sites and corresponding sequence specific recombinases known in the art (described herein below), which can be employed for the purpose of the invention.

In another preferred embodiment, deletion / excision of the dual-marker sequence is performed by intramolecular (preferably intrachromosomal) homologous recombination. Homologous recombination may occur spontaneous but is preferably induced by a sequence-specific double-strand break (e.g., between the homology sequences). The basic principals are disclosed in WO 03/004659. For this purpose the first expression construct (encoding for the dual-function marker) is flanked by homology sequences A and A', wherein said homology sequences have sufficient length and homology in order to ensure homologous recombination between A and A', and having an orientation which - upon recombination between A and A' - will lead to an excision of first expression cassette from the genome. Furthermore, the sequence flanked by said homology sequences further comprises at least one recognition sequence of at least 10 base pairs for the site-directed induction of DNA double-strand breaks by a sequence specific DNA double-strand break inducing enzyme, preferably a sequence-specific DNA-endonuclease, more preferably a homing-endonuclease, most preferably a endonuclease selected from the group consisting of I-Scel, I-Cpal, I-Cpall, I-Crel and I-Chul or chimeras thereof with ligand-binding domains. Suitable endonuclease are described herein below.

### III.1 Recombination Sites and Recombinases of the Invention

Sequence specific recombinases and their corresponding recombination sites suitable within the present invention may include but are not limited to the Cre/lox system of the bacteriophage P1 (Dale EC and Ow DW (1991) Proc Natl Acad Sci USA 88:10558-10562; Russell SH et al. (1992) Mol Gene Genet 234: 49-59; Osborne BI et al. (1995) Plant J. 7, 687-701), the yeast FLP/FRT system (Kilby NJ et al. (1995) Plant J 8:637-652; Lyznik LA et al. (1996) Nucleic Acids Res 24:3784-3789), the Mu phage Gin recombinase, the E. coli Pin recombinase or the R/RS system of the plasmid pSR1 (Onouchi H et al.(1995) Mol Gen Genet 247:653-660; Sugita Ket al. (2000) Plant J. 22:461-469). The recombinase (for example Cre or FLP) interacts specifically with its corresponding recombination sequences (34 bp lox sequence and 47 bp FRT sequence, respectively) in order to delete or invert the interposed sequences. Deletion of standard selection marker in plants which was flanked by two lox sequences by the Cre is described (Dale EC and Ow DW (1991) Proc Natl Acad Sci USA 88:10558-10562). The preferred recombination sites for suitable recombinases are described in Table 4 below:

**Tab 4: Suitable sequence specific recombinases for use in the method of the invention.**

| Recombinase | Organism of origin | Recombination Sites |
|---|---|---|
| CRE | Bacteriophage P1 | |
| FLP | Saccharomyces cerevisiae | |
| R | pSR1 Plasmids | |

### III.2 The Homology Sequences

Referring to the homology sequences (e.g., A, A') "sufficient length" preferably refers to sequences with a length of at least 20 base pairs, preferably at least 50 base pairs, especially preferably at least 100 base pairs, very especially preferably at least 250 base pairs, most preferably at least 500 base pairs.

Referring to the homology sequences (e.g., A, A'), "sufficient homology" preferably refers to sequences with at least 70%, preferably 80%, by preference at least 90%, especially preferably at least 95%, very especially preferably at least 99%, most preferably 100%, homology within these homology sequences over a length of at least 20 base pairs, preferably at least 50 base pairs, especially preferably at least 100 base pairs, very especially preferably at least 250 base pairs, most preferably at least 500 base pairs.

The homology sequences A and A' are preferably organized in the form of a direct repeat. The term "direct repeat" means a subsequent localization of two sequences on the same strand of a DNA molecule in the same orientation, wherein these two sequences fulfill the above given requirements for homologous recombination between said two sequences.

In a preferred embodiment, the homology sequences may be a duplication of a sequence having additional use within the DNA construct. For example, the homology sequences may be two transcription terminator sequences. One of these terminator sequences may be operably linked to the agronomically valuable trait, while the other may be linked to the dual-function selection marker, which is localized in 3'-direction of the trait gene. Recombination between the two terminator sequences will excise the marker gene but will reconstitute the terminator of the trait gene. In another example, the homology sequences may be two promoter sequences. One of these promoter sequences may be operably linked to the agronomically valuable trait, while the other may be linked to the dual-function selection marker, which is localized in 5'-direction of the trait gene. Recombination between the two promoter sequences will excise the marker gene but will reconstitute the promoter of the trait gene. The person skilled in the art will know that the homology sequences do not need to be restricted to a single functional element (e.g. promoter or terminator), but may comprise or extent to other sequences (e.g. being part of the coding region of the trait gene and the respective terminator sequence of said trait gene.

### III.3. Double-Strand Break Inducing Enyzme of the Invention

Preferably, deletion / excision of the dual-function marker is realized by homologous recombination between the above specified homology sequences induced by a sequence-specific double-strand break, preferably between the homology sequences which should recombine. General methods are disclosed for example in WO 03/004659, incorporated herein entirely by reference. Various enzyme suitable for induction of sequence-specific double-strand breaks (hereinafter together "endonuclease") are known in the art. The endonuclease may be for example selected from the group comprising:
1. Restriction endonucleases (type II), preferably homing endonucleases as described in detail hereinbelow.
2. Transposases, for example the P-element transposase (Kaufman PD and Rio DC (1992) Cell 69(1):27-39) or AcDs (Xiao YL and Peterson T (2000) Mol Gen Genet 263(1):22-29). In principle, all transposases or integrases are suitable as long as they have sequence specificity (Haren L et al. (1999) Annu Rev Microbiol. 1999;53:245-281; Beall EL, Rio DC (1997) Genes Dev. 11(16):2137-2151).
3. Chimeric nucleases as described in detail hereinbelow.
4. Enzymes which induce double-strand breaks in the immune system, such as the RAG1/RAG2 system (Agrawal A et al. (1998) Nature 394(6695):744-451).
5. Group II intron endonucleases. Modifications of the intron sequence allows group II introns to be directed to virtually any sequence in a double-stranded DNA, where group II introns can subsequently insert by means of a reverse splice mechanism (Mohr et al. (2000) Genes & Development 14:559-573; Guo et al. (2000) Science 289:452- 457). During this reverse splice mechanism, a double-strand break is introduced into the target DNA, the excised intron RNA cleaving the sense strand while the protein portion of the group II intron endonuclease hydrolyses the antisense strand (Guo et al. (1997) EMBO J 16: 6835- 6848). If it is only desired to induce the double-strand break without achieving complete reverse splicing, as is the case in the present invention, it is possible to resort to, for example, group II intron endonucleases which lack the reverse transcriptase activity. While this does not prevent the generation of the double-strand break, the reverse splicing mechanism cannot proceed to completion.

Suitable enzymes are not only natural enzymes, but also synthetic enzymes. Preferred enzymes are all those endonucleases whose recognition sequence is known and which can either be obtained in the form of their proteins (for example by purification) or expressed using their nucleic acid sequence.

In an preferred embodiment a sequence-specific endonuclease is employed for specific induction of double-strand breaks and subsequent induced homologous recombination. The term "Sequence specific DNA-endonuclease" generally refers to all those enzymes which are capable of generating double-strand breaks in double stranded DNA in a sequence-specific manner at one or more recognition sequences. Said DNA cleavage may result in blunt ends, or so called "sticky" ends of the DNA (having a 5'- or 3'-overhang). The cleavage site may be localized within or outside the recognition sequence. Various kinds of endonucleases can be employed. Endonucleases can be, for example, of the Class II or Class IIs type. Class Ils R-M restriction endonucleases catalyze the DNA cleavage at sequences other than the recognition sequence, i.e. they cleave at a DNA sequence at a particular number of nucleotides away from the recognition sequence (Szybalski et al. (1991) Gene 100:13-26). The following may be mentioned by way of example, but not by limitation:
1. Restriction endonucleases (e.g., type II or Ils), preferably homing endonucleases as described in detail hereinbelow.
2. Chimeric or synthetic nucleases as described in detail hereinbelow.

Unlike recombinases, restriction enzymes typically do not ligate DNA, but only cleave DNA. Restriction enzymes are described, for instance, in the New England Biolabs online catalog (www.neb.com), Promega online catalog (www.promega.com) and Rao et al. (2000) Prog Nucleic Acid Res Mol Biol 64:1-63. Within this invention "ligation" of the DNA ends resulting from the cleavage by the endonuclease is realized by fusion by homologous recombination of the homology sequences.

Preferably, the endonuclease is chosen in a way that its corresponding recognition sequences are rarely, if ever, found in the unmodified genome of the target plant organism. Ideally, the only copy (or copies) of the recognition sequence in the genome is (or are) the one(s) introduced by the DNA construct of the invention, thereby eliminating the chance that other DNA in the genome is excised or rearranged when the sequence-specific endonuclease is expressed.

One criterion for selecting a suitable endonuclease is the length of its corresponding recognition sequence. Said recognition sequence has an appropriate length to allow for rare cleavage, more preferably cleavage only at the recognition sequence(s) comprised in the DNA construct of the invention. One factor determining the minimum length of said recognition sequence is **-** from a statistical point of view - the size of the genome of the host organism. In an preferred embodiment the recognition sequence has a length of at least 10 base pairs, preferably at least 14 base pairs, more preferably at least 16 base pairs, especially preferably at least 18 base pairs, most preferably at least 20 base pairs.

A restriction enzyme that cleaves a 10 base pair recognition sequence is described in Huang B et al. (1996) J Protein Chem 15(5):481-9.

Suitable enzymes are not only natural enzymes, but also synthetic enzymes. Preferred enzymes are all those sequence specific DNA-endonucleases whose recognition sequence is known and which can either be obtained in the form of their proteins (for example by purification) or expressed using their nucleic acid sequence.

Especially preferred are restriction endonucleases (restriction enzymes) which have no or only a few recognition sequences - besides the recognition sequences present in the transgenic recombination construct - in the chromosomal DNA sequence of a par ticular eukaryotic organism. This avoids further double-strand breaks at undesired loci in the genome. This is why homing endonucleases are very especially preferred (Review: (Belfort M and Roberts RJ (1997) Nucleic Acids Res 25: 3379-3388; Jasin M (1996) Trends Genet. 12:224-228; Internet: http://rebase.neb.com/rebase/rebase.homing.html). Owing to their long recognition sequences, they have no, or only a few, further recognition sequences in the chromosomal DNA of eukaryotic organisms in most cases.

The sequences encoding for such homing endonucleases can be isolated for example from the chloroplast genome of Chlamydomonas (Turmel M et al. (1993) J Mol Biol 232: 446-467). They are small (18 to 26 kD) and their open reading frames (ORF) have a "codon usage" which is suitable directly for nuclear expression in eukaryotes (Monnat RJ Jr et al. (1999) Biochem Biophys Res Com 255:88-93). Homing endonucleases which are very especially preferably, isolated are the homing endonucleases I-Scel (WO96/14408 I-Scell (Sarguiel B et al. (1990) Nucleic Acids Res 18:5659-5665), I-SceIII (Sarguiel B et al. (1991) Mol Gen Genet. 255:340-341), I-Ceul (Marshall (1991) Gene 104:241-245), I-Crel (Wang J et al. (1997) Nucleic Acids Res 25: 3767-3776), I-Chul (Cote V et al.(1993) Gene 129:69-76), I-Tevl (Chu et al. (1990) Proc Natl Acad Sci USA 87:3574-3578; Bell-Pedersen et al. (1990) Nucleic Acids Res18:3763-3770), I-TevII (Bell-Pedersen et al. (1990) Nucleic Acids Res18:3763-3770), I-TevIII (Eddy et al. (1991) Genes Dev. 5:1032-1041), Endo SceI (Kawasaki et al. (1991) J Biol Chem 266:5342-5347), I-Cpal (Turmel M et al. (1995a) Nucleic Acids Res 23:2519-2525) and I-Cpall (Turmel M et al. (1995b) Mol. Biol. Evol. 12, 533-545).

Further homing endonucleases are detailed in the abovementioned Internet website, and examples which may be mentioned are homing endonucleases such as F-Scel, F-Scell, F-SuvI, F-TevI, F-TevII, I-AmaI, I-AniI, I-CeuI, I-CeuAIIP, I-ChuI, I-CmoeI, I-Cpal, I-Cpall, I-Crel, I-CrepsbIP, I-CrepsbIIP, I-CrepsbIIIP, I-CrepsbIVP, I-CsmI, I-CvuI, I-CvuAIP, I-DdiI, I-DdiII, I-DirI, I-DmoI, I-HmuI, I-HmuII, I-HspNIP, I-LIaI, I-Msol, I-NaaI, 1-NanI, I-NcIIP, I-NgrIP, I-NitI, I-Njal, I-Nsp236IP, I-PakI, I-PboIP, I-PcuIP, I-PcuAI, I-PcuVI, I-PgrIP, I-PobIP, I-PorI, I-PorIIP, I-PpbIP, I-Ppol, I-SPBetaIP, I-ScaI, I-SceI, I-Scell, I-SceIII, I-SceIV, I-SceV, I-SceVI, I-SceVII, I-SexIP, I-SneIP, I-SpomCP, I-SpomIP, I-SpomIIP, I-SquIP, I-Ssp6803I, I-SthPhiJP, I-SthPhiST3P, I-SthPhiS3bP, I-TdelP, I-TevI, I-TevII, I-TevIII, I-UarAP, I-UarHGPA1P, I-UarHGPA13P, I-VinIP, I-ZbiIP, PI-MtuI, PI-MtuHIP, PI-MtuHIIP, PI-PfuI, PI-PfuII, PI-PkoI, PI-PkoII, PI-PspI, PI-Rma43812IP, PI-SPBetaIP, PI-SceI, PI-TfuI, PI-TfuII, PI-ThyI, PI-TliI, PI-TliII, H-DreI, I-BasI, I-Bmol, I-PogI, I-TwoI, PI-MgaI, PI-PabI, PI-PabII.

Preferred in this context are the homing endonucleases whose gene sequences are already known, such as, for example, F-Scel, I-Ceul, I-Chul, I-Dmol, I-Cpal, I-Cpall, I-Crel, I-Csml, F-TevI, F-TevII, I-TevI, I-TevII, I-Anil, I-Cvul, I-DdiI, I-HmuI, I-HmuII, I-Llal, I-NanI, I-Msol, I-NitI, I-Njal, I-Pakl, I-PorI, I-Ppol, I-Scal, I-Ssp6803I, PI-PkoI, PI-PkoII, PI-PspI, PI-TfuI, PI-TliI. Especially preferred are commercially available homing endonucleases such as I-Ceul, I-Scel, I-DmoI, I-Ppol, PI-PspI or PI-SceI. Endonucleases with particularly long recognition sequences, and which therefore only rarely (if ever) cleave within a genome include: I-Ceul (26 bp recognition sequence), PI-PspI (30 bp recognition sequence), PI-SceI (39 bp recognition sequence), I-Scel (18 bp recognition sequence) and I-Ppol (15 bp recognition sequence). The enzymes can be isolated from their organisms of origin in the manner with which the skilled worker is familiar, and/or their coding nucleic acid sequence can be cloned. The sequences of various enzymes are deposited in GenBank. Very especially preferred are the homing endonucleases I-Scel, I-Cpal, I-Cpall, I-Crel and I-Chul. Sequences encoding said nucleases are known in the art and - for example - specified in WO 03/004659 (e.g., as SEQ ID NO: 2, 4, 6, 8, and 10 of WP 03/004659 hereby incorporated by reference).

In an preferred embodiment, the sequences encoding said homing endonucleases can be modified by insertion of an intron sequence. This prevents expression of a functional enzyme in procaryotic host organisms and thereby facilitates -cloning and transformations procedures (e.g., based on *E.coli* or *Agrobacterium).* In plant organisms, expression of a functional enzyme is realized, since plants are able to recognize and "splice" out introns. Preferably, introns are inserted in the homing endonucleases mentioned as preferred above (e.g., into I-Scel or I-Crel).

In some aspects of the invention, molecular evolution can be employed to create an improved endonuclease. Polynucleotides encoding a candidate endonuclease enzyme can, for example, be modulated with DNA shuffling protocols. DNA shuffling is a process of recursive recombination and mutation, performed by random fragmentation of a pool of related genes, followed by reassembly of the fragments by a polymerase chain reaction-like process. See, e.g., Stemmer (1994) Proc Natl Acad Sci USA 91:10747-10751; Stemmer (1994) Nature 370:389-391; and US 5,605,793, US 5,837,458, US 5,830,721 and US 5, 811,238.

Other synthetic endonucleases which may be mentioned by way of example are chimeric nucleases which are composed of an unspecific nuclease domain and a sequence-specific DNA binding domain consisting of zinc fingers (Bibikova M et al. (2001) Mol Cell Biol. 21:289-297). These DNA-binding zinc finger domaines can be adapted to suit any DNA sequence. Suitable methods for preparing suitable zinc finger domaines are described and known to the skilled worker (Beerli RR et al., Proc Natl Acad Sci U S A. 2000; 97 (4):1495-1500; Beerli RR, et al., J Biol Chem 2000; 275(42):32617-32627; Segal DJ and Barbas CF 3rd., Curr Opin Chem Biol 2000; 4(1):34-39; Kang JS and Kim JS, J Biol Chem 2000; 275(12):8742-8748; Beerli RR et al., Proc Natl Acad Sci USA 1998; 95(25):14628-14633; Kim JS et al., Proc Natl Acad Sci USA 1997; 94(8):3616-3620; Klug A, J Mol Biol 1999; 293(2):215-218; Tsai SY et al., Adv Drug Deliv Rev 1998;30(1-3):23-31; Mapp AK et al., Proc Natl Acad Sci USA 2000; 97(8):3930-3935; Sharrocks AD et al., Int J Biochem Cell Biol 1997; 29(12):1371-1387; Zhang L et al., J Biol Chem 2000; 275(43):33850-33860).

The endonuclease is preferably expressed .as a fusion protein with a nuclear localization sequence (NLS). This NLS sequence enables facilitated transport into the nucleus and increases the efficacy of the recombination system. A variety of NLS sequences are known to the skilled worker and described, inter alia, by Jicks GR and Raikhel NV (1995) Annu. Rev. Cell Biol. 11:155-188. Preferred for plant organisms is, for example, the NLS sequence of the SV40 large antigen. Examples are provided in WO 03/060133. However, owing to the small size of many DSBI enzymes (such as, for example, the homing endonucleases), an NLS sequence is not necessarily required. These enzymes are capable of passing through the nuclear pores even without any aid.

In a further preferred embodiment, the activity of the endonuclease can be induced. Suitable methods have been described for sequence-specific recombinases (Angrand PO et al. (1998) Nucl. Acids Res. 26(13):3263-3269; Logie C and Stewart AF (1995) Proc Natl Acad Sci USA 92(13):5940-5944; Imai T et al. (2001) Proc Natl Acad Sci USA 98(1):224-228). These methods employ fusion proteins of the endonuclease and the ligand binding domain for steroid hormone receptor (for example the human androgen receptor, or mutated variants of the human estrogen receptor as described therein). Induction may be effected with ligands such as, for example, estradiol, dexamethasone, 4-hydroxytamoxifen or raloxifen. Some endonucleases are active as dimers (homo- or heterodimers; I-Crel forms a homodimer; I-SecIV forms a heterodimerk) (Wernette CM (1998) Biochemical & Biophysical Research Communications 248(1):127-333)). Dimerization can be designed as an inducible feature, for example by exchanging the natural dimerization domains for the binding domain of a low-molecular-weight ligand. Addition of a dimeric ligand then brings about dimerization of the fusion protein. Corresponding inducible dimerization methods, and the preparation of the dimeric ligands, have been described (Amara JF et al. (1997) Proc Natl Acad Sci USA 94(20): 10618-1623; Muthuswamy SK et al. (1999) Mol Cell Biol 19(10):6845-685; Schultz LW and Clardy J (1998) Bioorg Med Chem Lett. 8(1):1-6; Keenan T et al. (1998) Bioorg Med Chem. 6(8):1309-1335).

Recognition sequences for sequence specific DNA endonuclease (e.g., homing endonucleases) are described in the art. "Recognition sequence" refers to a DNA sequence that is recognized by a sequence-specific DNA endonuclease of the invention. The recognition sequence will typically be at least 10 base pairs long, is more usually 10 to 30 base pairs long, and in most embodiments, is less than 50 base pairs long.

"Recognition sequence" generally refers to those sequences which, under the conditions in a plant cell used within this invention, enable the recognition and cleavage by the sequence specific DNA-endonuclease. The recognition sequences for the respective sequence specific DNA-endonucleases are mentioned in Table 5 hereinbelow by way of example, but not by limitation.

**Table 5: Recognition sequences and organisms of origin for endonucleases (e.g., homing endonucleases; "^" indicates the cleavage site of the sequence specific DNA-endonuclease within a recognition sequence).**

| DSBI Enzyme | Organism of origin | Recognition sequence |
|---|---|---|
| P-Element Transposase | Drosophila | 5'-CTAGATGAAATAACATAAGGTGG |
| I-Anil | Aspergillus nidulans | 5'-TTGAGGAGGTT^TCTCTGTAAATAANNNNNNNNNNNNNNN |
| | | 3'-AACTCCTCCAAAGAGACATTTATTNNNNNNNNNNNNNNN^ |
| I-Ddil | Dictyostelium | 5'-TTTTTTGGTCATCCAGAAGTATAT |
| | discoideumAX3 | 3'-AAAAAACCAG^TAGGTCTTCATATA |
| I-CvuI | Chlorella vulgaris | 5'-CTGGGTTCAAAACGTCGTGA^GACAGTTTGG |
| | | 3'-GACCCAAGTTTTGCA^CACTCTGTCAAACC |
| I-CsmI | Chlamydomonas smithii | 5'-GTACTAGCATGGGGTCAAATGTCTTTCTGG |
| I-CmoeI | Chlamydomonasmoewusii | 5'-TCGTAGCAGCT^CACGGTT |
| | | 3'-AGCATCG^TCGAGTGCCAA |
| I-CreI | Chlamydomonas | 5'-CTGGGTTCAAAACGTCGTGA^GACAGTTTGG |
| | reinhardtii | 3'-GACCCAAGTTTTGCAG^CACTCTGTCAAACC |
| I-Chul | Chlamydomonas | 5'-GAAGGTTTGGCACCTCG^ATGTCGGCTCATC |
| | humicola | 3'-CTTCCAAACGGTG^GAGCTACAGCCGAGTAG |
| I-Cpal | Chlamydomonas | 5'-CGATCCTAAGGTAGCGAA^ATTCA |
| | pallidostigmatica | 3'-GCTAGGATTCCATC^GCTTTAAGT |
| I-call | Chlamydomonas | 5'-CCCGGCTAACTC^TGTGCCAG |
| | pallidostigmatica | 3'-GGGCCGAT^TGAGACACGGTC |
| I-Ceul | Chlamydomonas | 5'-CGTAACTATAACGGTCCTAA^GGTAGCGAA |
| | eugametos | 3'-GCATTGATATTGCCAG^GATTCCATCGCTT |
| I-Dmol | Desulfuro- | 5'-ATGCCTTGCCGGGTAA^GTTCCGGCGCGCAT |
| | coccus mobilis | 3'-TACGGAACGGCC^CATTCAAGGCCGCGCGTA |
| I-SceI | Saccharomyces | 5'-AGTTACGCTAGGGATAA^CAGGGTAATATAG |
| | cerevisiae | 3'-TCAATGCGATCCC^TATTGTCCCATTATATC |
| | | 5'-TAGGGATAA^CAGGGTAAT |
| | | 3'-ATCCC^TATTGTCCCATTA(Core"-Sequence) |
| I-ScelI | S.cerevisiae | 5'-TTTTGATTCTTTGGTCACCC^TGAAGTATA |
| | | 3'-AAAACTAAGAAACCAG^TGGGACTTCATAT |
| I-SceIII | S.cerevisiae | 5'-ATTGGAGGTTTTGGTAAC^TATTTATTACC |
| | | 3'-TAACCTCCAAAACC^ATTGATAAATAATGG |
| I-SceIV | S.cerevisiae | 5'-TCTTTTCTCTTGATTA^GCCCTAA TCTACG |
| | | 3'-AGAAAAGAGAAC^TAATCGGGATTAGATGC |
| I-SceV | S.cerevisiae | 5'-AATAAT1TTCT^TCTTAGTAATGCC |
| | | 3'-TTATTAAAAGAAGAATCATTA^CGG |
| I-SceVI | S.cerevisiae | 5'-GTTATTTAATG^ATTTTAGTAGTTGG |
| | | 3'-CAATAAATTACAAAATCATCA^ACC |
| I-SceVII | S.cerevisiae | 5'-TGTCACATTGAGGTGCACTAGTTATTAC |
| PI-SceI | S.cerevisiae | 5'-ATCTATGTCGGGTGC^GGAGAAAGAGGTAAT |
| | | 3'-TAGATACAGCC^CACGCCTCTTTCTCCATTA |
| F-Scel | S.cerevisiae | 5'-GATGCTGTAGGC^ATAGGCTTGGTT |
| | | 3'-CTACGACA^TCCGTATCCGAACCAA |
| F-SceII | S.cerevisiae | 5'-CTTTCCGCAACA^GTAAAATT |
| | | 3'-GAAAGGCG^TTGTCATTTTAA |
| I-HmuI | Bacillus subtilis | 5'-AGTAATGAGCCTAACGCTCAGCAA |
| | bacteriophage SPO1 | 3'-TCATTACTCGGATTGC^GAGTCGTT |
| I-HmuII | Bacillus subtilis bacteriophage SP82 | |
| I-LlaI | Lactococcus lactis | 5'-CACATCCATAAC^CATATCATTTTT |
| | | 3'-GTGTAGGTATTGGTATAGTAA^AAA |
| I-MsoI | Monomastix species | 5'-CTGGGTTCAAAACGTCGTGA^GACAGTTTGG |
| | | 3'-GACCCAAGTTTTGCAG^CACTCTGTCAAACC |
| I-NanI | Naegleria andersoni | 5'-AAGTCTGGTGCCA^GCACCCGC |
| | | 3'-TTCAGACC^ACGGTCGTGGGCG |
| I-NitI | Naegleria italica | 5'-AAGTCTGGTGCCA^GCACCCGC |
| | | 3'-TTCAGACC^ACGGTCGTGGGCG |
| I-NjaI | Naegleria jamiesoni | 5'-AAGTCTGGTGCCA^GCACCCGC |
| | | 3'-TTCAGACC^ACGGTCGTGGGCG |
| I-PakI | Pseudendoclonium akinetum | 5'-CTGGGTTCAAAACGTCGTGA^GACAGTTTGG |
| | | 3'-GACCCAAGTITTGCAG^CACTCTGTCAAACC |
| I-PorI | Pyrobaculum | 5'-GCGAGCCCGTAAGGGT^GTGTACGGG |
| | organotrophum | 3'-CGCTCGGGCATT^CCCACACATGCCC |
| I-PpoI | Physarum polycephalum | 5'-TAACTATGACTCTCTTAA^GGTAGCCAAAT |
| | | 3'-ATTGATACTGAGAG^AAATTCCATCGGTTTA |
| I-ScaI | Saccharomyces | 5'-TGTCACATTGAGGTGCACT^AGTTATTAC |
| | capensis | 3'-ACAGTGTAACTCCAC^GTGATCAATAATG |
| I- | Synechocystis | 5'-GTCGGGCT^CATAACCCGAA |
| Ssp6803I | species | 3'-CAGCCCGAGTA^TTGGGCTT |
| PI-PfuI | Pyrococcus furiosus Vc1 | 5'-GAAGATGGGAGGAGGG^ACCGGACTCAACTT |
| | | 3'-CTTCTACCCTCC^TCCCTGGCCTGAGTTGAA |
| PI-PfuII | Pyrococcus furiosus Vc1 | 5'-ACGAATCCATGTGGAGA^AGAGCCTCTATA |
| | | 3'-TGCTTAGGTACAC^CTCTTCTCGGAGATAT |
| PI-PkoI | Pyrococcus kodakaraensis KOD1 | 5'-GATTTTAGAT^CCCTGTACC |
| | | 3'-CTAAAA^TCTAGGGACATGG |
| PI-PkoII | Pyrococcus ko- | 5'-CAGTACTACG^GTTAC |
| | dakaraensis | 3'-GTCATG^ATGCCAATG |
| | KOD1 | |
| PI-Pspl | Pyrococcus sp. | 5'-AAAATCCTGGCAAACAGCTATTAT^GGGTAT |
| | | 3'-TTTTAGGACCGTTTGTCGAT^AATACCCATA |
| PI-Tful | Thermococcus | 5'-TAGATTTTAGGT^CGCTATATCCTTCC |
| | fumicolans ST557 | 3'-ATCTAAAA^TCCAGCGATATAGGAAGG |
| PI-Tfull | Thermococcus | 5'-TAYGCNGAYACN^GACGGYTTYT |
| | fumicolans ST557 | 3'-ATRCGNCT^RTGNCTGCCRAARA |
| PI-Thyl | Thermococcus | 5'-TAYGCNGAYACN^GACGGYTTYT |
| | hydrothermalis | 3'-ATRCGNCT^RTGNCTGCCRAARA |
| PI-Tlil | Thermococcus | 5'-TAYGCNGAYACNGACGG^YTTYT |
| | litoralis | 3'-ATRCGNCTRTGNC^TGCCRAARA |
| PI-Tlill | Thermococcus litoralis | 5'-AAATTGCTTGCAAACAGCTATTACGGCTAT |
| I-Tevl | Bacteriophage | 5'-AGTGGTATCAAC^GCTCAGTAGATG |
| | T4 | 3'-TCACCATAGT^TGCGAGTCATCTAC |
| I-Tevll | Bacteriophage | 5'-GCTTATGAGTATGAAGTGAACACGT^TATTC |
| | T4 | 3'-CGAATACTCATACTTCACTTGTG^CAATAAG |
| F-Tevl | Bacteriophage | 5'-GAAACACAAGA^AATGTTTAGTAAANNNNNNNNNNNNNN |
| | T4 | 3'-CTTTGTGTTCTTTACAAATCAMNNNNNNNNNNNNNN^ |
| F-Tevll | Bacteriophage | 5'-TTTAATCCTCGCTTC^AGATATGGCAACTG |
| | T4 | 3'-AAATTAGGAGCGA^AGTCTATACCGTTGAC |
| H-Drel | *E. coli pl-Drel* | 5'-CAAAACGTCGTAA^GTTCCGGCGCG |
| | | 3'-GTTTTGCAG^CATTCAAGGCCGCGC |
| I-Basl | *Bacillus thurin-* | 5' AGTAATGAGCCTAACGCTCAGCAA |
| | *giensis phage Bastille* | 3'- TCATTACGAGTCGAACTCGGATTG |
| I-Bmol | *Bacillus mojavensis* s87-18 | 5'-GAGTAAGAGCCCG^TAGTAATGACATGGC |
| | | 3'-CTCATTCTCG^GGCATCATTACTGTACCG |
| I-Pogl | *Pyrobaculum* | 5'-CTTCAGTAT^GCCCCGAAAC |
| | *oguniense* | 3'-GAAGT^CATACGGGGCTTTG |
| I-Twol | *Staphylococcus* | 5'-TCTTGCACCTACACAATCCA |
| | *aureus phage Twort* | 3'-AGAACGTGGATGTGTTAGGT |
| PI-Mgal | *Mycobacterium* | 5'-CGTAGCTGCCCAGTATGAGTCA |
| | *gastri* | 3'-GCATCGACGGGTCATACTCAGT |
| PI-Pabl | *Pyrococcus abyssi* | 5'-GGGGGCAGCCAGTGGTCCCGTT |
| | | 3'-CCCCCGTCGGTCACCAGGGCAA |
| PI-Pabll | *Pyrococcus abyssi* | 5'-ACCCCTGTGGAGAGGAGCCCCTC |
| | | 3'-TGGGGACACCTCTCCTCGGGGAG |

Also encompassed are minor deviations (degenerations) of the recognition sequence which still enable recognition and cleavage by the sequence specific DNA-endonuclease in question. Such deviations - also in connection with different framework conditions such as, for example, calcium or magnesium concentration - have been described (Argast GM et al. (1998) J Mol Biol 280: 345-353). Also encompassed are core sequences of these recognition sequences and minor deviations (degenerations) in there. It is known that the inner portions of the recognition sequences suffice for an induced double-strand break and that the outer ones are not absolutely relevant, but can codetermine the cleavage efficacy. Thus, for example, an 18bp core sequence can be defined for I-Scel.

### III.4. Combination with other recombination enhancing techniques

In a further preferred embodiment, the efficacy of the recombination system is increased by combination with systems which promote homologous recombination. Such systems are described and encompass, for example, the expression of proteins such as RecA or the treatment with PARP inhibitors. It has been demonstrated that the intrachromosomal homologous recombination in tobacco plants can be increased by using PARP inhibitors (Puchta H et al. (1995) Plant J. 7:203-210). Using these inhibitors, the homologous recombination rate in the DNA constructs of the invention after induction of the sequence-specific DNA double-strand break, and thus the efficacy of the deletion of the transgene sequences, can be increased further. Various PARP inhibitors may be employed for this purpose. Preferably encompassed are inhibitors such as 3-aminobenzamide, 8-hydroxy-2-methylquinazolin-4-one (NU1025), 1,11b-dihydro-[2H]benzopyrano[4,3,2-de]isoquinolin-3-one (GPI 6150), 5-aminoisoquino-linone, 3,4-dihydro-5-[4-(1-piperidinyl)butoxy]-1 (2H)-isoquinolinone, or the compounds described in WO 00/26192, WO 00/29384, WO 00/32579, WO 00/64878, WO 00/68206, WO 00/67734, WO 01/23386 and WO 01/23390.

In addition, it was possible to increase the frequency of various homologous recombination reactions in plants by expressing the E. coli RecA gene (Reiss B et al. (1996) Proc Natl Acad Sci USA 93(7):3094-3098). Also, the presence of the protein shifts the ratio between homologous and illegitimate DSB repair in favor of homologous repair (Reiss B et al. (2000) Proc Natl Acad Sci USA 97(7):3358-3363). Reference may also be made to the methods described in WO 97/08331 for increasing the homologous recombination in plants. A further increase in the efficacy of the recombination system might be achieved by the simultaneous expression of the RecA gene or other genes which increase the homologous recombination efficacy (Shalev G et al. (1999) Proc might be achieved by the simultaneous expression of the RecA gene or other genes which increase the homologous recombination efficacy (Shalev G et al. (1999) Proc Natl Acad Sci USA 96(13)-7398-402). The above-stated systems for promoting homologous recombination can also be advantageously employed in cases where the DNA construct of the invention is to be introduced in a site-directed fashion into the genome of a eukaryotic organism by means of homologous recombination.

### III.5 Initiation of Deletion / Excision

There are various means to appropriately initiate deletion / excision of the dual-function marker. Preferably deletion is only initiated after the dual-function marker has successfully completed its function has negative selection marker resulting in insertion of the DNA construct used in the invention into the genome of the cell or organism to be transformed.

Various means are available for the person skilled in art to combine the deletion/excision inducing mechanism with the DNA construct used in the invention comprising the D-amino oxidase dual-function selection marker. Preferably, a recombinase or endonuclease (hereinafter together also "excision enzyme") employable in the method of the invention can be expressed or combined with its corresponding recombination or recognition site, respectively, by a method selected from the group consisting of
a) incorporation of a second expression cassette for expression of the excision enzyme (the recombinase or sequence-specific endonuclease) operably linked to a plant promoter into said DNA construct, preferably together with said first expression cassette flanked by said sequences which allow for specific deletion,
b) incorporation of a second expression cassette for expression of the excision enzyme (the recombinase or sequence-specific endonuclease) operably linked to a plant promoter into the plant cells or plants used as target material for the transformation thereby generating master cell lines or cells,
c) incorporation of a second expression cassette for expression of the excision enzyme (the recombinase or sequence-specific endonuclease) operably linked to a plant promoter into a separate DNA construct, which is transformed by way of co-transformation with said first DNA construct into said plant cells or transformed into cells already comprising said first DNA construct.

Accordingly the first DNA construct used in the invention and the excision enzyme (e.g., the recombinase or endonuclease) can be combined in a plant organism, cell, cell compartment or tissue for example as follows: mediated transformation). A second expression cassette for the excision enzyme is then combined with said DNA constructs by
a) a second transformation with said second expression cassette, or
b) crossing of the plants comprising the first DNA construct with master plants comprising the expression cassette for the excision enzyme.

2.) The expression cassette encoding for the excision enzyme can be integrated into the DNA construct which already bears the expression cassette for the dual-function marker. It is preferred to insert the sequence encoding the excision enzyme between the sequences allowing for deletion and thus to delete it from the genomic DNA after it has fulfilled its function. Very especially preferably, expression of the endonuclease is inducible in such a case (for example under the control of one of the inducible promoters described hereinbelow), in a development-dependent fashion using a development-dependent promoter, or else excision enzymes are employed whose activity is inducible in order to avoid premature deletion of the dual-function marker prior to its insertion into the genome.
4.) Relying on the co-transformation technique, the expression cassette which ensures the expression of the excision enzyme can be transformed into the cells simultaneously with the first DNA construct, but on a separate vector. Co-transformation can be in each case stable or transient. In such a case, expression of the excision enzyme is preferably inducible (for example under the control of one of the inducible promoters described hereinbelow), in a development-dependent fashion using a development-dependent promoter, or else excision enzymes are employed whose activity is inducible in order to avoid premature deletion of the dual-function marker prior to its insertion into the genome.
5.) Plants expressing the excision enzyme may also act as parent individuals. In the progeny from the crossing between plants expressing the excision enzyme on the one hand and plants bearing the first DNA construct on the other hand, the desired marker deletion (e.g., by double-strand breaks and recombination between the homology sequences) are observed.
6.) Expression of the excision enzyme is also conceivable in a transient transformation approach in which the possibilities 2 to 4 can be exploited.
7.) The excision enzyme can also be introduced into cells comprising or bearing the transgenic recombination construct directly, for example via microinjection, particle bombardment (biolistic method), polyethylene glycol transfection or liposome-mediated transfection. This embodiment is advantageous since no excision enzyme-encoding sequences remains in the genome. Such a method has been described for example by Segal DJ et al. (1995) Proc Natl Acad Sci USA 92:806-810.
8) The excision enzyme may also be generated by introducing the excision enzyme -encoding, in-vitro-generated mRNA into cells (for example via microinjection, particle bombardment (biolistic method) or liposome-mediated transfection). This embodiment is advantageous since no excision-enzyme-encoding sequences will remain In the genome.
9.) The excision enzyme can be introduced into plant cells as a fusion protein with the VIrE2 or VirF protein of an Agrobacterium. Such methods have been described for example for Cre recombinase (Vergunst AC et al (2000) Science. 290: 979-982). If the expression cassette for the fusion protein is located outside the border sequences, it is not inserted

As described above, the excision enzyme can be generated using an expression cassette which comprises the DNA encoding an excision enzyme and is introduced into a plant cell or organism. In this context, the expression cassette for the excision enzyme preferably comprises a nucleic acid sequence encoding an excision enzyme. Various suitable cassettes are described in WO 03/004659.

Also described are DNA constructs comprising both the expression cassette for the dual-function marker (the first expression cassette) and a second expression cassette for the excision enzyme (e.g., an endonuclease or recombinase encoding sequence linked to a plant promoter), preferably in a way that said second expression cassette is together with said first expression cassette flanked by said sequences which allow for specific deletion.

The mechanism of deletion/excision can be induced or activated in a way to prevent pre-mature deletion/excision of the dual-function marker. Preferably, thus expression and/or activity of an preferably employed excision enzyme can be induced, preferably by a method selected from the group consisting of
a) inducible expression by operably linking the sequence encoding said excision enzyme (e.g., a recombinase or endonuclease) to an inducible promoter,

Expression of the polynucleotide encoding the excision enzyme is preferably controlled by an excision promoter, which allows for expression in a timely manner so that the dual-function marker can perform its function as a negative selection marker before getting excised. Suitable promoters are for example described in the German Patent Application DE 03028884.9. Such promoters may have for example expression specificity for late developmental stages like e.g., reproductive tissue. The excision promoter may be selected from one of the following groups of promoters:
a) Pollen-specific promoters such as, for example, the promoter of the B. campestris bgp1 gene (GenBank Acc.-No: X68210; Xu H et al. (1993) Mol Gen Genet 239(1-2):58-65; WO 94/13809), of the Oryza sativa ory s 1 gene (GenBank Acc.-No.: AJ012760; Xu H et al. (1995) Gene 164 (2):255-259), of the pollen-specific maize gene ZM13 (Hamilton DA et al. (1998) Plant Mol Biol 38(4):663-669; US 5,086,169), and of the B.napus gene Bp10 (GenBank Acc.-No.: X64257; Albani D (1992) Plant J 2(3):331-342; US 6,013,859). The promoter of the potato invGF gene from potato (Plant Mol. Biol., 1999, 41:741-751; EMBL Acc No. AJ133765; especially preferred is the promoter described in German Patent Application DE 03028884.9 by SEQ ID NO: 1). The Lcg1 promoter (WO 99/05281; XU H et al. (1999) Proc. Natl. Acad. Sci. USA Vol. 96:2554-2558).
b) Promoters active in ovules (i.e. egg cells)
   The promoter of the Arabidopsis AtSERK1 gene (Somatic Embryogenesis Receptor-Like Kinase 1; At1G71830; Hecht et al. (2001) Plant Physiol 127:803-816). Especially preferred is the promoter described in German Patent Application DE 03028884.9 by SEQ I D NO: 2.
c) Promoters active in zygotes
   The promoter of the Arabidopsis gene Atcyc1A (Cyclin cyc1 gene, type cyclin B; At4g37490; Plant Cell 6: 1763- 1774 (1994)). Especially preferred is the promoter described in German Patent Application DE 03028884.9 by SEQ ID NO: 5.
   USP promoter from *Vicia faba* (Bäumlein H et al. (1991) Mol Gen Genet 225:459-467; Fiedler U et al. (1993) Plant Mol Biol 22:669-679). Especially preferred is the promoter described in German Patent Application DE 03028884.9 by SEQ ID NO: 3. The USP promoter has further activity also in early immature embryos.
d) Promoters active in meristems
   The promoter of the gene erecta (Acc. No. D83257) from Arabidopsis is active in meristematic cells (Yokoyama et al., 1998, Plant J. 15: 301-310). Especially preferred is the promoter described in German Patent Application DE 03028884.9 by SEQ ID NO: 4.

Alternatively, an inducible promoter (which may have ubiquitous expression activity when induced) can be employed with the application of the corresponding inducer at the appropriate time point. Preferably, the inducer for the inducible promoter is applies together with (or briefly before) application of the compound M (e.g., D-isoleucine or D-valine) which is converted by action of the dual-function DAAO marker into a phytotoxic compound.

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Whatever the level of expression is in the absence of the stimulus, expression from any inducible promoter is increased in the presence of the correct stimulus. The preferable situation is where the level of expression increases upon in the presence of the relevant stimulus by an amount effective to alter a phenotypic characteristic (i.e. to express a DAAO and modify tolerance of a D-amino acid). Thus an inducible (or "switchable") promoter may be used which causes a basic level of expression in the absence of the stimulus which level is too low to bring about the desired D-amino acid tolerant or sensitive phenotype (and may in fact be zero). Upon application of the stimulus, expression is increased (or switched on) to a level that causes enhanced D-amino acid tolerance or sensitivity. Many examples of inducible promoters will be known to those skilled in the art.

The inducer can be a physical stimulus like light, heat, drought (low moisture), wounding etc. However, preferably, the inducer is an externally applied chemical substance. It is preferred that the inducible excision promoter only causes functional expression of the endonuclease operably linked if this chemical inducer is externally applied. This leads to a controlled, governable expression and deletion.

Inducible and repressible promoters have been developed for use in plants (Rewiew: Gatz, Annu Rev Plant Physiol Plant Mol Biol 1997, 48:89-108), based on-for example - bacterial repressor (Gatz C & Quail PH (1988) Proc. Natl Acad. Sci. USA 85:1394-1397), animal steroid (Aoyama T & Chua NH (1997) Plant J. 11:605-612; Martinez A et al. (1999) Plant J. 19:97-106) or fungal regulatory elements (Caddick MX et al. (1998) Nature Biotechnol 16:177-180). Promoter systems that are positively regulated by chemical ligands (inducible systems) include the tetracycline(doxycycline)-induced 'Triple-Op' promoter (Gatz C & Quail PH (1988) Proc Natl Acad Sci USA 85:1394-1397; Gatz C et al. (1991) Mol Gen Genet 277:229-237; Gatz C et al. (1992) Plant J. 2:397-404), the glucocorticoid-inducible'GAL4-UAS' promoter (Aoyama T & Chua NH (1997) Plant J. 11:605-612), the ecdysone-inducible 'GRHEcR' promoter (Martinez A et al. (1999) Plant J. 19:97-106) and the ethanol-inducible 'alcA promoter (Caddick MX et al. (1998) Nature Biotechnol 16:177-180). Hormones that have been used to regulate gene expression include, for example, estrogen, tomoxifen, toremifen and ecdysone (Ramkumar and Adler (1995) Endocrinology 136: 536-542). See, also, Gossen and Bujard Proc. Natl. Acad. Sci. USA 89: 5547 (1992); Gossen et al. Science 268: 1766 (1995). In tetracycline-inducible systems, tetracycline or doxycycline modulates the binding of a repressor to the promoter, thereby modulating expression from the promoter.

Inducible expression system can be distinguished into positively and negatively regulated systems. For positively regulated system, expression is induced by adding the corresponding inducer, for negatively regulated systems expression is induced by removing the inducer (better named repressor in this case). An example for a negatively regulated (repressible) system is the tetracycline-inactivated 'Top10' promoter and derivatives (Bohner S et al. (1999) Plant J. 19. 87-95; Weinmann P et al. (1994) Plant J 5:559-569). The Top10 promoter sequence contains a tandem repeat of seven copies of the Tn10 *tet* operator (*tet-OP*) DNA sequence that tightly bind the tetracycline repressor polypeptide TetR (Lederer T et al. (1995) Anal Biochem 232:190-196). This element is fused to a truncated version of e.g., the CaMV 35s promoter (nucleotide positions -53 to 0). The Top10 promoter sequence is recognized by a transactivator that effectively acts as an artificial transcription factor. The transactivator is a chimeric protein fusion between amino acids 1-207 of TetR (Postle K et al. (1984) Nucl Acids Res 12:4849- 4963) and amino acids 363-490 of the transcriptional activation domain (VP16) from the *Herpes simplex* virus (Triezenberg SJ et al. (1988) Genes Dev. 2:718-729), and is labelled 'TetR/VP16' or'tTA (tetracycline transactivator). In the absence of tetracycline, the TetR portion of the tTA binds the tet-OP DNA sequences within the Top10 promoter with high affinity (Hinrichs W et al. (1994) Science 264:418-420; Lederer T et al. (1995) Anal Biochem 232:190-196; Lederer T et al. (1996) Biochemistry 35:7439-7446). This interaction positions the VP16 domain of the tTA in close proximity to the Top10 promoter TATA box, enabling transgene transcription. However, in the presence of tetracycline; the TetR undergoes a conformational change (Hinrichs W et al. (1994) Science 264:418-420; Orth P et al. (1998) J Mol Biol 279: 439-447) that lowers its affinity for the Top10 promoter to non-specific binding levels (Lederer T et al. (1996) Biochemistry 35:7439-7446). Consequently, tTA binding to the Top10 promoter is inhibited, and transcription is switched off. Use of the Top10 promoter system is particularly advantageous in plants. First the Top10 promoter is not functional in the absence of the tTA. Second, transcriptional control is stringent, and tightly controlled by tetracycline. Third, tetracycline has no naturally occurring analogue in plant cells, which might otherwise interfere with promoter regulation. Fourth, the levels of tetracycline used to repress the Top10 promoter are extremely low, normally of the order of 1 µg/ml, and have no discernible secondary effect on plants (Weinmann P et al. (1994) Plant J 5:559-569). Finally, coupling the two transformations required for promoter function can be achieved by transforming the same plants first with the 35S::tTA plasmid construct and then with the Top10 promoter driving the gene of interest, or by mating transgenics which have independently been transformed with the appropriate constructs. The Top10 promoter has been successfully used in *Nicotiana sp.* (Weinmann P et al. (1994) Plant J 5:559-569) and in the moss *Physcomitrella patens* (Zeidler M et al. (1996) Plant Mol Biol 30:199-205).

Alternatively, a positively regulated tetracyclin based inducible expression system can be employed. Especially preferred is the inducible reverse tetracycline system, which allows expression to be up-regulated only upon addition of tetracyclin or a lipid-soluble derivative of tetracycline, doxycyclin (dox, Gossen M. et al. (1995) Science 268:1766-1769; Jiang DM et al. (2001) J. Neurochem. 76(6);1745-1755).

Inducible promoters that are directly responsive to physiologically active stimuli such as heat-shock (prandl R et al. (1995) Plant Mol. Biol. 28:73-82; 1995; Severin K & Schoeffl F (1990) Plant Mol. Biol. 15:827-834), stress signalling molecules (Suehara KI et al. (1996) J. Ferm. Bioeng. 82, 51-55) or heavy metals (McKenzie, M.J., et al. (1998) Plant Physiol. 116,969-977) may also be employed. However, chemically inducible promoter systems are preferred.

Inducibel expression systems have been used in several plant species, including tobacco (Gatz C et al. (1991) Mol. Gen. Genet. 277:229-237), potato (Kumar A et al. (1996) Plant J. 9:147-158), tomato (Thompson AJ & Myatt SC (1997) Plant Mol. Biol. 34:687-692) and Arabidopsis thaliana (Aoyama T & Chua NH (1997) Plant J. 11:605-612).

An additional example includes the ecdysone responsive element (No et al., Proc. Natl. Acad. Sci. USA 93: 3346 (1997)). Other examples of inducible promoters include the glutathione-S-transferase II promoter which is specifically induced upon treatment with chemical safeners such as N, N-diallyl-2,2-dichloroacetamide (PCT Application Nos. WO 90/08826 and WO 93/01294) and the alcA promoter from Aspergillus, which in the presence of the alcR gene product is induced with cyclohexanone (Lockington, et al., Gene 33: 137-149 (1985); Felenbok, et al. Gene 73: 385-396 (1988); Gwynne, et al. Gene 51: 205-216 (1987)) as well as ethanol. Chemical inducers of promoters can be combined with other active chemicals or inert carriers prior to application to an organism. For example, other agronomically useful chemical compositions such as pesticides or fertilizers as well as carriers and solvents can be combined with the inducer.

Further examples for inducible promoters include the PRP1 promoter (Ward et al., Plant. Mol. Biol. 22. (1993), 361-366), a salicylic-acid-inducible promoter (WO 95/19443), a benzenesulfonamide-inducible promoter (EP-A-0388186), a tetracyclin-inducible promoter (Gatz et al., (1992) Plant J. 2, 397-404), an abscisic acid-inducible promoter (EP-A 335528), a salicylic acid-inducible promoter (WO 95/19443) or an ethanol- (Salter MG et al. (1998) Plant J. 16:127-132) or cyclohexanone-inducible (WO 93/21334) promoter may likewise be used.

Other preferred promoters are promoters induced by biotic or abiotic stress, such as, for example, the pathogen-inducible promoter of the PRP1 gene (Ward et al., Plant Mol Biol 1993, 22: 361-366), the tomato heat-inducible hsp80 promoter (US 5,187,267), the potato chill-inducible alpha-amylase promoter (WO 96/12814) or the wound-induced pinII promoter (EP375091).

In an especially preferred embodiment, the excision enzyme is expressed under the control of an inducible promoter. This leads to a controlled, governable expression and deletion - for example in plants -, and any problems caused by a constitutive expression of an excision enzyme are avoided.

Obviously, also the promoter controlling expression of the agronomically valuable trait or selection marker gene may be selected from the promoters preferred as excision promoters.

### III.6 Optional Methods of Preventing Premature Excision of the Excision Construct

It is useful to have a system to maintain the dual-function marker comprising construct of the invention especially during transformation and selection. In general, a control polynucleotide can be introduced into the DNA-construct encoding for the excision enzyme to achieve this goal. The control polynucleotide generally functions either to inhibit expression of the excision enzyme when inhibition is desired (e. g., during transformation and selection; for preferred time frames see above) or to release repression of the excision promoter, thus allowing for expression from the excision promoter. Those of skill will recognize that there are numerous variations for controlling or preventing expression of the excision enzyme in a particular cell or tissue or at a particular developmental stage.

In one aspect, expression from the first excision promoter (i. e. the promoter operably linked to the a first excision enzyme, which excises the dual-function marker) can be countered by a second no-excision promoter. For example, the second no-excision promoter can be operably linked to a repressor gene, which, when expressed, prevents expression of the first excision promoter. Examples of repressors include the tet and lac repressors (Gatz, et al. (1991) Mol Gen Genet 227:229-237). The second no-excision promoter is preferably a promoter which has the highest activity in the tissue used for transformation / selection but has low activity in the reproductive cell (e.g., pollen or oocyte), a precursor cell or tissue of said reproductive cell, or an omnipotent cell (e.g. zygote) resulting from reproduction. Also an inducible promoter can be employed and induction is used during the transformation / selection phase. Such an inducible promoter can be for example a tetracycline (doxycycline) -inducible system, which is induced by tetracycline or doxycycline (see above). Antibiotics like this can be employed during transformation / selection.

Alternatively, the second no-excision promoter can be linked to the polynucleotide encoding the endonuclease in the opposite orientation of the first excision promoter (i.e., from the 3'-end of the coding sequence towards the 5'-end of the sequence), thereby interrupting expression of the DNA cleaving enzyme. In these embodiments, the transcriptional activity of the second no-excision promoter prevents completion of transcripts from the first excision promoter, thereby preventing expression of the excision enzyme.

In other embodiments, an antisense polynucleotide or a polynucleotide producing a double-stranded RNA molecule can be operably linked to the second no-excision promoter, thereby preventing the translation of the DNA cleaving enzyme mRNA. See, e. g., Sheehy et al. (1988) Proc Natl Acad Sci USA 85:8805-8809, and US 4,801,340 for a description of antisense technology; and EP-A1 1 042 462, EP-A1 1 068 311 for a description of the double-stranded RNA interference technique. The antisense or double-stranded RNA molecule should have homology to the nucleotide encoding the excision enzyme to guarantee efficient suppression. In general, antisense technology involves the generation of RNA transcripts that hybridize to a target transcript (i.e., the transcript encoding the sequence-specific endonuclease). Alternatively, the second no-excision promoter can be operably linked to a DNA cleaving enzyme polynucleotide in the sense orientation to induce sense suppression of the gene (see, e.g., Napoli et al. (1990) Plant Cell 2:279-289, US 5,034,323, US 5,231,020, and US 5,283,184 for a description of sense suppression technology).

In some embodiments, aptamer technology can be used to repress expression of the first excision promoter. See, e. g., Hermann et al. (2000) Science 287(5454):820-5; and Famulok et al. (1999) Curr Top Microbiol Immunol 243:123-36. For example, a small oligonucleotide could be developed that only binds and represses the first excision promoter when stabilized by a particular chemical which can be applied when transgenic seed are desired. For example, combinatorial library selections through the systematic evolution of ligands by exponential enrichment (SELEX) technique can be used to identify nucleic acid aptamers that bind with high affinity and specificity to a wide range of selected molecules. See, e. g., Conrad et al. (1995) Mol Divers 1(1):69-78; and Kusser (2000) J Biotechnol 74(1):27-38.

In some embodiments, a multi-tiered excision system is used. For example, the first excision promoter can be interrupted by a second recombination cassette. This second recombination cassette may again be flanked by a second set of homology sequences B and B' flanking a chemically-induced promoter operably linked to a polynucleotide encoding a second sequence-specific DNA cleaving enzyme. In general, this system allows for the transgenic construct to remain intact in the genome (e.g., during transformation and selection) as long as the chemical inducer is not provided. Once the chemical inducer is presented, the second DNA cleaving enzyme is induced and excises its own coding region, induces homologous recombination between B and B', thereby reconstituting the first excision promoter to an intact promoter. Since B remains after excision, B and B' are preferably a sub-sequence of said first excision promoter.

### IV. Additional elements in the DNA constructs of the invention

The DNA construct may - beside the various promoter sequences - comprise additional genetic control sequences. The term "genetic control sequences" is to be understood in the broad sense and refers to all those sequences which affect the making or function of the DNA construct to the invention or an expression cassette comprised therein. Preferably, an expression cassettes according to the invention encompass 5'-upstream of the respective nucleic acid sequence to be expressed a promoter and 3'-downstream a terminator sequence as additional genetic control sequence, and, if appropriate, further customary regulatory elements, in each case in operable linkage with the nucleic acid sequence to be expressed.

Genetic control sequences are described, for example, in "Goeddel; Gene Expression Technology: Methods in Enzymology 185, Academic Press, San Diego, CA (1990)" or "Gruber and Crosby, in: Methods in Plant Molecular Biology and Biotechnolgy, CRC Press, Boca Raton, Florida, eds.: Glick and Thompson, Chapter 7, 89-108" and the references cited therein.

Examples of such control sequences are sequences to which inductors or repressors bind and thus regulate the expression of the nucleic acid. Genetic control sequences furthermore also encompass the 5'-untranslated region, introns or the non-coding 3'-region of genes. It has been demonstrated that they may play a significant role in the regulation of gene expression. Thus, it has been demonstrated that 5'-untranslated sequences are capable of enhancing the transient expression of heterologous genes. Furthermore, they may promote tissue specificity (Rouster J et al. (1998) Plant J 15:435-440.). Conversely, the 5'-untranslated region of the opaque-2 gene suppresses expression. Deletion of the region in question leads to an increased gene activity (Lohmer S et al. (1993) Plant Cell 5:65-73). Genetic control sequences may also encompass ribosome binding sequences for initiating translation. This is preferred in particular when the nucleic acid sequence to be expressed does not provide suitable sequences or when they are not compatible with the expression system.

The expression cassette can advantageously comprise one or more of what are known as enhancer sequences in operable linkage with the promoter, which enable the increased transgenic expression of the nucleic acid sequence. Additional advantageous sequences, such as further regulatory elements or terminators, may also be inserted at The expression cassette can advantageously comprise one or more of what are known as enhancer sequences in operable linkage with the promoter, which enable the increased transgenic expression of the nucleic acid sequence. Additional advantageous sequences, such as further regulatory elements or terminators, may also be inserted at the 3' end of the nucleic acid sequences to be expressed recombinantly. One or more copies of the nucleic acid sequences to be expressed recombinantly may be present in the gene construct. Genetic control sequences are furthermore understood as meaning sequences which encode fusion proteins consisting of a signal peptide sequence. Polyadenylation signals which are suitable as genetic control sequences are plant polyadenylation signals, preferably those which correspond essentially to T-DNA polyadenylation signals from *Agrobacterium tumefaciens.* Examples of particularly suitable terminator sequences are the OCS (octopine synthase) terminator and the NOS (nopaline synthase) terminator.

The DNA-constructs used in the invention may encompass further nucleic acid sequences. Such nucleic acid sequences may preferably constitute expression cassettes. Said further sequences may include but shall not be limited to:
i) Additional negative, positive or counter selection marker as described above.
ii) Report genes which encode readily quantifiable proteins and which, via intrinsic color or enzyme activity, ensure the assessment of the transformation efficacy or of the location or timing of expression. Very especially preferred here are genes encoding reporter proteins (see also Schenborn E, Groskreutz. D. Mol Biotechnol 1999; 13(1):29-44) such as
   - "green fluorescence protein" (GFP) (Chui WL et al., Curr Biol 1996, 6:325-330: Leffel SM et al., Biotechniques. 23(5):912-8, 1997; Sheen et al.(1995) Plant Journal 8(5):777-784; Haseloff et al.(1997) Proc Natl Acad Sci USA 94(6):2122-2127; Reichel et al.(1996) Proc Natl Acad Sci USA 93(12):5888-5893; Tian et al. (1997) Plant Cell Rep 16:267-271; WO 97/41228).
   - Chloramphenicol transferase,
   - luciferase (Millar et al., Plant Mol Biol Rep 1992 10:324-414; Ow et al, (1986) Science, 234:856-859); permits the detection of bioluminescence,
   - β-galactosidase, encodes an enzyme for which a variety of chromogenic substrates are available,
   - β-glucuronidase (GUS) (Jefferson et al., EMBO J. 1987, 6, 3901-3907) or the uidA gene, which encodes an enzyme for a variety of chromogenic substrates.
   - R locus gene product: protein which regulates the production of anthocyanin pigments (red coloration) in plant tissue and thus makes possible the direct analysis of the promotor activity without the addition of additional adjuvants or chromogenic substrates (Dellaporta et al., In; Chromosome Structure and Function: Impact of New Concepts, 18th Stadler Genetics Symposium, 11:263-282, 1988),
   - β-lactamase (Sutcliffe (1978) Proc Natl Acad Sci USA 75:3737-3741), enzyme for a variety of chromogenic substrates (for example PADAC, a chromogenic cephalosporin),
   - xylE gene product (Zukowsky et al. (1983) Proc Natl Acad Sci USA 80:1101-1105), catechol dioxygenase capable of converting chromogenic catechols,
   - alpha-amylase (Ikuta et al. (1990) Bio/technol. 8:241-242),
   - tyrosinase (Katz et al.(1983) J Gene Microbiol 129:2703-2714), enzyme which oxidizes tyrosine to give DOPA and dopaquinone which subsequently form melanine, which is readily detectable,
   - aequorin (Prasher et al.(1985) Biochem Biophys Res Commun 126(3):1259-1268), can be used in the calcium-sensitive bioluminescence detection.

The DNA construct and any vectors derived therefrom may comprise further functional elements. The term "further functional elements" is to be understood in the broad sense. It preferably refers to all those elements which affect the generation, multiplication, function, use or value of said DNA construct or vectors comprising said DNA construct, or cells or organisms comprising the before mentioned. These further functional elements may include but shall not be limited to:
i) Origins of replication which ensure replication of the expression cassettes or vectors according to the invention in, for example, E. coli. Examples which may be mentioned are ORI (origin of DNA replication), the pBR322 ori or the P15A ori (Sambrook et al.: Molecular Cloning. A Laboratory Manual, 2nd ed. Cold Spring Harbor Laboratory Press, Cold Spring Harbor, NY, 1989).
ii) Multiple cloning sites (MCS) to enable and facilitate the insertion of one or more nucleic acid sequences.
iii) Sequences which make possible homologous recombination or insertion into the genome of a host organism.
iv) Elements, for example border sequences, which make possible the Agrobacterium-mediated transfer in plant cells for the transfer and integration into the plant genome, such as, for example, the right or left border of the T-DNA or the vir region.

### V. Construction of the DNA Constructs

Typically, constructs to be introduced into these cells are prepared using transgene expression techniques. Recombinant expression techniques involve the construction of recombinant nucleic acids and the expression of genes in transfected cells.

Molecular cloning techniques to achieve these ends are known in the art. A wide variety of cloning and in vitro amplification methods suitable for the construction of recombinant nucleic acids are well-known to persons of skill. Examples of these techniques and instructions sufficient to direct persons of skill through many cloning exercises are found in Berger and Kimmel, Guide to Molecular Cloning Techniques, Methods in Enzymology, Vol.152, Academic Press, hic., San Diego. CA (Berger) ; T. Maniatis, E.F. Fritsch and J. Sambrook, Molecular Cloning: A Laboratory Manual, Cold Spring Harbor Laboratory, Cold Spring Harbor, NY (1989), in T.J. Silhavy, M.L. Berman and L.W. Enquist, Experiments with Gene Fusions, Cold Spring Harbor Laboratory, Cold Spring Harbor, NY (1984) and Current Protocols in Molecular Biology, F. M. Ausubel et al., eds., Current Protocols, a joint venture between Greene Publishing Associates, Inc. and John Wiley & Sons, Inc., (1998 Supplement). Preferably, the DNA construct according to the invention is generated by joining the abovementioned essential constituents of the DNA construct together in the abovementioned sequence using the recombination and cloning techniques with which the skilled worker is familiar.

Generally, a gene to be expressed will be present in an expression cassette, meaning that the gene is operably linked to expression control signals, e. g., promoters and terminators, that are functional in the host cell of interest. The genes that encode the sequence-specific DNA cleaving enzyme and, optionally, the selectable marker, will also be under the control of such signals that are functional in the host cell. Control of expression is most easily achieved by selection of a promoter. The transcription terminator is not generally as critical and a variety of known elements may be used so long as they are recognized by the cell. The invention contemplates polynucleotides operably linked to a promoter in the sense or antisense orientation.

A DNA construct used in the invention (or a expression cassette or other nucleic acid employed herein) is preferably introduced into cells using vectors into which these constructs or cassettes are inserted. Examples of vectors may be plasmids, cosmids, phages, viruses, retroviruses or else agrobacteria. of plasmids from bacteria. For their proper use, follow the manufacturer's instructions (see, for example, EasyPrep™, FlexiPrep™, both from Pharmacia Biotech; StrataClean™, from Stratagene; and, QIAexpress™ Expression System, Qiagen). The isolated and purified plasmids can then be further manipulated to produce other plasmids, used to transfect cells or incorporated into Agrobacterium tumefaciens to infect and transform plants. Where Agrobacterium is the means of transformation, shuttle vectors are constructed.

However, an expression cassette (e.g., for an excision enzyme) may also be constructed in such a way that the nucleic acid sequence to be expressed (for example one encoding an excision enzyme) is brought under the control of an endogenous genetic control element, for example a promoter, for example by means of homologous recombination or else by random insertion. Such constructs are likewise understood as being expression cassettes for the purposes of the invention. The skilled worker furthermore knows that nucleic acid molecules may also be expressed using artificial transcription factors of the zinc finger protein type (Beerli RR et al. (2000) Proc Natl Acad Sci USA 97(4):1495-500). These factors can be adapted to suit any sequence region and enable expression independently of certain promoter sequences.

### VI. Target Organisms

The methods of the invention are useful for obtaining marker-free plants, or cells, parts, tissues, harvested material derived therefrom. Accordingly, another subject matter of the invention relates to transgenic plants or plant cells comprising in their genome, preferably in their nuclear, chromosomal DNA, the DNA construct according to the invention, and to cells, cell cultures, tissues, parts or propagation material - such as, for example, in the case of plant organisms leaves, roots, seeds, fruit, pollen and the like - derived from such plants.

The term "plant" includes whole plants, shoot vegetative organs/structures (e. g. leaves, stems and tubers), roots, flowers and floral organs/structures (e. g. bracts, sepals, petals, stamens, carpels, anthers and ovules), seeds (including embryo, endosperm, and seed coat) and fruits (the mature ovary), plant tissues (e. g. vascular tissue, ground tissue, and the like) and cells (e. g. guard cells, egg cells, trichomes and the like), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid and hemizygous.

Included within the scope of the invention are all genera and species of higher and lower plants of the plant kingdom. Included are furthermore the mature plants, seed, shoots and seedlings, and parts, propagation material (for example seeds and fruit) and cultures, for example cell cultures, derived therefrom.

Preferred are plants and plant materials of the following plant families: Amaranthaceae, Brassicaceae, Carophyllaceae, Chenopodiaceae, Compositae, Cucurbitaceae, Labiatae, Leguminosae, Papilionoideae, Liliaceae, Linaceae, Malvaceae, Rosaceae, Saxifragaceae, Scrophulariaceae, Solanaceae, Tetragoniaceae.

Annual, perennial, rnonocotyledonous and dicotyledonous plants are preferred host organisms for the generation of transgenic plants. The use of the recombination system, or method according to the invention is furthermore advantageous in all ornamental plants, forestry, fruit, or ornamental trees, flowers, cut flowers, shrubs or turf. Said plant may include - but shall not be limited to - bryophytes such as, for example, Hepaticae (hepaticas) and Musci (mosses); pteridophytes such as ferns, horsetail and clubmosses; gymnosperms such as conifers, cycads, ginkgo and Gnetaeae; algae such as Chlorophyceae, Phaeophpyceae, Rhodophyceae, Myxophyceae, Xanthophyceae, Bacillariophyceae (diatoms) and Euglenophyceae.

Plants for the purposes of the invention may comprise the families of the Rosaceae such as rose, Ericaceae such as rhododendrons and azaleas, Euphorbiaceae such as poinsettias and croton, Caryophyllaceae such as pinks, Solanaceae such as petunias, Gesneriaceae such as African violet, Balsaminaceae such as touch-me-not, Orchidaceae such as orchids, Iridaceae such as gladioli, iris, freesia and crocus, Compositae such as marigold, Geraniaceae such as geraniums, Liliaceae such as drachaena, Moraceae such as ficus, Araceae such as philodendron and many others.

The transgenic plants according to the invention are furthermore selected in particular from among dicotyledonous crop plants such as, for example, from the families of the Leguminosae such as pea, alfalfa and soybean; the family of the Umbelliferae, particularly the genus Daucus (very particularly the species carota (carrot)) and Apium (very particularly the species graveolens dulce (celery)) and many others; the family of the Solanaceae, particularly the genus Lycopersicon, very particularly the species esculentum (tomato) and the genus Solanum, very particularly the species tuberosum (potato) and melongena (aubergine), tobacco and many others; and the genus Capsicum, very particularly the species annum (pepper) and many others; the family of the Leguminosae, particularly the genus Glycine, very particularly the species max (soybean) and many others; and the family of the Cruciferae, particularly the genus Brassica, very particularly the species napus (oilseed rape), campestris (beet), oleracea cv Tastie (cabbage), oleracea cv Snowball Y (cauliflower) and oleracea cv Emperor (broccoli); and the genus Arabidopsis, very particularly the species thaliana and many others; the family of the Compositae, particularly the genus Lactuca, very particularly the species sativa (lettuce) and many others. family of the Compositae, particularly the genus Lactuca, very particularly the species sativa (lettuce) and many others.

The transgenic plants according to the invention are selected in particular among monocotyledonous crop plants, such as, for example, cereals such as wheat, barley, sorghum and millet, rye, triticale, maize, rice or oats, and sugar cane.

Further preferred are trees such as apple, pear, quince, plum, cherry, peach, nectarine, apricot, papaya, mango, and other woody species including coniferous and deciduous trees such as poplar, pine, sequoia, cedar, oak, etc.

Especially preferred are Arabidopsis thaliana, Nicotiana tabacum, oilseed rape, soybean, corn (maize), wheat, linseed, potato and tagetes.

Plant varieties may be excluded, particularly registrable plant varieties according to Plant Breeders Rights. It is noted that a plant need not be considered a "plant variety" simply because it contains stably within its genome a transgene, introduced into a cell of the plant or an ancestor thereof.

In addition to a plant, the present invention provides any clone of such a plant, seed, selfed or hybrid progeny and descendants, and any part or propagule of any of these, such as cuttings and seed, which may be used in reproduction or propagation, sexual or asexual. Also encompassed by the invention is a plant which is a sexually or asexually propagated off-spring, clone or descendant of such a plant, or any part or propagule of said plant, off- spring, clone or descendant.

Plant organisms are furthermore, for the purposes of the invention, other organisms which are capable of photosynthetic activity, such as, for example, algae or cyanobacteria, and also mosses. Preferred algae are green algae, such as, for example, algae of the genus Haematococcus, Phaedactylum tricornatum, Volvox or Dunaliella.

Genetically modified plants according to the invention which can be consumed by humans or animals can also be used as food or feedstuffs, for example directly or following processing known in the art.

### VII. Methods for Introduction Constructs into Target Cells

A DNA construct used in the invention may advantageously be introduced into cells using vectors into which said DNA construct is inserted. Examples of vectors may be plasmids, cosmids, phages, viruses, retroviruses or agrobacteria. In an advantageous embodiment, the expression cassette is introduced by means of plasmid vectors. Preferred vectors are those which enable the stable integration of the expression cassette into the host genome.

The DNA construct can be introduced into the target plant cells and/or organisms by any of the several means known to those of skill in the art, a procedure which is termed transformation (see also Keown et al. (1990) Meth Enzymol 185:527-537). Production of stable, fertile transgenic plants in almost all economically relevant monocot plants is now routine:(Toriyama, et al. (1988) Bio/Technology 6:1072-1074; Zhang et al. (1988) Plant Cell Rep. 7:379-384; Zhang, et al. (1988) Theor Appl Genet 76:835-840; Shimamoto et al. (1989) Nature 5338:274-276; Datta et al. (1990) Bio/Technology 8:736- 740; Christou et al. (1991) Bio/Technology 9:957-962; Peng, et al. (1991) International Rice Research Institute, Manila, Philippines 563-574; Cao et al. (1992) Plant Cell Rep 11:585-591; Li et al. (1993) Plant Cell Rep. 12:250-255; Rathore et al. (1993) Plant Mol Biol 21:871-884; Fromm et al. (1990) Biol/Technology 8:833-839; Gordon-Kamm et al. (1990) Plant Cell 2:603-618; D'Halluin et al. (1992) Plant Cell 4:1495-1505; Walters et al. (1992) Plant Mol Biol 18:189-200; Koziel et al. (1993) Biotechnology 11:194-200; Vasil IK (1994) Plant Mol Biol 25, 925-937; Weeks et al. 11993) Plant Physiology 102, 1077-1084; Somers et al. (1992) Biol/Technology 10, 1589-1594; W0 92/14828).

For instance, the DNA constructs can be introduced into cells, either in culture or in the organs of a plant by a variety of conventional techniques. For example, the DNA constructs can be introduced directly to plant cells using ballistic methods, such as DNA particle bombardment, or the DNA construct can be introduced using techniques such as electroporation and microinjection of a cell. Particle-mediated transformation techniques (also known as "biolistics") are described in, e.g., Klein et al. (1987) Nature 327:70-73; Vasil V et al. (1993) Bio/Technol 11:1553-1558; and Becker D et al. (1994) Plant J 5:299-307. These methods involve penetration of cells by small particles with the nucleic acid either within the matrix of small beads or particles, or on the surface. The biolistic PDS-1000 Gene Gun (Biorad, Hercules, CA) uses helium pressure to accelerate DNA-coated gold or tungsten microcarriers toward target cells. The process is applicable to a wide range of tissues and cells from organisms, including plants. Other transformation methods are also known to those of skill in the art.

Microinjection techniques are known in the art and are well described in the scientific and patent literature. Also, the cell can be permeabilized chemically, for example using polyethylene glycol, so that the DNA can enter the cell by diffusion. The DNA can also be introduced by protoplast fusion with other DNA-containing units such as minicells, cells, lysosomes or liposomes. The introduction of DNA constructs using polyethylene glycol (PEG) precipitation is described in Paszkowski et al. (1984) EMBO J 3:2717. Liposome-based gene delivery is e.g., described in WO 93/24640; Mannino and Gould-Fogerite (1988) BioTechniques 6(7):682-691; US 5,279,833; WO 91/06309; and Felgner et al. (1987) Proc Natl Acad Sci USA 84:7413-7414).

Another suitable method of introducing DNA is electroporation, where the cells are permeabilized reversibly by an electrical pulse. Electroporation techniques are described in Fromm et al. (1985) Proc Natl Acad Sci USA 82:5824. PEG-mediated transformation and electroporation of plant protoplasts are also discussed in Lazzeri P (1995) Methods Mol Biol 49:95-106. Preferred general methods which may be mentioned are the calcium-phosphate-mediated transfection, the DEAE-dextran-mediated transfection, the cationic lipid-mediated transfection, electroporation, transduction and infection. Such methods are known to the skilled worker and described, for example, in Davis et al., Basic Methods In Molecular Biology (1986). For a review of gene transfer methods for plant and cell cultures, see, Fisk et al. (1993) Scientia Horticulturae 55:5-36 and Potrykus (1990) CIBA Found Symp 154:198.

Methods are known for introduction and expression of heterologous genes in both monocot and dicot plants. See, e.g., US 5,633,446, US 5,317,096, US 5,689,052, US 5,159,135, and US 5,679,558; Weising et al. (1988) Ann. Rev. Genet. 22: 421-477. Transformation of monocots in particular can use various techniques including electroporation (e.g., Shimamoto et al. (1992) Nature 338:274-276; biolistics (e.g., EP-A1 270,356); and Agrobacterium (e.g., Bytebier et al. (1987) Proc Natl Acad Sci USA 84:5345-5349). In particular, Agrobacterium mediated transformation is now a highly efficient transformation method in monocots (Hiei et al. (1994) Plant J 6:271-282). Aspects of the invention provide an expression vector for use in such transformation methods which is a disarmed Agrobacterium Ti plasmid, and an Agrobacterium tumefaciens bacteria comprising such an expression vector. The generation of fertile transgenic plants has been achieved using this approach in the cereals rice, maize, wheat, oat, and barley (reviewed in Shimamoto K (1994) Current Opinion in Biotechnology 5:158-162; Vasil et al. (1992) Bio/Technology 10:667-674; Vain et al. (1995) Biotechnology Advances 13(4):653-671; Vasil (1996) Nature Biotechnology 14:702; Wan & Lemaux (1994) Plant Physiol. 104:37-48)

Other methods, such as microprojectile or particle bombardment (US 5,100,792, EP-A-444 882, EP-A-434 616), electroporation (EP-A 290 395, WO 87/06614), microinjection (WO 92/09696, WO 94/00583, EP-A 331 083, EP-A 175 966, Green et al. (1987) Plant Tissue and Cell Culture, Academic Press) direct DNA uptake (DE 4005152, WO 90/12096, US 4,684,611), liposome mediated DNA uptake (e.g. Freeman et al. (1984) Plant Cell Physiol 2 9:1353), or the vortexing method (e.g., Kindle (1990) Proc Natl Acad Sci USA 87:1228) may be preferred where Agrobacterium transformation is inefficient or ineffective.

In particular, transformation of gymnosperms, such as conifers, may be performed using particle bombardment 20 techniques (Clapham D et al. (2000) Scan J For Res 15: 151-160). Physical methods for the transformation of plant cells are reviewed in Oard, (1991) Biotech. Adv. 9 :1-11. Alternatively, a combination of different techniques may be employed to enhance the efficiency of the transformation process, e.g. bombardment with Agrobacterium coated microparticles (EP-A-486234) or microprojectile bombe employed to enhance the efficiency of the transformation process, e.g. bombardment with Agrobacterium coated microparticles (EP-A-486234) or microprojectile bombardment to induce wounding followed by co-cultivation with Agrobacterium (EP-A-4862333).

In plants, methods for transforming and regenerating plants from plant tissues or plant cells with which the skilled worker is familiar are exploited for transient or stable transformation. Suitable methods are especially protoplast transformation by means of polyethylene-glycol-induced DNA uptake, biolistic methods such as the gene gun ("particle bombardment" method), electroporation, the incubation of dry embryos in DNA-containing solution, sonication and microinjection, and the transformation of intact cells or tissues by micro- or macroinjection into tissues or embryos, tissue electroporation, or vacuum infiltration of seeds. In the case of injection or electroporation of DNA into plant cells, the plasmid used does not need to meet any particular requirement. Simple plasmids such as those of the pUC series may be used. If intact plants are to be regenerated from the transformed cells, the presence of an additional selectable marker gene on the plasmid is useful.

In addition to these "direct" transformation techniques, transformation can also be carried out by bacterial infection by means of Agrobacterium tumefaciens or Agrobacterium rhizogenes. These strains contain a plasmid (Ti or Ri plasmid). Part of this plasmid, termed T-DNA (transferred DNA), is transferred to the plant following agrobacterial infection and integrated into the genome of the plant cell.

For Agrobacterium-mediated transformation of plants, the DNA construct used in the invention may be combined with suitable T-DNA flanking regions and introduced into a conventional Agrobacterium tumefaciens host vector. The virulence functions of the A. tumefaciens host will direct the insertion of a transgene and adjacent marker gene(s) (if present) into the plant cell DNA when the cell is infected by the bacteria. Agrobacterium tumefaciens-mediated transformation techniques are well described in the scientific literature. See, for example, Horsch et al. (1984) Science 233:496-498, Fraley et al. (1983) Proc Natl Acad Sci USA 80:4803-4807, Hooykaas (1989) Plant Mol Biol 13:327-336, Horsch RB (1986) Proc Natl Acad Sci USA 83(8):2571-2575), Bevans et al. (1983) Nature 304:184-187, Bechtold et al. (1993) Comptes Rendus De L'Academie Des Sciences Serie III-Sciences De La Vie-Life Sciences 316:1194-1199, Valvekens et al. (1988) Proc Natl Acad Sci USA 85:5536-5540.

The DNA construct is preferably integrated into specific plasmids, either into a shuttle, or intermediate, vector or into a binary vector). If, for example, a Ti or Ri plasmid is to be used for the transformation, at least the right border, but in most cases the right and the left border, of the Ti or Ri plasmid T-DNA is linked with the expression cassette to be introduced as a flanking region. Binary vectors are preferably used. Binary vectors are capable of replication both in E. coli and in Agrobacterium. As a rule, they contain a selection marker gene and a linker or polylinker flanked by the right or left T-DNA flanking sequence. They can be transformed directly into Agrobacterium (Holsters et al. (1978) Mol Gen Genet 163:181-187). The selection marker gene permits the selection of transformed agrobacteria and is, for example, the DAAO gene of the invention, which imparts resistance to D-amino acids like D-alanine (see above). The Agrobacterium, which acts as host organism in this case, should already contain a plasmid with the vir region. The latter is required for transferring the T-DNA to the plant cell. An Agrobacterium thus transformed can be used for transforming plant cells.

Many strains of Agrobacterium tumefaciens are capable of transferring genetic material - for example the DNA construct according to the invention -, such as, for example, the strains EHA101[pEHA101] (Hood EE et al. (1996) J Bacteriol 168(3):1291-1301), EHA105[pEHA105] (Hood et al. 1993, Transgenic Research 2, 208-218). LBA4404[pAL4404] (Hoekema et al. (1983) Nature 303:179-181), C58C1[pMP90] (Koncz and Schell (1986) Mol Gen Genet 204,383-396) and C58C1[pGV2260] (Deblaere et al. (1985) Nucl Acids Res. 13, 4777-4788).

The agrobacterial strain employed for the transformation comprises, in addition to its disarmed Ti plasmid, a binary plasmid with the T-DNA to be transferred, which, as a rule, comprises a gene for the selection of the transformed cells and the gene to be transferred. Both genes must be equipped with transcriptional and translational initiation and termination signals. The binary plasmid can be transferred into the agrobacterial strain for example by electroporation or other transformation methods (Mozo & Hooykaas (1991) Plant Mol Biol 16:917-918). Co-culture of the plant explants with the agrobacterial strain is usually performed for two to three days.

A variety of vectors could, or can, be used. In principle, one differentiates between those vectors which can be employed for the agrobacterium mediated transformation or agroinfection, i.e. which comprise the DNA construct used in the invention within a T-DNA, which indeed permits stable integration of the T-DNA into the plant genome. Moreover, border-sequence-free vectors may be employed, which can be transformed into the plant cells for example by particle bombardment, where they can lead both to transient and to stable expression.

The use of T-DNA for the transformation of plant cells has been studied and described intensively (EP-A1 120 516; Hoekema, In: The Binary Plant Vector System, Offset-drukkerij Kanters B. V., Alblasserdam, Chapter V; Fraley et al. (1985) Crit Rev Plant Sci 4:1-45 and An et al. (1985) EMBO J 4:277-287). Various binary vectors are known, some of which are commercially available such as, for example, pBIN19 (Clontech Laboratories, Inc. USA).

To transfer the DNA to the plant cell, plant explants are cocultured with Agrobacterium tumefaciens or Agrobacterium rhizogenes. Starting from infected plant material (for example leaf, root or stalk sections, but also protoplasts or suspensions of plant cells), intact plants can be regenerated using a suitable medium which may contain, for example, antibiotics or biocides for selecting transformed cells. The plants obtained can then be screened in the presence of the DNA introduced, in this case the DNA construct according to the invention. As soon as the DNA has integrated into the host genome, the genotype in question is, as a rule, stable and the insertion in question is also found in the subsequent generations. Preferably the stably transformed plant is selected using the method of the invention (however other selection schemes employing other selection markers comprised in the DNA construct of the invention may be used). The plants obtained can be cultured and hybridized In the customary fashion. Two or more generations should be grown in order to ensure that the genomic integration is stable and hereditary.

The abovementioned methods are described, for example, in B. Jenes et al., Techniques for Gene Transfer, in: Transgenic Plants, Vol. 1, Engineering and Utilization, edited by SD Kung and R Wu, Academic Press (1993), 128-143 and in Potrykus (1991) Annu Rev Plant Physiol Plant Molec Biol 42:205-225). The construct to be expressed is preferably cloned into a vector which is suitable for the transformation of Agrobacterium tumefaciens, for example pBin19 (Bevan et al. (1984) Nucl Acids Res 12:8711).

The DNA construct can be used to confer desired traits on essentially any plant. One of skill will recognize that after DNA construct is stably incorporated in transgenic plants and confirmed to be operable, it can be introduced into other plants by sexual crossing. Any of a number of standard breeding techniques can be used, depending upon the species to be crossed.

### VIII. Regeneration of Transgenic Plants

Transformed cells, i.e. those which comprise the DNA integrated into the DNA of the host cell, can be selected from untransformed cells preferably using the selection method of the invention. As soon as a transformed plant cell has been generated, an Intact plant can be obtained using methods known to the skilled worker. For example, callus cultures are used as starting material. The formation of shoot and root can be induced in this as yet undifferentiated cell biomass in the known fashion. The shoots obtained can be planted and cultured.

Transformed plant cells, derived by any of the above transformation techniques, can be cultured to regenerate a whole plant which possesses the transformed genotype and thus the desired phenotype. Such regeneration techniques rely on manipulation of certain phytohormones in a tissue culture growth medium, typically relying on a biocide and/or herbicide marker that has been introduced together with the desired nucleotide lian Publishing Company, New York (1983); and in Binding, Regeneration of Plants, Plant Protoplasts, pp. 21-73, CRC Press, Boca Raton (1985). Regeneration can also be obtained from plant callus, explants, somatic embryos (Dandekar et al. (1989) J Tissue Cult Meth 12:145; McGranahan et al. (1990) Plant Cell Rep 8:512), organs, or parts thereof. Such regeneration techniques are described generally in Klee et al. (1987) Ann Rev Plant Physiol 38:467-486. Other available regeneration techniques are reviewed in Vasil et al., Cell Culture and Somatic Cell Genetics of Plants , Vol I, II, and III, Laboratory Procedures and Their Applications, Academic Press, 1984, and Weissbach and Weissbach, Methods for Plant Molecular Biology, Academic Press, 1989.

### IX. Generation of descendants

After transformation, selection and regeneration of a transgenic plant (comprising the DNA construct of the invention) descendants are generated, which - because of the activity of the excision promoter - underwent excision and do not comprise the marker sequence(s) and expression cassette for the endonuclease.

Descendants can be generated by sexual or non-sexual propagation. Non-sexual propagation can be realized by introduction of somatic embryogenesis by techniques well known in the art. Preferably, descendants are generated by sexual propagation / fertilization. Fertilization can be realized either by selfing (self-pollination) or crossing with other transgenic or non-transgenic plants. The transgenic plant of the invention can herein function either as maternal or paternal plant.

After the fertilization process, seeds are harvested, germinated and grown into mature plants. Isolation and identification of descendants which underwent the excision process can be done at any stage of plant development. Methods for said identification are well known in the art and may comprise - for example - PCR analysis, Northern blot, Southern blot, or phenotypic screening (e.g., for an negative selection marker).

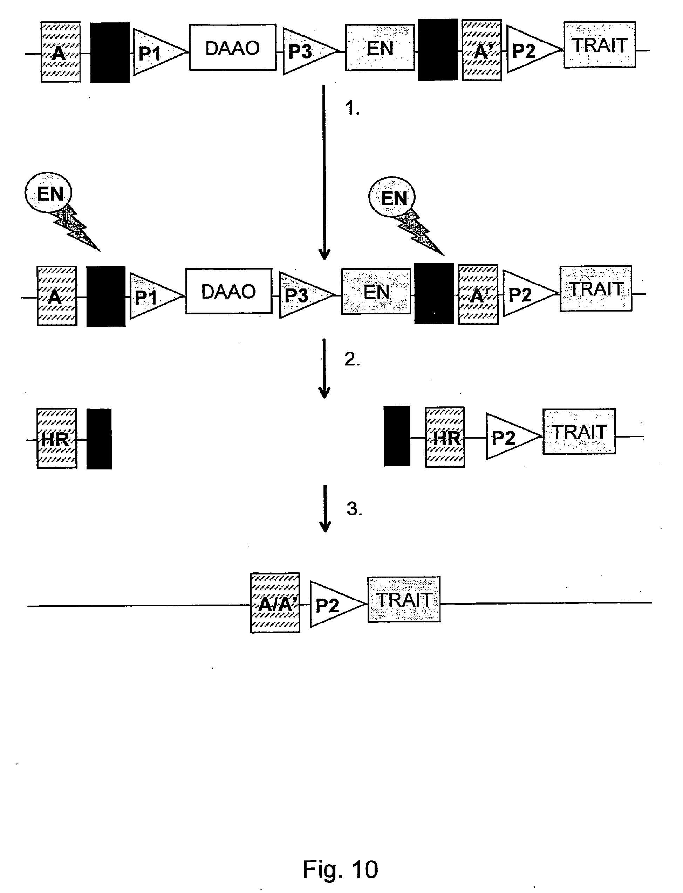

Descendants may comprise one or more copies of the agronomically valuable trait gene. Preferably, descendants are isolated which only comprise one copy of said trait gene. It is another inventive feature of the present invention that multiple insertion (e.g., of a T-DNA) in one genomic location will be reduced to a single insertion event by excision of the redundant copies (Fig. 10).

In a preferred embodiment the transgenic plant made by the process of the invention is marker-free. The terms "marker-free" or "selection marker free" as used herein with respect to a cell or an organisms are intended to mean a cell or an organism which is not able to express a functional selection marker protein (encoded by expression cassette b; as defined above) which was inserted into said cell or organism in combination with the gene encoding for the agronomically valuable trait. The sequence encoding said selection marker protein may be absent in part or -preferably - entirely. Further-with the gene encoding for the agronomically valuable trait. The sequence encoding said selection marker protein may be absent in part or -preferably - entirely. Furthermore the promoter operably linked thereto may be dysfunctional by being absent in part or entirely.

The resulting plant may however comprise other sequences which may function as a selection marker. For example the plant may comprise as a agronomically valuable trait a herbicide resistance conferring gene. However, it is most preferred that the resulting plant does not comprise any selection marker.

Also in accordance with the invention are cells, cell cultures, parts - such as, for example, in the case of transgenic plant organisms, roots, leaves and the like - derived from the above-described transgenic organisms, and transgenic propagation material (such as seeds or fruits).

Genetically modified plants according to the invention which can be consumed by humans or animals can also be used as food or feedstuffs, for example directly or following processing known per se. Here, the deletion of, for example, resistances to antibiotics and/or herbicides, as are frequently introduced when generating the transgenic plants, makes sense for reasons of customer acceptance, but also product safety.

A further subject matter of the invention relates to the use of the above-described transgenic organisms according to the invention and the cells, cell cultures, parts - such as, for example, in the case of transgenic plant organisms, roots, leaves and the like - derived from them, and transgenic propagation material such as seeds or fruits, for the production of food or feedstuffs, pharmaceuticals or fine chemicals. Here again, the deletion of, for example, resistances to antibiotics and/or herbicides is advantageous for reasons of customer acceptance, but also product safety.

Fine chemicals is understood as meaning enzymes, vitamins, amino acids, sugars, fatty acids, natural and synthetic flavors, aromas and colorants. Especially preferred is the production of tocopherols and tocotrienols, and of carotenoids. Culturing the transformed host organisms, and isolation from the host organisms or from the culture medium, is performed by methods known to the skilled worker. The production of pharmaceuticals such as, for example, antibodies or vaccines, is described by Hood EE, Jilka JM. (1999) Curr Opin Biotechnol. 10(4):382-386; Ma JK and Vine ND (1999) Curr Top Microbiol Immunol.236:275-92).

### X.Sequences

| | | | |
|---|---|---|---|
| 1. SEQ ID NO:1: | Nucleic acid sequence encoding D-amino acid oxidase from Rhodosporidium toruloides (Yeast) | | |
| | | | |
| 2. SEQ ID NO:2: | Amino acid sequence encoding D-amino acid oxidase from Rhodosporidium toruloides (Yeast) | | |
| | | | |
| 3. SEQ ID NO:3: | Nucleic acid sequence encoding D-amino acid oxidase from Caenorhabditis elegans | | |
| | | | |
| 4. SEQ ID NO:4: | Amino acid sequence encoding D-amino acid oxidase from Caenorhabditis elegans | | |
| | | | |
| 5. SEQ ID NO:5: | Nucleic acid sequence encoding D-amino acid oxidase from Nectria haematococca | | |
| | | | |
| 6. SEQ ID NO:6: | Amino acid sequence encoding D-amino acid oxidase from Nectria haematococca | | |
| | | | |
| 7. SEQ ID NO:7: | Nucleic acid sequence encoding D-amino acid oxidase from Trigonopsis variabilis | | |
| | | | |
| 8. SEQ ID NO:8: | Amino acid sequence encoding D-amino acid oxidase from Trigonopsis variabilis | | |
| | | | |
| 9. SEQ ID NO:9: | Nucleic acid sequence encoding D-amino acid oxidase from Schizosaccharomyces pombe (fission yeast) | | |
| | | | |
| 10. SEQ ID NO:10: | Amino acid sequence encoding D-amino acid oxidase from Schizosaccharomyces pombe (fission yeast) | | |
| | | | |
| 11. SEQ ID NO:11: | Nucleic acid sequence encoding D-amino acid oxidase from Streptomyces coelicolor A3(2) | | |
| | | | |
| 12. SEQ ID NO:12: | Amino acid sequence encoding D-amino acid oxidase from Streptomyces coelicolor A3(2) | | |
| | | | |
| 13. SEQ ID NO:13: | Nucleic acid sequence encoding D-amino acid oxidase from Candida boidinii | | |
| 14. SEQ ID NO:14: | Amino acid sequence encoding D-amino acid oxidase from Candida boidinii | | |
| | | | |
| 15. SEQ ID NO:15: | Nucleic acid sequence encoding vector daaoScelTetON (length: 12466 bp) | | |
| | | | |
| | Feature | Position (Base No.) | Orientation |
| | LB (Left Border) | 7618 - 7834 | direct |
| | 35SpA (35S terminator) | 7345 - 7549 | complement |
| | ptxA promoter | 6479 - 7341 | complement |
| | rtTA (reverse tetracycline | | |
| | transcription transactivator) | 5418-6425 | complement |
| | OCS-T (OCS terminator) | 5118 - 5343 | complement |
| | nit1 P (Nit1 promoter) | 3217 - 5028 | complement |
| | daao (D-amino acid oxidase) | 2067 - 3173 | complement |
| | nosT (nos terminator) | 1735 - 1990 | complement |
| | pTOP10P (tet regulated promoter) | 1270 - 1660 | complement |
| | IScel (I-Scel endonuclease) | 515 -1222 | complement |
| | I-Sce recognition/cleavage site | 445 -462 | direct |
| | 35SpA (35S terminator) | 196 - 400 | complement |
| | RB (right border) | 38-183 | direct |
| | | | |
| 16. SEQ ID NO:16: | Nucleic acid sequence encoding vector daaoNit-PRecombination (length:12539 bp) | | |
| | | | |
| | Feature | Position (Base No.) | Orientation |
| | LB (Left Border) | 7691 - 7907 | direct |
| | STPT (sTPT promoter) | 7619 - 6302 | complement |
| | GUS (GUS gene) | 6248 - 4251 | complement |
| | 35SpA (35S terminator) | 4176 - 3972 | complement |
| | Nit1 P (Nit1 promoter) | 3882 - 2071 | complement |
| | daao (D-amino acid oxidase) | 2027 - 921 | complement |
| | nosT (nos terminator) | 844 - 589 | complement |
| | I-Sce recognition/cleavage site | 445-462 | direct |
| | 35SpA (35S terminator) | 400 - 196 | complement |
| | RB (right border) | 38-183 | direct |

### XI. BRIEF DESCRIPTION OF THE FIGURES

Fig. 1: Basic Principle of the dual-function selection marker A: A mixture population consisting of wild-type, non-transgenic plants (gray color) and transgenic plants comprising the dual-function marker (black color) is treated with either D-alanine or D-isoleucine. While the toxic effect of D-alanine on non-transgenic plants is detoxified by the transgene-mediated conversion (thereby selectively killing the wild-type plantlets), the non-toxic D-isoleucine is converted by the same enzymatic mechanism into a phytotoxic compound (thereby selectively killing the transgenic plantlets). B: The dual-function of the marker can be employed subsequently for construction of marker-free transgenic plants. While the function as a negative selection marker is utilized to allow for insertion of a transgene comprising a gene of interest (GOI) into a wild-type plant (gray color), the counter-selection-function is employed to subsequently delete the selection marker by combining marker-deletion technology and counter-selection (thereby killing the dual-function marker comprising plantlets (black-color)) resulting in plantlets comprising the GOI but lacking the dual function marker (gray hatching).

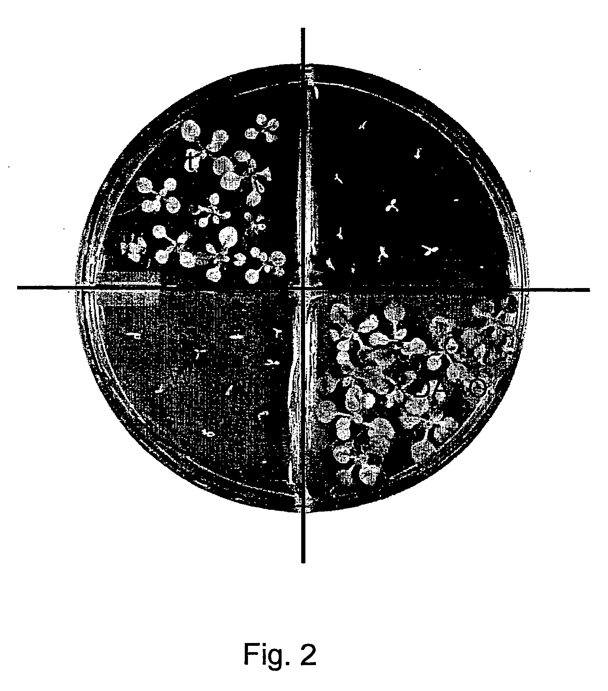

Fig. 2: Wild-type Arabidopsis thaliana plantlets (left side) and transgenic plantlets comprising the dual function marker (DAAO gene from Rhodotorula gracilis) are treated with either 30 mM D-isoleucine (upper side) or 30 mM D- alanine (bottom side). A toxic effect of D-isoleucine on the transgenic plants and D-alanine on the wild-type plants, respectively, can be observed, while no severe damage can be detected on the respective other group, thereby allowing for clear distinguishing and easy selection of either transgenic or wild-type plants.

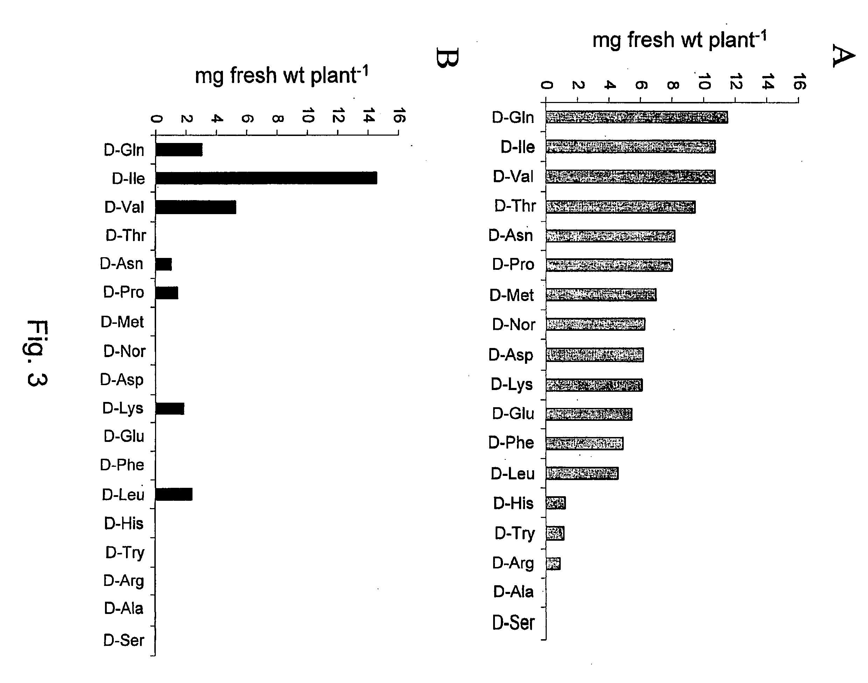

Fig. 3 Effect of various D-amino acids on plant growth. Wild type Arabidopsis thaliana plantlets were grown on half-concentrated Murashige-Skoog medium (0.5% (wt/vol) sucrose, 0.8% (wt/vol) agar) supplemented with the indicated D-amino acid at either 3 mM (Panel A) or 30 mM (Panel B). While D-alanine and D-serine are imposing severe phytotoxic effects even at 3 mM concentrations no significant effects can be observed for D-isoleucine.
Fig. 4 D-amino acid dose responses of *dao1* transgenic and wild-type A. *thaliana.* (a-d) Growth of *dao1* transgenic line 3:7 (white), 10:7 (light gray), 13:4 (gray) and wild-type (black) plants, in fresh weight per plant, on media containing various concentrations of D-serine, D-alanine, D-isoleucine and D-valine in half-strength MS with 0.5% (wt/vol) sucrose and 0.8% (wt/vol) agar. Different concentration ranges were used for different D-amino acids. The plants were grown for 10 d after germination under 16 h photoperiods at 24 °C; *n* = 10 ± s.e.m., except for plants grown on D-isoleucine, where smaller Petri dishes were used, (*n* = 6 ± s.e.m.).
(e-I) Photographs of *dao1* transgenic line 10:7 (e-h) and wild-type plants (i-l), grown for 10 d on the highest concentrations of the D-amino acid shown in the respective graphs above. All pictures have the same magnification. FW, fresh weight. respective graphs above. All pictures have the same magnification. FW, fresh weight.

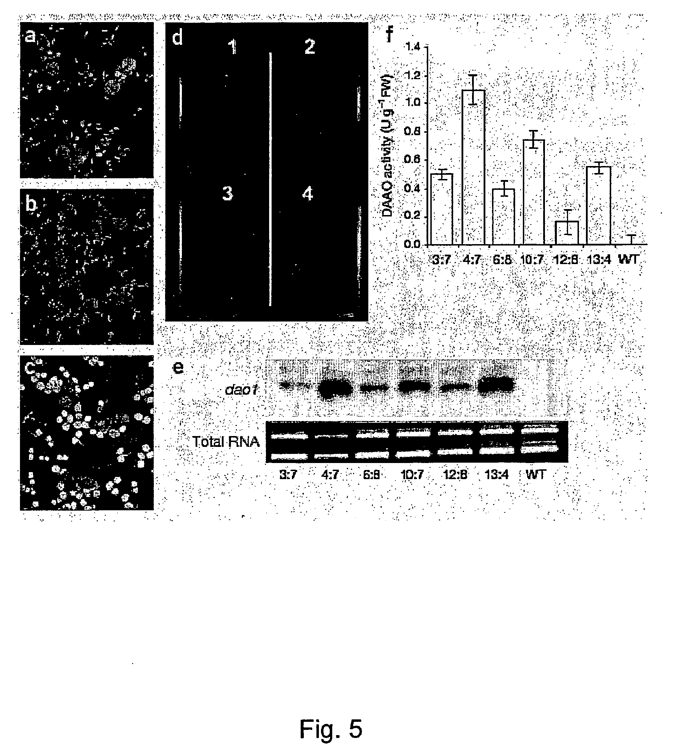

Fig. 5: Selection of primary transformants with the DAAO marker.
(a-c) DAAO T1 seedlings on media containing 3 mM D-alanine (a), 3 mM D-serine (b) and 50 µg ml-1 kanamycin (c). Seeds were surface-sterilized and sown on half-strength MS plates with 0.5% (wt/vol) sucrose, 0.8% (wt/vol) agar and the respective selective compound, then grown for 5 d after germination under 16 h photoperiods at 24 °C.
(d) DAAO transgenic plants grown on soil photographed after selection by spraying with (1) D-alanine and (2) D-serine, and wild-type plants sprayed with (3) D-alanine and (4) D-serine. Eight seeds per plot and treatment were sown on soil, and grown for 7 d after germination before applying the selective treatment, which consisted of spraying with aqueous 50 mM solutions of D-alanine or D-serine with 0.05% Tween 80 on three consecutive days.
(e) Northern blot analysis of *dao1* mRNA levels from six D-serine- and D-alanine-resistant lines and wild-type plants. Ten µg total RNA was loaded per lane and separated on an agarose gel. Ethidium bromide-stained total RNA bands are shown as loading controls. (f) DAAO activity in six transgenic lines and wild type. A unit of DAAO activity is defined as the turnover of one micromole of substrate per minute. Bars represent means ± s.e.m., *n* = 3.

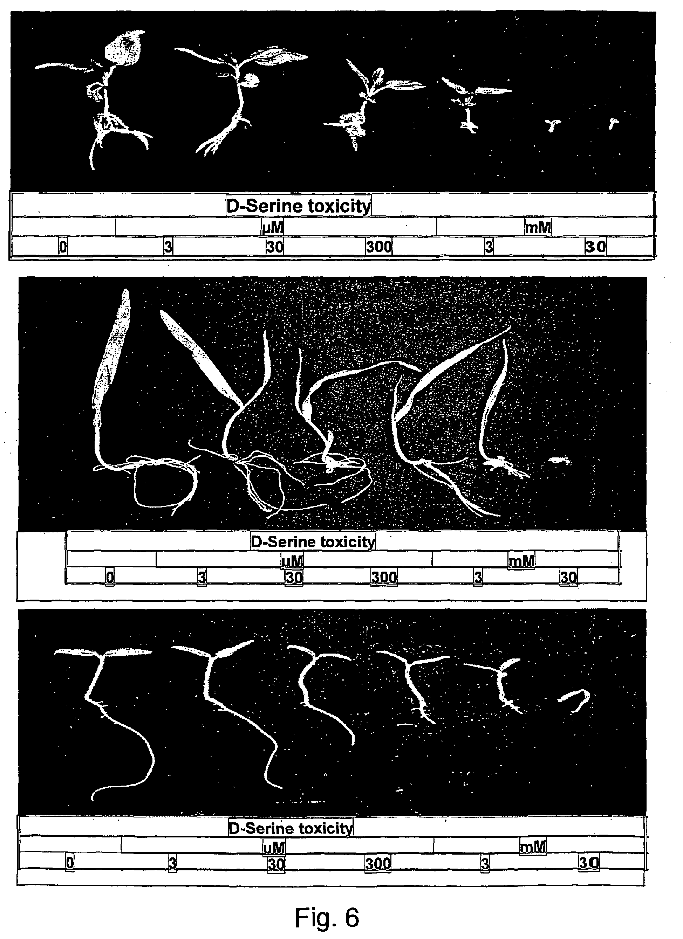

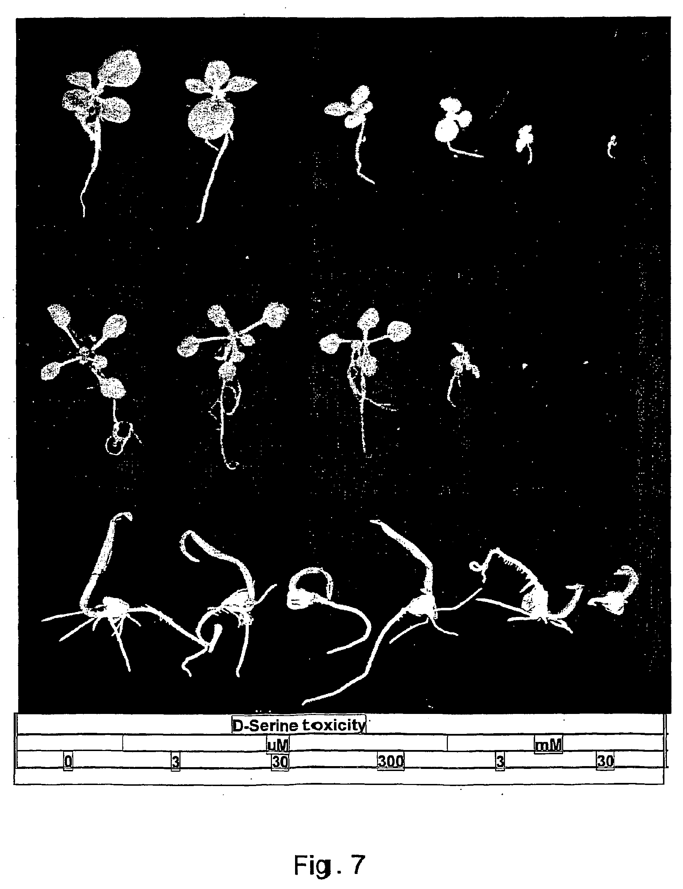

Fig. 6+7 Demonstration of broad applicability of the selection system. D-serine is imposing toxic effects on a variety of different plant species both monocotyledonous and dicotyledonous plants. Effects are demonstrated for popular (Fig. 6A), barley (Fig. 6B), tomato (Fig. 6C), tobacco (Fig. 7A), Arabidopsis thaliana (Fig. 7B), and Corn (Zea mays, Fig. 7C). Similar effects are obtained when using D-alanine instead of D-serine.

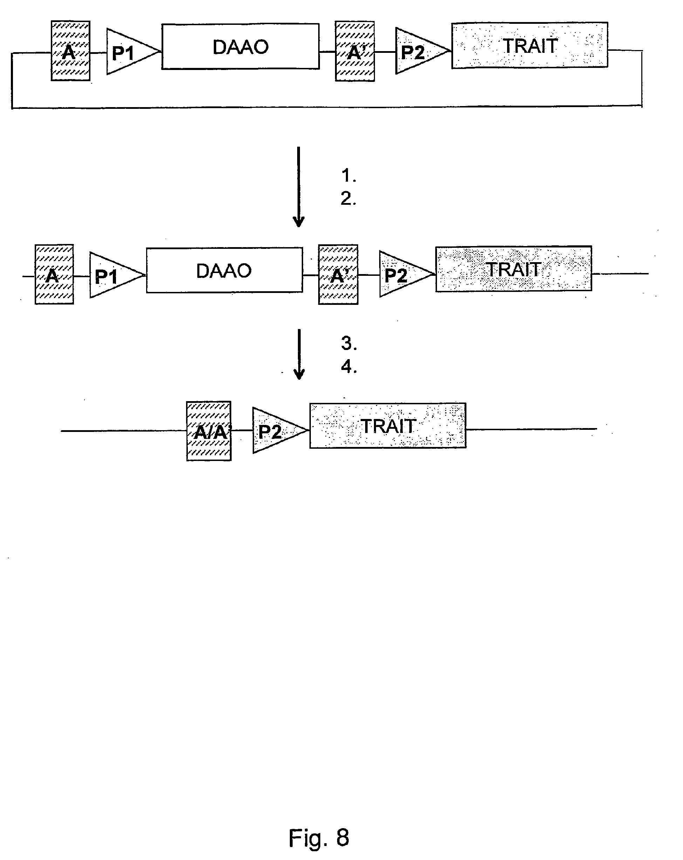

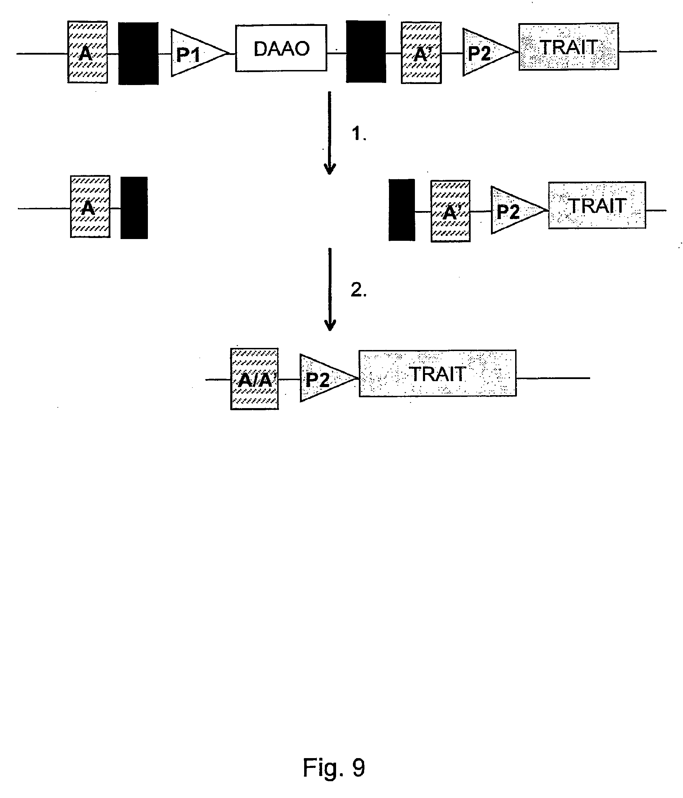

Fig. 8-10: Preferred constructs used in the invention The following abbreviations apply to the figures in general:
A: Sequence A allowing for sequence deletion (e.g., recognition site for recombinase or homology sequence)
A': Sequence A' allowing for sequence deletion (e.g., recognition site for recombinase or homology sequence)
A/A': Sequence as the result of (homologous) recombination of A and A'
DAAO; Sequence encoding a d-amino acid oxidase having dual-function marker activity.
EN: Sequence encoding sequence specific DNA-endonuclease
RSₙ: Recognition sequence for the site-directed induction of DNA double-strand breaks (e.g., S1: First recognition sequence). The recognition sequences may be different (e.g., functioning for different endonucleases) or -preferably - identical (but only placed in different locations).
Rₙ or Sₙ: Part of recognition sequence RSₙ remaining after cleavage
Fig.: 8 Preferred basic construct and method A vector comprising the DNA construct (preferably a circular Agrobacterium binary vector) is employed comprising: A first expression cassette for the dual-function marker (DAAO) under control of a promoter functional in plants (P1) and a second expression cassette for an agronomically valuable trait (TRAIT) also under control of (preferably a different) promoter functional in plants (P2). The first expression cassette is flanked by sequences which allow for specific deletion of said first expression cassette (A and A'). A and A' may be sequences for a sequence-specific recombinase or sequences which allow for homologous recombination between each other. For the later alternative, two identical sequences can be arranged in form of a directed repeat.
The DNA construct is inserted into plant cells (1.) and selection is performed making use of the negative selection function of the dual function marker (2.) e.g., employing D-alanine or D-serine. Thereby plant cells or plants are selected comprising the DNA construct. Based on said plant cells or plants deletion of the first expression cassette is initiated (3.) and selection is performed making use of the counter-selection function of the dual function marker (4.) e.g., employing D-isoleucine or D-valine. Thereby plant cells or plants are selected comprising the second expression cassette but lacking the first expression cassette.
Fig.: 9 Construct mediating marker excision via induced homologous recombination The DNA construct introduced into the plant genome by utilizing the negative selection marker function of the dual-function marker is comprising: A first expression cassette for the dual-function marker (DAAO) under control of a promoter functional in plants (P1) and a second expression cassette for an agronomically valuable trait (TRAIT) also under control of (preferably a different) promoter functional in plants (P2). The first expression cassette is flanked by homology sequences A and A' which allow for homologous recombination between each other, being arranged in form of a directed repeat. Within the DNA construct there is at least one (preferably - as depicted here - two) recognition sequences (RS) (cleavage sites) for a sequence specific endonuclease (RS₁, RS₂). The two sequences may be different (i.e., for different endonucleases) or - preferably - identical. Cleavage at said recognition se- quences (RS₁ and RS₂) is initiated by the corresponding endonuclease (1.) resulting in double-strand breaks, which are "repaired" by homologous recombination between the homologous end-sequences (probably supported by the cellular DNA repair mechanism). The resulting genome still comprises the second expression cassette for the trait but lacks the first expression cassette for the dual-function marker. Selection is performed making use of the counter-selection function of the dual function marker (2.) e.g., employing D-isoleucine or D-valine. Thereby plant cells or plants are selected comprising the second expression cassette but lacking the first expression cassette.
In an preferred embodiment the DNA construct introduced into the plant genome further comprises a third expression cassette for the sequence specific endonuclease (or if recombinases are utilized for the recombinase). The first expression cassette (for the dual-function marker) and the third expression cassette (for the endonuclease) are together flanked by homology sequences A and A' which allow for homologous recombination between each other, being arranged in form of a directed repeat. Within the DNA construct there is at least one (preferably - as depicted here - two) recognition sequences (RS) (cleavage sites) for a sequence specific endonuclease (RS₁, RS₂). The two sequences may be different (i.e., for different endonucleases) or - preferably-identical. Expression of the corresponding endonuclease from the third expression cassette is initiated (1.), resulting in cleavage at said recognition sequences (RS₁ and RS₂) thereby forming in double-strand breaks (2.), which are "repaired" by homologous recombination between the homologous end-sequences (probably supported by the cellular DNA repair mechanism). The resulting genome still comprises the second expression cassette for the trait but lacks the first and third expression cassette. Selection is performed making use of the counter-selection function of the dual function marker (3.) e.g., employing D-isoleucine or D-valine. Thereby plant cells or plants are selected comprising the second expression cassette but lacking the first and third expression cassette.
Preferably the expression of the endonuclease is controllable e.g., by employing an inducible promoter (see below for details).
Fig.: 11 D- amino acids are applicable by spraying procedure DAAO transgenic plants grown on soil photographed after selection by spraying with (1) D-alanine and (2) D-serine, and wild-type plants sprayed with (3) D-alanine and (4) D-serine. Eight seeds per plot and treatment were sown on soil, and grown for 7 d after germination before applying the selective treatment, which consisted of spraying with aqueous 50 mM solutions of D-alanine or D-serine with 0.05% Tween 80 on three consecutive days.
Fig.: 12 Alignment of the catalytic site of various D-amino acid oxidases Multiple alignment of the catalytic site of various D-amino acid oxidases allows for determination of a characteristic sequence motif [LIVM]-[LIVM]-H*-[NHA]-Y-G-x-[GSA]-[GSA]-x-G-x₅-G-x-A, which allows for easy identification of additional D-amino acid oxidases suitable to be employed within the method and DNA-constructs of the invention.
Fig.: 13 Vector map of construct daaoScelTetOn (Seq ID NO: 15) (length: 12466 bp)

| Abbreviation | Feature | Position (Base No.) | Orientation |
|---|---|---|---|
| LB | Left Border | 7618 - 7834 | direct |
| 35SpA | 35S terminator | 7345 - 7549 | complement |
| ptxA promoter | | 6479 - 7341 | complement |
| rtTA | Tet repressor | 5418 - 6425 | complement |
| OCS-T | OCS terminator | 5118 - 5343 | complement |
| nit1P | Nit1 promoter | 3217 - 5028 | complement |
| daao | D-amino acid oxidase | 2067 - 3173 | complement |
| nosT | nos terminator | 1735 - 1990 | complement |
| pTOP10P | tet regulated promoter | 1270 - 1660 | complement |
| ISecI | I-SecI endonuclease | 515 -1222 | complement |
| I-Sce recognition/cleavage site | | 445 - 462 | direct |
| 35SpA | 35S terminator | 196 - 400 | complement |
| RB | right border | 38 - 183 | direct |
| | | | |
| ColE1 | ColE1 origin of replication (E.coli) | | |
| aadA | Spectomycin/Strepotomycin resistance | | |
| repA/pVS1 | repA origin of replication (Agrobacterium) | | |

Furthermore, important restriction sites are indicated with their respective cutting position.
Fig.: 14 Vector map of construct daaoNit-PRecombination (Seq ID NO: 16) (length: 12539 bp)

| Abbreviation | Feature | Position (Base No.) | Orientation |
|---|---|---|---|
| LB | Left Border | 7691 - 7907 | direct |
| STPT | sTPT promoter | 7619 - 6302 | complement |
| GUS | GUS gene | 6248 - 4251 | complement |
| 35SpA | 35S terminator | 4176 - 3972 | complement |
| Nit1P | Nit1 promoter | 3882 - 2071 | complement |
| daao | D-amino acid oxidase | 2027 - 921 | complement |
| nosT | nos terminator | 844 - 589 | complement |
| I-Sce recognition/cleavage site | | 445 - 462 | direct |
| 35SpA | 35S terminator | 400 - 196 | complement |
| RB | right border | 38-183 | direct |

| | |
|---|---|
| ColE1 | ColE1 origin of replication (E.coli) |
| aadA | Spectomycin/Strepotomycin resistance |
| repA/pVS1 | repA origin of replication (Agrobacterium) |

Furthermore, important restriction sites are indicated with their respective cutting position. The GUS gene is comrpising an intron (int).

### XII. Examples

### General methods:

The chemical synthesis of oligonucleotides can be effected for example in the known manner using the phosphoamidite method (Voet, Voet, 2nd edition, Wiley Press New York, pages 896-897). The cloning steps carried out for the purposes of the present invention, such as, for example, restriction cleavages, agarose gel electrophoresis, purification of DNA fragments, the transfer of nucleic acids to nitrocellulose and nylon membranes, the linkage of DNA fragments, the transformation of E. coli cells, bacterial cultures, the propagation of phages and the sequence analysis of recombinant DNA are carried out as described by Sambrook et al. (1989) Cold Spring Harbor Laboratory Press; ISBN 0-87969-309-6. Recombinant DNA molecules were sequenced using an ALF Express laser fluorescence DNA sequencer (Pharmacia, Sweden) following the method of Sanger (Sanger et al., Proc. Natl. Acad. Sci. USA 74 (1977), 5463-5467).

### Example 1: Vector construction and plant transformation

DNA and RNA manipulation were done using standard techniques.

The yeast *R. gracilis* was grown in liquid culture containing 30 mM D-alanine to induce *dao1*, the gene encoding DAAO. Total RNA was isolated from the yeast and used for cDNA synthesis. The PCR primers
5'-ATTAGATCTTACTACTCGAAGGACGCCATG-3' and
5'-ATTAGATCTACAGCCACAATTCCCGCCCTA- 3'
were used to amplify the dao1 gene from the cDNA template by PCR. The PCR fragments were sub-cloned into the pGEM-T Easy vector (Promega) and subsequently ligated into the BamHI site of the CaMV 35S expression cassette of the binary vector pPCV702kana17 giving pPCV702:*dao1*. The vectors were subjected to restriction analysis and sequencing to check that they contained the correct constructs.

### Example 1 a: Transformation of Arabidopsis thaliana

*A. thaliana* plants (ecotype Col-0) were grown in soil until they flowered. *Agrobacterium tumefaciens* (strain GV3101:pMP110 RK) transformed with the construct of interest was grown in 500 mL in liquid YEB medium (5 g/L Beef extract, 1 g/L Yeast Extract (Duchefa), 5 g/L Peptone (Duchefa), 5 g/L sucrose (Duchefa), 0,49 g/L MgSO₄ (Merck)) until the culture reached an OD₆₀₀ 0.8-1.0. The bacterial cells were harvested by centrifugation (15 minutes, 5,000 rpm) and resuspended in 500 mL infiltration solution (5% sucrose, 0.05% SILWET L-77 [distributed by Lehle seeds, Cat.No. VIS-02]).

Flowering *A. thaliana* plants were then transformed by the floral dip method (Clough SJ & Bent AF (1998) Plant J. 16, 735-743 (1998) with the transgenic *Agrobacterium tumefaciens* strain currying the vector described above by dipping for 10-20 seconds into the *Agrobacterium* solution. Afterwards the plants were kept in the greenhouse until seeds could be harvested. Transgenic seeds were selected by plating surface sterilized seeds on growth medium A (4.4g/L MS salts [Sigma-Aldrich]; 0.5g/L MES [Duchefa]; 8g/L Plant Agar [Duchefa]) supplemented with 50 mg/L kanamycin for plants carrying the nptll resistance marker, or 0.3 to 30 mM D-amino acids (as described below) for plants comprising the dual-function marker of the invention. Surviving plants were transferred to soil and grown in the greenhouse.

Lines containing a single T-DNA insertion locus were selected by statistical analysis of T-DNA segregation in the T2 population that germinated on kanamycin or D-amino acid -containing medium. Plants with a single locus of inserted T-DNA were grown and self fertilized. Homozygous T3 seed stocks were then identified by analyzing T-DNA segregation in T3 progenies and confirmed to be expressing the introduced gene by northern blot analyses.

### Example 1b: Agrobacterium-mediated transformation of Brassica napus

*Agrobacterium tumefaciens* strain GV3101 transformed with the plasmid of interest was grown in 50 mL YEB medium (see Example 4a) at 28°C overnight. The *Agrobacterium* solution is mixed with liquid co-cultivation medium (double concentrated MSB5 salts (Duchefa), 30 g/L sucrose (Duchefa), 3.75 mg/l BAP (6-benzylamino purine, Duchefa), 0.5 g/l MES (Duchefa), 0.5 mg/l GA3 (Gibberellic Acid, Duchefa); pH5.2) until OD₆₀₀ of 0.5 is reached. Petiols of 4 days old seedlings of *Brassica napus* cv. Westar grown on growth medium B (MSB5 salts (Duchefa), 3% sucrose (Duchefa), 0.8% oxoidagar (.Oxoid GmbH); pH 5,8) are cut. Petiols are dipped for 2-3 seconds in the *Agrobacterium* solution and afterwards put into solid medium for co-cultivation (co-cultivation medium supplemented with 1.6% Oxoidagar). The co-cultivation lasts 3 days (at 24°C and ~50 µMol/m²s light intensity). Afterwards petiols are transferred to co-cultivation medium supplemented with the appropriate selection agent (18 mg/L kanamycin (Duchefa) for plants comprising the nptII marker kanamycin for plants carrying the nptII resistance marker, or 0.3 to 30 mM D-amino acids; as described below) for plants comprising the dual-function marker of the invention) and 300 mg/L Timetin (Duchefa)

Transformed petioles are incubated on the selection medium for four weeks at 24°C. This step is repeated until shoots appear. Shoots are transferred to A6 medium (MS salts (Sigma Aldrich), 20 g/L sucrose, 100 mg/L myo-inositol (Duchefa), 40 mg/L adeninesulfate (Sigma Aldrich), 500 mg/L MES, 0.0025 mg/L BAP (Sigma), 5 g/L oxoidagar (Oxoid GmbH), 150 mg/L timetin (Duchefa), 0.1 mg/L IBA (indol butyric acid, Duchefa); pH 5,8) supplemented with the appropriate selection agent (18 mg/L kanamycin (Duchefa) for plants comprising the nptII marker kanamycin for plants carrying the nptII resistance marker, or 0.3 to 30 mM D-amino acids; as described below) until they elongated. Elongated shoots are cultivated in A7 medium (A6 medium without BAP) for rooting. Rooted plants are transferred to soil and grown in the greenhouse.

### Example 2: Selection analysis.

T1 seeds of transgenic Arabidopsis plants were surface-sterilized and sown in Petri plates that were sealed with gas-permeable tape. The growth medium was half strength MS19 with 0.5% (wt/vol) sucrose and 0.8% (wt/vol) agar, plus 3 mM D-alanine, 3 mM D-serine or 50 µg/ml kanamycin as the selective agent. Plants were grown for 5 d after germination with a 16 h photoperiod at 24°C. To evaluate the selection efficiency on different substrates, 2,074, 1,914 and 1,810 T1 seeds were sown on D-alanine-, D-serine- and kanamycin-selective plates, respectively, and the number of surviving seedlings was counted (44, 32 and 43, respectively).

### Example 3. Toxicity studies.

To evaluate the toxic action of 3-methyl-2-oxopentanoate and 3-methyl-2-oxobutanoate, wild-type plants were sown on two sets of half strength MS agar plates, each containing one of the compounds in a range of concentrations (0.01-10 mM). Plants were slightly affected by 3-methyl-2- oxopentanoate at 0.1 mM, and total growth inhibition was observed at 1 mM. For 3-methyl-2-oxobutanoate, 5 mM was required for complete inhibition. Further, several attempts were made to probe the nature of D-serine's toxicity. In accordance with studies on *E. coli*, wildtype it was tried to rescue *A. thaliana* grown on lethal concentrations of D-serine through amendments with five potential inhibitors of D-serine toxicity (L-serine, calcium- pantothenate, β-alanine, leucine and threonine) added both separately and in combinations in a very wide range of concentrations (0.001-50 µg ml-1), without any success.

### Example 4: Enzyme assays

Soluble proteins were extracted by shaking 0.1 g samples of plant material that had been finely pulverized in a 1.5 ml Eppendorf tube in 1 ml of 0.1 M potassium phosphate buffer, pH 8. DAAO activity was then assayed as follows. Reaction mixtures were prepared containing 2,120 µl of 0.1 M potassium phosphate buffer, pH 8, 80 µl of crude protein extract and 100 µl of 0.3 M D-alanine. The samples were incubated for 2 h at 30 °C. The enzyme activity was then assessed, by measuring the increase in absorbance at 220 nm (E = 1.090 M⁻¹ cm⁻¹) associated with the conversion of D-alanine to pyruvate, after transferring the test tubes to boiling water for 10 min to stop the reaction. In control reactions, D-alanine was added immediately before boiling. One unit of DAAO activity is defined as the turnover of one micromole of substrate per minute, and activity was expressed per gram plant biomass (fresh weight). The breakdown of D-isoleucine and D-valine in DAAO incubations, and the associated production of 3-methyl-2-oxopentanoate and 3-methyl-2-oxobutanoate, were analyzed by high-performance liquid chromatography. In other respects the reactions were carried out as described above.

### Example 5: Dual-Function Selection Marker

The qualification of the DAAO enzyme as a dual-function selection marker was demonstrated by testing germinated T1 seeds on different selective media. The T-DNA contained both 35S:*dao1* and pNos:*nptII,* allowing D-amino acid and kanamycin selection to be compared in the same lot of seeds.

T1 seeds were sown on medium containing kanamycin (50 µg/ml), D-alanine (3 mM) or D-serine (3 mM), and the transformation frequencies found on the different selective media were 2.37%, 2.12% and 1.67%, respectively (Fig. 5a-c). D-alanine had no negative effect on the transgenic plants, even at a concentration of 30 mM, but at this concentration, D-serine induced significant growth inhibition. Fewer transgenic plants were found after selection on 3 mM D-serine because the compound slightly inhibited the growth of the transgenic plants at this concentration.

Further studies using lower concentrations corroborated this conclusion, and efficient selection using D-serine was achieved on concentrations lower than 1 mM (Fig. 4a). Progeny from the transgenic lines selected on D-serine and D-alanine were later confirmed to be kanamycin resistant, hence ensuring there would be no wild-type escapes from these lines.

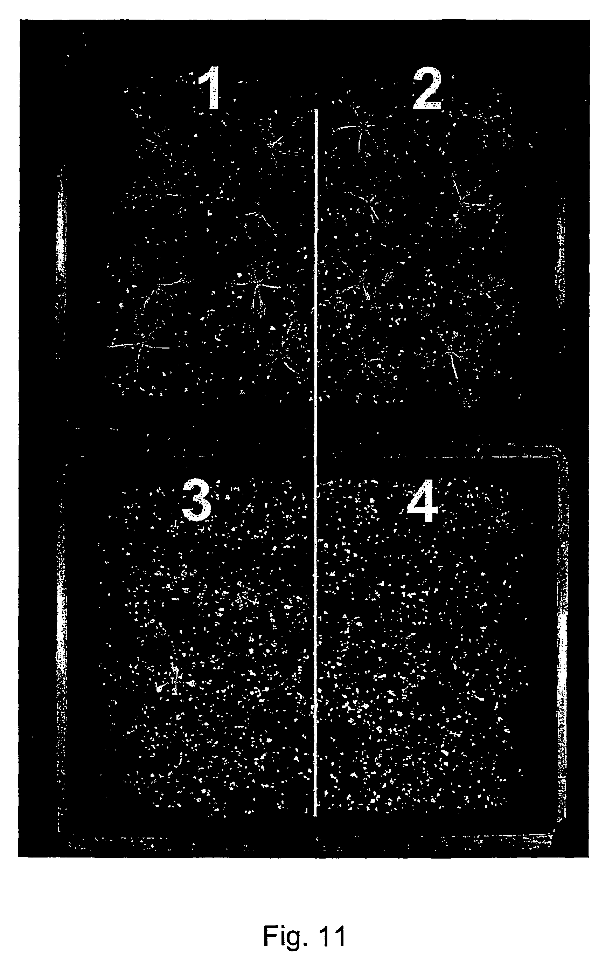

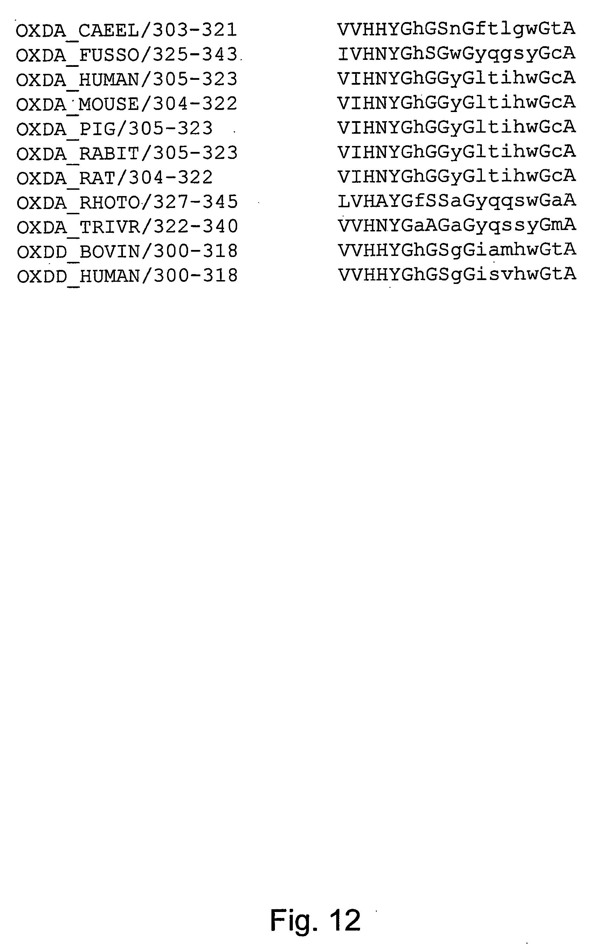

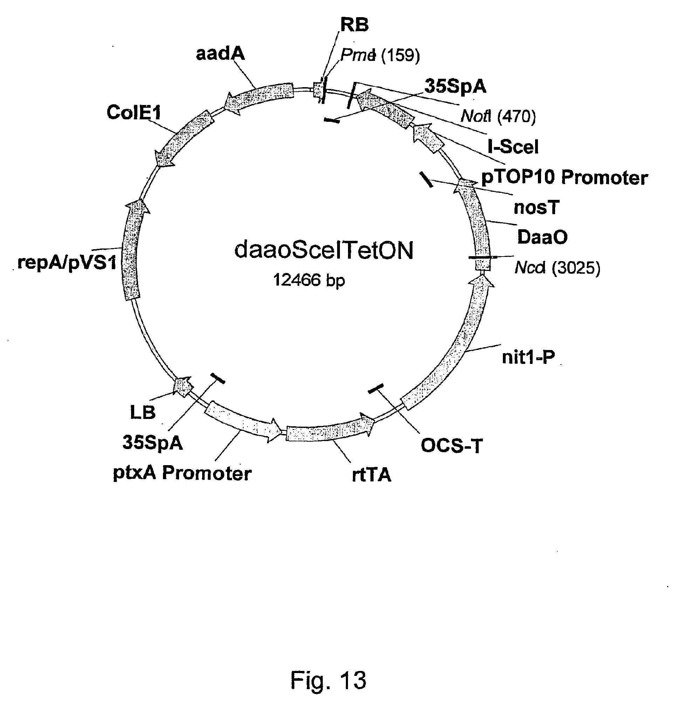

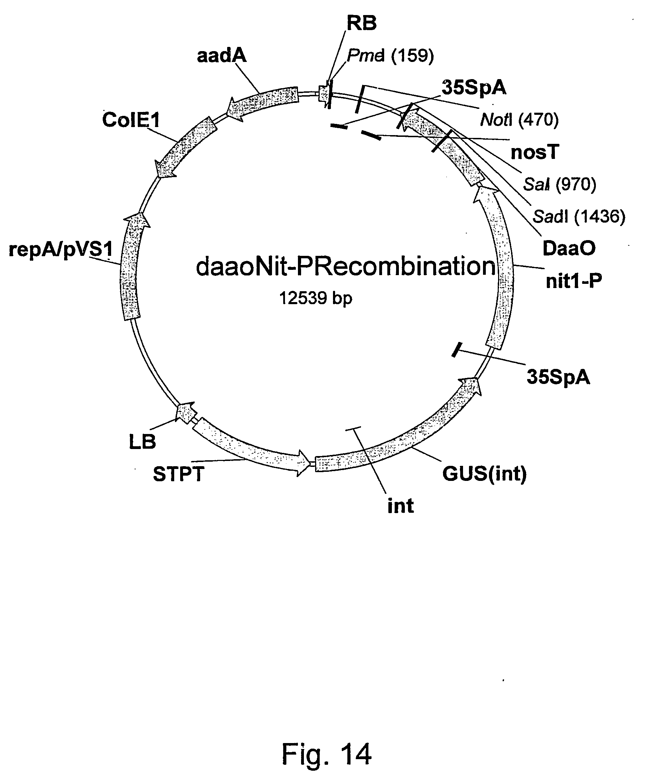

Selection of seedlings on media containing D-alanine or D-serine was very rapid compared to selection on kanamycin. These D-amino acids inhibited growth of wild-type plants immediately after the cotyledons of wild-type plants had emerged. Therefore, transformants could be distinguished from non-transformed plants directly after germination. The difference between wild-type and Transgenic plants after D-amino acid selection was unambiguous, with no intermediate phenotypes. In contrast, intermediate phenotypes are common when kanamycin resistance is used as a selection marker (Fig. 5c). Furthermore, wild-type seedlings were found to be sensitive to sprayed applications of D-serine and D-alanine. One-week-old seedlings were effectively killed when sprayed on three consecutive days with either 50 mM D-serine or D-alanine, although the sensitivity of wild-type plants rapidly decreased with age, presumably because as the cuticle and leaves became thicker, uptake by the leaves was reduced. Transgenic seedlings were resistant to foliar application of D-alanine or D-serine, so selection on soil was possible (Fig. 5d, 11).

Transgenic plants grown under D-alanine and D-serine selection conditions developed normally. Early development of transgenic plants from line 3:7, 10:7 and 13:4 was compared with that of wild-type plants by cultivation on vertical agar plates. No differences in biomass, number of leaves, root length or root architecture were detected for the different sets of plants. Furthermore, soil-cultivated wild-type and transgenic plants (line 10:7) showed no differences in the total number of rosette leaves, number of inflorescences and number of siliqua after 4 weeks of growth.

Also, the phenotypes of 17 individual T1 lines, which were picked for T-DNA segregation, were studied and found indistinguishable from that of wild type when grown on soil. A problem sometimes encountered after selection on antibiotics is the growth lag displayed by transformants. This phenomenon is explained as an inhibitory effect of the antibiotic on the transgenic plants (Lindsey K & Gallois P (1990) J. Exp. Bot. 41, 529-536). However, unlike seedlings picked from antibiotic selection plates, transgenic seedlings picked from D-amino acid selection plates and transferred to soil were not hammered in their growth and development, even temporarily. A possible reason for this difference is that the DAAO scavenging of D-amino acids may effectively remove the D-amino acid in the plants. Furthermore, D-alanine and D-serine may merely provide additional growth substrates, because their catabolic products are carbon and nitrogen compounds that are central compounds in plant metabolism. Quantification of *dao1* mRNA from six independent D-alanine- and D-serine-resistant lines showed a range of different expression levels (Fig. 5e). These different expression levels were mirrored in a range of different DAAO activities (Fig. 5f). In spite of these differences in mRNA levels and enzyme activities, no phenotypic variation associated with the D-serine and D-alanine treatment was found, suggesting that the DAAO marker is effective over a range of expression levels. As described above, D-isoleucine and D-valine were found to inhibit growth of the transgenic plants, but not the wild-type plants.

Therefore, plants containing the construct were tested as described above on two sets of media, one containing D-isoleucine and the other containing D-valine at various concentrations, to assess whether DAAO could also be used as a counter-selection marker. Unambiguous counter-selection selection was achieved when seeds were sown on either D-isoleucine or D-valine at concentrations greater than 10 mM (Fig. 4 c,d).

Thirteen individual lines expressing DAAO were tested for their response to D-isoleucine and all of them were effectively killed, whereas wild-type plants grew well, with no sign of toxicity. Similar results were obtained for D-valine, although this compound was found to have a moderately negative effect on wild-type plants at higher concentrations (Fig. 4 d). The keto acid produced in DAAO catabolism of D-isoleucine is the same as that formed when L-isoleucine is metabolized by the endogenous branched-chain amino acid transaminase [EC: 2.6.1.42], namely 3-methyl-2-oxopentanoate (Kyoto Encyclopedia of Genes and Genomes, metabolic pathway website, http://www.genome.ad.jp/ kegg/metabolism.html).

Presumably endogenous transaminase may be specific for the L-enantiomer, so the corresponding D-enantiomer is not metabolized in wildtype plants, but only in plants expressing DAAO. The negative effects of L-isoleucine (but not of the D-form) observed on wildtype plants, supports this speculation. Incubation of cell-free extracts from dao1 transgenic line 10:7 with D-isoleucine and D-valine resulted in 15-fold and 7-fold increases in production of 3-methyl-2-oxopentanoate and 3-methyl-2-oxobutanoate, respectively, compared to extracts of wild-type plants. Further, 3-methyl-2-oxopentanoate and 3- methyl-2-oxobutanoate impaired growth of *A. thaliana,* corroborating the suggestion that these compounds, or products of their metabolism, are responsible for the negative effects of D-isoleucine and D-valine on the transgenic plants.

The toxicity of some D-amino acids on organisms is not well understood, and has only occasionally been studied in plants (Gamburg KZ & Rekoslavskaya NI (1991) Fiziologiya Rastenii 38, 1236-1246). Apart from A. *thaliana,* we have also tested the susceptibility of other plant species to D-serine, including poplar, tobacco, barley, maize, tomato and spruce. We found all tested species susceptible to D-serine at concentrations similar to those shown to be toxic for A. *thaliana.* A proposed mechanism for D-serine toxicity in bacteria is competitive inhibition of a-alanine coupling to pantoic acid, thus inhibiting formation of pantothenic acid (Cosloy SD & McFall E (1973) J. Bacteriol. 114, 685-694). It is possible to alleviate D-serine toxicity in D-serine- sensitive strains of *Escherichia coli* by providing pantothenic acid or â-alanine in the medium, but D-serine toxicity in *A. thaliana* could not be mitigated using these compounds. A second putative cause of D-amino acid toxicity is through competitive binding to tRNA. Knockout studies of the gene encoding D-Tyr-tRNATyr deacylase in E. coli have shown that the toxicity of D-tyrosine increases in the absence of deacylase activity (Soutourina J et al. (1996) J. Biol. Chem. 274, 19109-19114), indicating that D-amino acids interfere at the tRNA level. Genes similar to that encoding bacterial deacylase have also been identified in *A. thaliana* (Soutourina J et al. (1996) J. Biol. Chem. 274, 19109-19114), corroborating the possibility that the mode of toxic action of D-amino acids might be through competitive binding to tRNA.

### Example 6: Constructs useful for self-excising expression cassettes using I-Scel

Two expression constructs are constructed for carrying out the present invention (SEQ ID NO: 15, 16). The backbone of both plasmid constructs (pSUN derivative) contains origins for the propagation in *E. coli* as well as in *Agrobacterium* and an aadA expression cassette (conferring spectinomycin and streptomycin resistance) to select for transgenic bacteria cells. The sequences for constructing the DNA constructs are amplified incorporating the appropriate restriction sites for subsequent cloning by PCR. Cloning was done by standard methods as described above. The sequence of the constructs is verified by DNA sequence analysis.

### Example 6a: DAAO driven by constitutive Nitrilase Promoter

The first DNA construct (SEQ ID NO: 16) comprises an expression cassette for the D-amino acid oxidase (DAAO) from Rhodotorula gracilis under control of the Arabidopsis thaliana Nitrilase promoter. The DAAO cassette is flanked by a direct repeat of the 35S terminator functioning both as transcription terminator of the DAAO expression cassette and as homology sequences.

Further comprised is a expression cassette for the β-glucuronidase which may function as a substitute for an agronomically valuable trait under control of the *Arabidopsis* sTPT promoter (i.e. TPT promoter truncated version, WO 03/006660; SEQ ID NO: 27 cited therein), and the CaMV 35S terminator.

### Example 6b: Self-excisable DAAO cassette

The second DNA (SEQ ID NO: 15) comprises an expression cassette for the D-amino acid oxidase (DAAO) from Rhodotorula gracilis under control of the Arabidopsis thaliana Nitrilase promoter. The DNA construct further comprises a Tet on expression system. This allows for induced expression of the I-Sce-I homing endonuclease which is placed under control of a Tet-regulatable promoter. The system further requires expression of the Tet-repressor, which is realized under control of the constitutive ptXA promoter from Pisum sativa.

Both the sequences encoding the DAAO cassette, the I-Sce-I expression cassette, and the rtTA expression cassette (for the reverse tetracycline responsive repressor) are flanked by a direct repeat of the 35S terminator functioning both as transcription terminator of the I-Sce-I expression cassette and as homology sequences.

### Example 7: Use of the constructs for the method of the invention

### Example 7.1: Co-transformation

Arabidopsis thaliana plants are transformed as described above with a mixture of DNA construct I (binary vector SEQ ID NO: 16) and a second binary vector comprising a GFP (green fluorescence protein) expression cassette. In a first selection process transgenic plants are selected comprising both constructs by employing D-alanine mediated selection. 3 mM and 30 mM D-alanine are used.

D-alanine resistant plants comprising the first DNA construct (detectable by GUS staining) also comprising the gfp gene (as assessed by green fluorescence) are isolated and crossed with wild-type plants. Resulting seeds are used for a second counter-selection process, wherein said seeds are germinated on D-isoleucine comprising medium (comprising either 3 mM or 30 mM D-isoleucine). D-isoleucine resistant plants - comprising the gfp gene - can be easily selected.

### Example 7.2: Marker excision

Arabidopsis thaliana plants are transformed as described above with a mixture of DNA construct I (binary vector SEQ ID NO: 16). In a first selection process transgenic plants are selected comprising construct I by employing D-alanine mediated selection. 3 mM and 30 mM D-alanine are used.

D-alanine resistant plants comprising the first DNA construct (detectable by GUS staining) are isolated and crossed with a transgenic master plant comprising a transgenic expression cassette for the I-Sce-I homing endonuclease under control of a constitutive promoter (as described in WO 03/004659). Resulting seeds are used for a second counter-selection process, wherein said seeds are germinated on D-isoleucine comprising medium (comprising either 3 mM or 30 mM D-isoleucine). D-isoleucine resistant plants still comprising the GUS-expression cassette can be easily selected.

### Example 7.3: Use of a self-excisable marker cassette

Arabidopsis thaliana plants are transformed as described above with a mixture of DNA construct II (binary vector SEQ ID NO: 15). In a first selection process transgenic plants are selected comprising construct II by employing D-alanine mediated selection. 3 mM and 30 mM D-alanine are used.

D-alanine resistant plant cells com prising the DNA construct II are isolated and further cultivated on medium lacking D-alanine. Doxycycline (Sigma; 1 to 5 µg/ml) is added for induction of the marker excision process and cells are incubated for 24 to 48 h on said induction medium. Subsequently cells are further incubated for 3 to 5 days on medium lacking the inducer and D-amino acids (to allow for reduction of DAAO protein levels from prior expression). The resulting cells are used for a second counter-selection process, wherein said cells are further selected on D-isoleucine comprising medium (comprising either 3 mM or 30 mM D-isoleucine). Selected D-isoleucine resistant cells are regenerated into fertile plants and assessed for their transgenic status. By PCR mediated analysis it can be demonstrated that the region flanked by the 35S terminator sequences was accurately excised from the plant genome deleting both the I-SceI expression cassett, the DAAO expression cassette, and the rtTA expression cassette (for the reverse tetracycline responsive repressor)

## Claims

1. A method for producing a transgenic plant comprising:
i) transforming a plant cell with a first expression cassette comprising a nucleic acid sequence encoding a D-amino acid oxidase operably linked with a promoter allowing expression in plant cells or plants, in combination with at least one second expression cassette suitable for conferring to said plant an agronomically valuable trait, and
ii) providing at least one first compound X, wherein compound X is either D-alanine or D-serine , which is phytotoxic against plant cells not functionally expressing said D-amino acid oxidase, wherein said compound X can be metabolized by said D-amino acid oxidase into one or more compound(s) Y which are non-phytotoxic or less phytotoxic than compound X, and
iii) treating plants in soil comprising transformed plant cells of step i) with said first compound X in a phytotoxic concentration for three consecutive days, wherein if compound x is D-alanine the concentration is 5 to 100 mM, and if compound X is D-serine the concentrations is 5 to 80 mM: or treating seeds comprising transformed plant cells of step i) with said first compound X in a phytotoxic concentration for five days during germination on media, wherein if compound x is D-alanine the concentration is 0,1 mM to 100 mM and if compound X is D-serine the concentrations is 0,1 to 10 mM, and selecting plant cells comprising in their genome both said first and said second expression cassette, wherein said first expression cassette is conferring resistance to said transformed plant cells against said compound X by expression of said D-amino acid oxidase, and
iv) providing at least one second compound M, which is non-phytotoxic or moderately phytotoxic against plant cells not functionally expressing said D-amino acid oxidase, wherein said compound M can be metabolized by said D-amino acid oxidase into one or more compound(s) N which are phytotoxic or more phytotoxic than compound M, and
v) separation of said first expression cassette and said second expression cassette and treating resulting said plant cells with said second compound M in a concentration toxic to plant cells still comprising said first expression cassette, and selecting plant cells comprising said second expression cassette but lacking said first expression cassette,
whereby M is comprising a structure selected from the group consisting of D-valine, D-leucine, D-lysine, and D-proline, or D-isoleucine applied via the cell culture medium in concentrations of about 10 mM to about 30 mM or D-asparagine or D-glutamine applied via the cell culture medium in concentrations of about 3 mM to about 20 mM.

2. The method of Claim 1, wherein at least 1914 Arabidopsis seeds per plate are treated during germination of the seeds of step iii)

3. The method of claims 1 or 2, wherein said first expression cassette for said D-amino acid oxidase and said second expression cassette for said agronomically valuable trait are
a) both comprised in one DNA construct and combination is broken by deletion or excision of said first expression cassette for said D-amino acid oxidase, or
b) are comprised on separate DNA constructs which are transformed in combination by co-transformation into said plant cells, and combination is broken by subsequent segregation of the two expression cassettes.

4. The method of Claim 1 to 3, wherein said method for producing a transgenic plant comprises the steps of:
i) transforming a plant cell with a first DNA construct comprising
a) a first expression cassette comprising a nucleic acid sequence encoding a D-amino acid oxidase operably linked with a promoter allowing expression in plant cells or plants, wherein said first expression cassette is flanked by sequences which allow for specific deletion of said first expression cassette, and
b) at least one second expression cassette suitable for conferring to said plant an agronomically valuable trait, wherein said second expression cassette is not localized between said sequences which allow for specific deletion of said first expression cassette, and
ii) providing at least one first compound X, which is phytotoxic against plant cells not functionally expressing said D-amino acid oxidase, wherein said compound X can be metabolized by said D-amino acid oxidase into one or more compound(s) Y which are non-phytotoxic or less phytotoxic than compound X, and
iii) treating said transformed plant cells of step i) with said first compound X in a phytotoxic concentration and selecting plant cells comprising in their genome said first DNA construct, conferring resistance to said transformed plant cells against said compound X by expression of said D-amino acid oxidase, and
iv) providing at least one second compound M, which is non-phytotoxic or moderately phytotoxic against plant cells not functionally expressing said D-amino acid oxidase, wherein said compound M can be metabolized by said D-amino acid oxidase into one or more compound(s) N which are phytotoxic or more phytotoxic than compound M, and
v) inducing deletion of said first expression cassette from the genome of said transformed plant cells and treating said plant cells with said second compound M in a concentration toxic to plant cells still comprising said first expression cassette, thereby selecting plant cells comprising said second expression cassette but lacking said first expression cassette.

5. The method of any of Claim 1 to 4 further comprising the step of regeneration of a fertile plant.

6. The method of any of Claim 1 to 5, wherein said first compound X when applied via spraying, is D-alanine applied in concentrations of about 5 to about 100 mM, or when applied via spraying D-serine is applied in concentrations of about 5 to about 80 mM,

7. The method of any of Claim 1 to 6, wherein deletion of said first expression cassette for the D-amino acid oxidase is realized by a method selected from:
a) recombination induced by a sequence specific recombinase, wherein said first expression cassette is flanked by corresponding recombination sites in a way that recombination between said flanking recombination sites results in deletion of the sequences in-between from the genome, or
b) homologous recombination between homology sequences A and A' flanking said first expression cassette, preferably induced by a sequence-specific double-strand break caused by a sequence specific endonuclease, wherein said homology sequences A and A' have sufficient length and homology in order to ensure homologous recombination between A and A', and having an orientation which - upon recombination between A and A' - will lead to excision of said first expression cassette from the genome of said plant.

8. The method of Claim 7, wherein the recombinase or sequence-specific endonuclease, respectively, is expressed or combined with its corresponding recombination or recognition site, respectively, by a method selected from the group consisting of:
a) incorporation of a second expression cassette for expression of the recombinase or sequence-specific endonuclease operably linked to a plant promoter into said DNA construct, preferably together with said first expression cassette flanked by said sequences which allow for specific deletion, or
b) incorporation of a second expression cassette for expression of the recombinase or sequence-specific endonuclease operably linked to a plant promoter into the plant cells or plants used as target material for the transformation thereby generating master cell lines or cells, or
c) incorporation of a second expression cassette for expression of the recombinase or sequence-specific endonuclease operably linked to a plant promoter into a separate DNA construct, which is transformed by way of co-transformation with said first DNA construct into said plant cells, or
d) incorporation of a second expression cassette for expression of the recombinase or sequence-specific endonuclease operably linked to a plant promoter into the plant cells or plants which are subsequently crossed with plants comprising the DNA construct of the invention.

9. The method of Claim 7 or 8, wherein deletion of said first expression cassette for the D-amino acid oxidase is induced or activated by inducing expression and/or activity of said sequence-specific recombinase or endonuclease by a method selected from the group consisting of
a) inducible expression by operably linking the sequence encoding said recombinase or endonuclease to an inducible promoter, and
b) inducible activation, by employing a modified recombinase or endonuclease comprising a ligand-binding-domain, wherein activity of said modified recombinase or endonuclease can by modified by treatment of a compound having binding activity to said ligand-binding-domain.

## Patentansprüche

1. Verfahren zur Herstellung einer transgenen Pflanze, das Folgendes umfasst:
i) Transformieren einer Pflanzenzelle mit einer ersten Expressionskassette, umfassend eine Nukleinsäuresequenz, die für eine D-Aminosäureoxidase codiert, in operativer Verknüpfung mit einem Promoter, der die Expression in Pflanzenzellen oder Pflanzen gestattet, in Kombination mit mindestens einer zweiten Expressionskassette, die sich dazu eignet, um dieser Pflanze ein landwirtschaftlich wertvolles Merkmal zu verleihen, und
ii) Bereitstellen von mindestens einer ersten Verbindung X, wobei es sich bei der Verbindung X entweder um D-Alanin oder D-Serin handelt, die für Pflanzenzellen, die diese D-Aminosäureoxidase nicht funktionell exprimieren, phytotoxisch ist, wobei die Verbindung X von der D-Aminosäureoxidase zu einer oder mehreren Vorbindung(en) Y metabolisiert werden kann, die nicht phytotoxisch oder weniger phytotoxisch als die Verbindung X ist/sind, und
iii) Behandeln von Pflanzen im Boden, die die transformierten Pflanzenzellen nach Schritt i) umfassen, mit der ersten Verbindung X in einer phytotoxischen Konzentration über drei aufeinanderfolgende Tage, wobei, wenn es sich bei der Verbindung X um D-Alanin handelt, die Konzentration 5 bis 100 mM beträgt, und wenn es sich bei der Verbindung X um D-Serin handelt, die Konzentration 5 bis 80 mM beträgt; oder Behandeln von Samen, die die transformierten Pflanzenzellen nach Schritt i) umfassen, mit der ersten Verbindung X in einer phytotoxischen Konzentration über fünf Tage während der Keimung auf Substraten, wobei, wenn es sich bei der Verbindung X um D-Alanin handelt, die Konzentration 0,1 mM bis 100 mM beträgt und, wenn es sich bei der Verbindung X um D-Serin handelt, die Konzentration 0,1 bis 10 mM beträgt, und Selektieren von Pflanzenzellen, die in ihrem Genom sowohl die erste als auch die zweite Expressionskassette umfassen, wobei die erste Expressionskassette durch Expression der D-Aminosäureoxidase den transformierten Pflanzenzellen Resistenz gegen die Verbindung X verleiht, und
iv) Bereitstellen von mindestens einer zweiten Verbindung M, die gegenüber Pflanzenzellen, die die D-Aminosäureoxidase nicht funktionell exprimieren, nicht phytotoxisch oder mäßig phytotoxisch ist, wobei die Verbindung M von der D-Aminosäureoxidase zu einer oder mehreren Verbindung(en) N metabolisiert werden kann, die phytotoxisch oder stärker phytotoxisch als die Verbindung M ist/sind,
und
v) Trennen der ersten Expressionskassette und der zweiten Expressionskassette und Behandeln der erhaltenen Pflanzenzellen mit der zweiten Verbindung M in einer Konzentration, die für Pflanzenzellen, die noch die erste Expressionskassette umfassen, toxisch ist, und Selektieren von Pflanzenzellen, die die zweite Expressionskassette umfassen, denen jedoch die erste Expressionskassette fehlt,
wobei M eine Struktur ausgewählt aus der Gruppe D-Valin, D-Leucin, D-Lysin und D-Prolin oder D-Isoleucin, appliziert über das Zellkulturmedium in Konzentrationen von ungefähr 10 mM bis ungefähr 30 mM, oder D-Asparagin oder D-Glutamin, appliziert über das Zellkulturmedium in Konzentrationen von ungefähr 3 mM bis ungefähr 20 mM umfasst.

2. Verfahren nach Anspruch 1, wobei während des Keimens der Samen nach Schritt iii) mindestens 1914 Arabidopsis-Samen pro Platte behandelt werden.

3. Verfahren nach Anspruch 1 oder 2, wobei die erste Expressionskassette für die D-Aminosäureoxidase und die zweite Expressionskassette für das landwirtschaftlich wertvolle Merkmal
a) beide in einem DNA-Konstrukt umfasst sind und die Kombination durch Deletieren oder Herausschneiden der ersten Expressionskassette für die D-Aminosäureoxidase aufgetrennt wird, oder
b) auf separaten DNA-Konstrukten umfasst sind, die in Kombination durch Kotransformation in die Pflanzenzellen transformiert werden, und die Kombination durch anschließende Trennung der zwei Expressionskassetten aufgetrennt wird.

4. Verfahren nach Anspruch 1 bis 3, wobei das Verfahren zur Herstellung einer transgenen Pflanze die folgenden Schritte umfasst:
i) Transformieren einer Pflanzenzelle mit einem ersten DNA-Konstrukt, das Folgendes umfasst:
a) eine erste Expressionskassette, umfassend eine Nukleinsäuresequenz, die für eine D-Aminosäureoxidase codiert, in operativer Verknüpfung mit einem Promoter, der die Expression in Pflanzenzellen oder Pflanzen gestattet, wobei die erste Expressionskassette von Sequenzen flankiert ist, die die spezifische Deletion der ersten Expressionskassette gestatten, und
b) mindestens eine zweite Expressionskassette, die sich dazu eignet, um der Pflanze ein landwirtschaftlich wertvolles Merkmal zu verleihen, wobei sich die zweite Expressionskassette nicht zwischen den Sequenzen, die die spezifische Deletion der ersten Expressionskassette gestatten, befindet, und
ii) Bereitstellen von mindestens einer ersten Verbindung X, die für Pflanzenzellen, die die D-Aminosäureoxidase nicht funktionell exprimieren, phytotoxisch ist, wobei die Verbindung X von der D-Aminosäureoxidase zu einer oder mehr Verbindung(en) Y metabolisiert werden kann, die nicht phytotoxisch oder weniger phytotoxisch als die Verbindung X ist/sind, und
iii) Behandeln der transformierten Pflanzenzellen nach Schritt i) mit der ersten Verbindung X in einer phytotoxischen Konzentration, und Selektieren von Pflanzenzellen, die in ihrem Genom das erste DNA-Konstrukt umfassen, das durch Expression der D-Aminosäureoxidase den transformierten Pflanzenzellen Resistenz gegen die Verbindung X verleiht, und
iv) Bereitstellen von mindestens einer zweiten Verbindung M, die gegenüber Pflanzenzellen, die die D-Aminosäureoxidase nicht funktionell exprimieren, nicht phytotoxisch oder mäßig phytotoxisch ist, wobei die Verbindung M von der D-Aminosäureoxidase zu einer oder mehreren Verbindung(en) N metabolisiert werden kann, die phytotoxisch oder stärker phytotoxisch als die Verbindung M ist/sind, und
v) Indizieren einer Deletion der ersten Expressionskassette aus dem Genom der transformierten Pflanzenzellen und Behandeln der Pflanzenzellen mit der zweiten Verbindung M in einer Konzentration, die für Pflanzenzellen, die noch die erste Expressionskassette umfassen, toxisch ist, wodurch Pflanzenzellen selektiert werden, die die zweite Expressionskassette umfassen, denen jedoch die erste Expressionskassette fehlt.

5. Verfahren nach einem der Ansprüche 1 bis 4, das weiterhin den Schritt des Regenerierens einer fruchtbaren Pflanze umfasst.

6. Verfahren nach einem der Ansprüche 1 bis 5, wobei es sich bei der ersten Verbindung X, wenn sie durch Sprühen appliziert wird, um D-Alanin, das in Konzentrationen von ungefähr 5 bis ungefähr 100 mM appliziert wird order um D-Serin, das in Konzentrationen von ungefähr 5 bis ungefähr 80 mM appliziert wird, handelt.

7. Verfahren nach einem der Ansprüche 1 bis 6, wobei die Deletion der ersten Expressionskassette für die D-Aminosäureoxidase durch ein aus der folgenden Reihe ausgewähltes Verfahren erfolgt:
a) Rekombination, die durch eine sequenzspezifische Rekombinase induziert wird, wobei die erste Expressionskassette so von entsprechenden Rekombinationsschnittstellen flankiert ist, dass eine Rekombination zwischen den flankierenden Rekombinationsschnittstellen zu der Deletion der dazwischenliegenden Sequenzen aus dem Genom führt, oder
b) homologe Rekombination zwischen den die erste Expressionskassette flankierenden Homologesequenzen A und A', die vorzugsweise durch einen durch eine sequenzspezifische Endonuklease verursachten sequenzspezifischen Doppelstrangbruch induziert wird, wobei die Homologiesequenzen A und A' ausreichend lang und homolog sind, um eine homologe Rekombination zwischen A und A' zu gewährleisten und wobei die Homologiesequenzen A und A' eine Orientierung aufweisen, die - bei Rekombination zwischen A und A' zum Herausschneiden der ersten Expressionskassette aus dem Genom der Pflanze führt.

8. Verfahren nach Anspruch 7, wobei die Rekombinase bzw. sequenzspezifische Endonuklease durch ein Verfahren, das aus der unten stehenden Gruppe ausgewählt ist, exprimiert oder mit ihrer entsprechenden Rekombinations- oder Erkennungsstelle kombiniert wird:
a) Einbauen einer zweiten Expressionskassette für die Expression der Rekombinase oder der sequenzspezifischen Endonuklease in operativer Verknüpfung mit einem pflanzlichen Promoter in das DNA-Konstrukt, vorzugsweise gemeinsam mit der ersten Expressionskassette, die von den Sequenzen, die die spezifische Deletion gestatten, flankiert wird, oder
b) Einbauen einer zweiten Expressionskassette für die Expression der Rekombinase oder der sequenzspezifischen Endonuklease in operativer Verknüpfung mit einem pflanzlichen Promoter in das die für die Transformation als Zielmaterial eingesetzten Pflanzenzellen oder Pflanzen, wodurch man zu Master-Zelllinien oder -Zellen gelangt, oder
c) Einbauen einer zweiten Expressionskassette für die Expression der Rekombinase oder der sequenzspezifischen Endonuklease in operativer Verknüpfung mit einem pflanzlichen Promoter in ein separates DNA-Konstrukt, das durch Kotransformation mit dem ersten DNA-Konstrukt in die Pflanzenzellen transformiert wird, oder
d) Einbauen einer zweiten Expressionskassette für die Expression der Rekombinase oder der sequenzspezifischen Endonuklease in operativer Verknüpfung mit einem pflanzlichen Promoter in Pflanzenzellen oder Pflanzen, die anschließend mit Pflanzen, die das erfindungsgemäße DNA-Konstrukt umfassen, gekreuzt werden.

9. Verfahren nach Anspruch 7 oder 8, wobei die Deletion der ersten Expressionskassette für die D-Aminosäureoxidase dadurch induziert oder aktiviert wird, dass man die Expression und/oder Aktivität der sequenzspezifischen Rekombinase oder Endonuklease durch ein Verfahren induziert, das aus der folgenden Gruppe ausgewählt ist:
a) induzierbare Expression durch operatives Verknüpfen der Sequenz, die für die Rekombinase oder Endonuklease codiert, mit einem induzierbaren Promoter, und
b) induzierbares Aktivieren durch Einsatz einer modifizierten Rekombinase oder Endonuklease umfassend eine Ligandenbindungsdomäne, wobei die Aktivität der modifizierten Rekombinase oder Endonuklease durch Behandeln einer Verbindung mit Bindungsaktivität für die Ligandenbindungsdomäne modifiziert werden kann.

## Revendications

1. Procédé de production d'une plante transgénique, comprenant :
i) la transformation d'une cellule végétale avec une première cassette d'expression comprenant une séquence d'acide nucléique codant pour une acide D-aminé oxydase, liée d'une manière opérationnelle à un promoteur permettant une expression dans des cellules végétales ou dans des plantes, en combinaison avec au moins une deuxième cassette d'expression apte à conférer à ladite plante une caractéristique intéressante d'un point de vue agronomique, et
ii) la mise à disposition d'au moins un premier composé X, le composé X étant la D-alanine ou la D-sérine, qui est phytotoxique pour des cellules végétales n'exprimant pas d'une manière fonctionnelle ladite acide D-aminé oxydase, ledit composé X pouvant être métabolisé par ladite acide D-aminé oxydase en un ou plusieurs composés Y qui ne sont pas phytotoxiques, ou qui sont moins phytotoxiques que le composé X, et
iii) le traitement de plantes dans un sol comprenant les cellules végétales transformées de l'étape i), avec le premier composé X à une concentration phytotoxique pendant trois jours consécutifs, où, si le composé X est la D-alanine, la concentration est de 5 à 100 mM, et, si le composé X est la D-sérine, la concentration est de 5 à 80 mM ; ou le traitement de semences comprenant les cellules végétales transformées de l'étape i) avec ledit premier composé X à une concentration phytotoxique pendant cinq jours pendant une germination sur un milieu, où, si le composé X est la D-alanine, la concentration est de 0,1 mM à 100 mM, et si le composé X est la D-sérine, la concentration est de 0,1 à 10 mM, et la sélection des cellules végétales comprenant dans leur génome tant ladite première que ladite deuxième cassettes d'expression, ladite première cassette d'expression conférant auxdites cellules végétales transformées une résistance audit composé X, par expression de ladite acide D-aminé oxydase, et
iv) la mise à disposition d'au moins un deuxième composé M, qui est non phytotoxique ou modérément phytotoxique pour les cellules végétales n'exprimant pas d'une manière fonctionnelle ladite acide D-aminé oxydase, ledit composé M pouvant être métabolisé par ladite acide D-aminé oxydase en un ou plusieurs composés N, qui sont phytotoxiques ou plus phytotoxiques que le composé M, et
v) la séparation de ladite première cassette d'expression et de ladite deuxième cassette d'expression, et le traitement desdites cellules végétales obtenues avec ledit deuxième composé M à une concentration qui est toxique pour les cellules végétales comprenant encore ladite première cassette d'expression, et la sélection des cellules végétales comprenant ladite deuxième cassette d'expression mais ne contenant pas ladite première cassette d'expression,
M comprenant une structure choisie dans le groupe consistant en la D-valine, la D-leucine, la D-lysine et la D-proline, ou la D-isoleucine, appliquée par l'intermédiaire du milieu de culture cellulaire à des concentrations d'environ 10 mM à environ 30 mM, ou la D-asparagine ou la D-glutamine appliquée par l'intermédiaire du milieu de culture cellulaire à des concentrations d'environ 3 mM à environ 20 mM.

2. Procédé de la revendication 1, dans lequel au moins 1 914 semences d'Arabidopsis par plaque sont traitées pendant la germination des semences de l'étape iii).

3. Procédé des revendications 1 ou 2, dans lequel ladite première cassette d'expression, pour ladite acide D-aminé oxydase, et ladite deuxième cassette d'expression pour ladite caractéristique intéressante d'un point de vue agronomique,
a) sont toutes les deux comprises dans une construction d'ADN, et la combinaison est rompue par délétion ou excision de ladite cassette d'expression pour ladite acide D-aminé oxydase, ou
b) sont comprises sur des constructions d'ADN distinctes, qui sont transformées en combinaison par co-transformation en lesdites cellules végétales, et la combinaison est rompue par une ségrégation ultérieure des deux cassettes d'expression.

4. Procédé des revendications 1 à 3, ledit procédé de production d'une plante transgénique comprenant les étapes de :
i) transformation d'une cellule végétale avec une première construction d'ADN comprenant
a) une première cassette d'expression comprenant une séquence d'acide nucléique codant pour une acide D-aminé oxydase liée d'une manière opérationnelle permettant une expression dans des cellules végétales ou des plantes, ladite première cassette d'expression étant flanquée de séquences qui permettent une délétion spécifique de ladite première cassette d'expression, et
b) au moins une deuxième cassette d'expression apte à conférer à ladite plante une caractéristique intéressante d'un point de vue agronomique, ladite deuxième cassette d'expression n'étant pas localisée entre lesdites séquences qui permettent une délétion spécifique de ladite première cassette d'expression, et
ii) la mise à disposition d'au moins un premier composé X, qui est phytotoxique pour les cellules végétales n'exprimant pas d'une manière fonctionnelle ladite acide D-aminé oxydase, ledit composé X pouvant être métabolisé par ladite acide D-aminé oxydase en un ou plusieurs composés Y qui sont non phytotoxiques ou moins phytotoxiques que le composé X, et
iii) le traitement desdites cellules végétales transformées de l'étape i) avec ledit premier composé X à une concentration phytotoxique, et la sélection des cellules végétales comprenant dans leur génome ladite première construction d'ADN, conférant auxdites cellules végétales transformées une résistance audit composé X par expression de ladite acide D-aminé oxydase, et
iv) la mise à disposition d'au moins un deuxième composé M, qui est non phytotoxique ou modérément phytotoxique pour les cellules végétales n'exprimant pas d'une manière fonctionnelle ladite acide D-aminé oxydase, ledit composé M pouvant être métabolisé par ladite acide D-aminé oxydase en un ou plusieurs composés N qui sont phytotoxiques ou plus phytotoxiques que le composé M, et
v) l'induction d'une délétion de ladite première cassette d'expression, à partir du génome desdites cellules végétales transformées, et le traitement desdites cellules végétales avec ledit deuxième composé M à une concentration qui est toxique pour les cellules végétales comprenant encore ladite première cassette d'expression, ce qui va sélectionner les cellules végétales comprenant ladite deuxième cassette d'expression mais ne contenant pas ladite première cassette d'expression.

5. Procédé de l'une quelconque des revendications 1 à 4, qui comprend en outre l'étape de régénération d'une plante fertile.

6. Procédé de l'une quelconque des revendications 1 à 5, dans lequel ledit premier composé X, quand il est appliqué par pulvérisation, est la D-alanine appliquée à des concentrations d'environ 5 à environ 100 mM, ou, quand il est appliqué par pulvérisation, on applique de la D-sérine à des concentrations d'environ 5 à environ 80 mM.

7. Procédé de l'une quelconque des revendications 1 à 6, dans lequel la délétion de ladite première cassette d'expression pour l'acide D-aminé oxydase est réalisée par un procédé choisi parmi :
a) une recombinaison induite par une recombinase spécifique de séquence, ladite première cassette d'expression étant flanquée de sites de recombinaison correspondants de telle sorte qu'une recombinaison entre lesdits sites de recombinaison flanquants conduise à une délétion des séquences intermédiaires provenant du génome, ou
b) une recombinaison homologue entre les séquences d'homologie A et A' flanquant ladite première cassette d'expression, de préférence induite par une rupture double brin spécifique de séquence provoquée par une endonucléase spécifique de séquence, lesdites séquences d'homologie A et A' ayant une longueur et une homologie suffisantes pour assurer une recombinaison homologue entre A et A', et ayant une orientation qui, après recombinaison entre A et A', va conduire à l'excision de ladite première cassette d'expression à partir du génome de ladite plante.

8. Procédé de la revendication 7, dans lequel respectivement la recombinase ou l'endonucléase spécifique de séquence est exprimée, respectivement avec son site de recombinaison correspondant et à son site de reconnaissance correspondant, par un procédé choisi dans le groupe consistant en :
a) l'incorporation d'une deuxième cassette d'expression pour expression de la recombinase ou de l'endonucléase spécifique de séquence liée d'une manière opérationnelle à un promoteur de plante dans ladite construction d'ADN, de préférence en même temps que ladite première cassette d'expression flanquée desdites séquences qui permettent une délétion spécifique, ou
b) l'incorporation d'une deuxième cassette d'expression pour expression de la recombinase ou de l'endonucléase spécifique de séquence, liée d'une manière opérationnelle à un promoteur de plante dans des cellules végétales ou dans des plantes utilisées en tant que matériau cible pour la transformation, ce qui va générer des lignées de cellules maîtresses ou des cellules maîtresses, ou
c) l'incorporation d'une deuxième cassette d'expression pour expression de la recombinase ou de l'endonucléase spécifique de séquence, liée d'une manière opérationnelle à un promoteur de plante, dans une construction d'ADN distincte, qui est transformée par co-transformation avec ladite première construction d'ADN en lesdites cellules végétales, ou
d) l'incorporation d'une deuxième cassette d'expression pour expression de la recombinase ou de l'endonucléase spécifique de séquence liée d'une manière opérationnelle à un promoteur de plante, dans des cellules végétales ou des plantes qui ultérieurement sont croisées avec des plantes comprenant la construction d'ADN de l'invention.

9. Procédé de la revendication 7 ou 8, dans lequel la délétion de ladite première d'expression pour l'acide D-aminé oxydase est induite ou activée par induction de l'expression et/ou de l'activité de ladite recombinase ou endonucléase spécifique de séquence par un procédé choisi dans le groupe consistant en :
a) l'induction inductible par liaison opérationnelle de la séquence codant pour ladite recombinase ou endonucléase à un promoteur inductible, et
b) l'activation inductible, par utilisation d'une recombinase ou d'une endonucléase modifiée comprenant un domaine de liaison à un ligand, l'activité de ladite recombinase ou endonucléase modifiée pouvant être modifiée par traitement d'un composé ayant une activité de liaison audit domaine de liaison à un ligand.


### SEQUENCE LISTING

<110> BASF Plant Science GmbH
   SweTree Technologies AB
<120> IMPROVED CONSTRUCTS FOR MARKER EXCISION BASED ON DUAL-FUNCTION SELECTION MARKER
<130> PF 55443 EP
<160> 16
<170> PatentIn version 3.1
<210> 1
   <211> 1160
   <212> DNA
   <213> Rhodosporidium toruloides
<220>
   <221> CDS
   <222> (1)..(1104)
   <223> coding for DAAO
<400> 1
   atg cac tcg cag aag cgc gtc gtt gtc ctc gga tca ggc gtt atc ggt      48
   Met His Ser Gln Lys Arg Val Val Val Leu Gly Ser Gly Val Ile Gly
   1               5                   10                  15
   ctg agc agc gcc ctc atc ctc gct cgg aag ggc tac agc gtg cat att      96
   Leu Ser Ser Ala Leu Ile Leu Ala Arg Lys Gly Tyr Ser Val His Ile
               20                  25                  30
   ctc gcg cgc gac ttg ccg gag gac gtc tcg agc cag act ttc gct tca     144
   Leu Ala Arg Asp Leu Pro Glu Asp Val Ser Ser Gln Thr Phe Ala Ser
           35                  40                  45
   cca tgg gct ggc gcg aat tgg acg cct ttc atg acg ctt aca gac ggt     192
   Pro Trp Ala Gly Ala Asn Trp Thr Pro Phe Met Thr Leu Thr Asp Gly
       50                  55                  60
   cct cga caa gca aaa tgg gaa gaa tcg act ttc aag aag tgg gtc gag     240
   Pro Arg Gln Ala Lys Trp Glu Glu Ser Thr Phe Lys Lys Trp Val Glu
   65                  70                  75                  80
   ttg gtc ccg acg ggc cat gcc atg tgg ctc aag ggg acg agg cgg ttc        288
   Leu Val Pro Thr Gly His Ala Met Trp Leu Lys Gly Thr Arg Arg Phe
                    85                  90                  95
   gcg cag aac gaa gac ggc ttg ctc ggg cac tgg tac aag gac atc acg        336
   Ala Gln Asn Glu Asp Gly Leu Leu Gly His Trp Tyr Lys Asp Ile Thr
               100                 105                 110
   cca aat tac cgc ccc ctc cca tct tcc gaa tgt cca cct ggc gct atc        384
   Pro Asn Tyr Arg Pro Leu Pro Ser Ser Glu Cys Pro Pro Gly Ala Ile
           115                 120                 125
   ggc gta acc tac gac acc ctc tcc gtc cac gca cca aag tac tgc cag        432
   Gly Val Thr Tyr Asp Thr Leu Ser Val His Ala Pro Lys Tyr Cys Gln
       130                 135                 140
   tac ctt gca aga gag ctg cag aag ctc ggc gcg acg ttt gag aga cgg        480
   Tyr Leu Ala Arg Glu Leu Gln Lys Leu Gly Ala Thr Phe Glu Arg Arg
   145                 150                 155                 160
   acc gtt acg tcg ctt gag cag gcg ttc gac ggt gcg gat ttg gtg gtc        528
   Thr Val Thr Ser Leu Glu Gln Ala Phe Asp Gly Ala Asp Leu Val Val
                   165                 170                 175
   aac gct acg gga ctt ggc gcc aag tcg att gcg ggc atc gac gac caa        576
   Asn Ala Thr Gly Leu Gly Ala Lys Ser Ile Ala Gly Ile Asp Asp Gln
               180                 185                 190
   gcc gcc gag cca atc cgc ggg caa acc gtc ctc gtc aag tcc cca tgc        624
   Ala Ala Glu Pro Ile Arg Gly Gln Thr Val Leu Val Lys Ser Pro Cys
           195                 200                 205
   aag cga tgc acg atg gac tcg tcc gac ccc gct tct ccc gcc tac atc        672
   Lys Arg Cys Thr Met Asp Ser Ser Asp Pro Ala Ser Pro Ala Tyr Ile
       210                 215                 220
   att ccc cga cca ggt ggc gaa gtc atc tgc ggc ggg acg tac ggc gtg        720
   Ile Pro Arg Pro Gly Gly Glu Val Ile Cys Gly Gly Thr Tyr Gly Val
   225                 230                 235                 240
   gga gac tgg gac ttg tct gtc aac cca gag acg gtc cag cgg atc ctc        768
   Gly Asp Trp Asp Leu Ser Val Asn Pro Glu Thr Val Gln Arg Ile Leu
                   245                 250                 255
   aag cac tgc ttg cgc ctc gac ccg acc atc tcg agc gac gga acg atc      816
   Lys His Cys Leu Arg Leu Asp Pro Thr Ile Ser Ser Asp Gly Thr Ile
               260                 265                 270
   gaa ggc atc gag gtc ctc cgc cac aac gtc ggc ttg cga cct gca cga      864
   Glu Gly Ile Glu Val Leu Arg His Asn Val Gly Leu Arg Pro Ala Arg
           275                 280                 285
   cga ggc gga ccc cgc gtt gag gca gaa cgg atc gtc ctg cct ctc gac      912
   Arg Gly Gly Pro Arg Val Glu Ala Glu Arg Ile Val Leu Pro Leu Asp
       290                 295                 300
   cgg aca aag tcg ccc ctc tcg ctc ggc agg ggc agc gca cga gcg gcg      960
   Arg Thr Lys Ser Pro Leu Ser Leu Gly Arg Gly Ser Ala Arg Ala Ala
   305                 310                 315                 320
   aag gag aag gag gtc acg ctt gtg cat gcg tat ggc ttc tcg agt gcg     1008
   Lys Glu Lys Glu Val Thr Leu Val His Ala Tyr Gly Phe Ser Ser Ala
                   325                 330                 335
   gga tac cag cag agt tgg ggc gcg gcg gag gat gtc gcg cag ctc gtc     1056
   Gly Tyr Gln Gln Ser Trp Gly Ala Ala Glu Asp Val Ala Gln Leu Val
               340                 345                 350
   gac gag gcg ttc cag cgg tac cac ggc gcg gcg cgg gag tcg aag ttg     1104
   Asp Glu Ala Phe Gln Arg Tyr His Gly Ala Ala Arg Glu Ser Lys Leu
           355                 360                 365
   tagggcggga tttgtggctg tattgcgggc atctacaaga aaaaaaaaaa aaaaaa       1160
<210> 2
   <211> 368
   <212> PRT
   <213> Rhodosporidium toruloides
<400> 2
   Met His Ser Gln Lys Arg Val Val Val Leu Gly Ser Gly Val Ile Gly
   1               5                   10                  15
   Leu Ser Ser Ala Leu Ile Leu Ala Arg Lys Gly Tyr Ser Val His Ile
               20                  25                  30
   Leu Ala Arg Asp Leu Pro Glu Asp Val Ser Ser Gln Thr Phe Ala Ser
           35                  40                  45
   Pro Trp Ala Gly Ala Asn Trp Thr Pro Phe Met Thr Leu Thr Asp Gly
   50 55 60
   Pro Arg Gln Ala Lys Trp Glu Glu Ser Thr Phe Lys Lys Trp Val Glu
   65 70 75 80
   Leu Val Pro Thr Gly His Ala Met Trp Leu Lys Gly Thr Arg Arg Phe
   85 90 95
   Ala Gln Asn Glu Asp Gly Leu Leu Gly His Trp Tyr Lys Asp Ile Thr
   100 105 110
   Pro Asn Tyr Arg Pro Leu Pro Ser Ser Glu Cys Pro Pro Gly Ala Ile
   115 120 125
   Gly Val Thr Tyr Asp Thr Leu Ser Val His Ala Pro Lys Tyr Cys Gln
   130 135 140
   Tyr Leu Ala Arg Glu Leu Gln Lys Leu Gly Ala Thr Phe Glu Arg Arg
   145 150 155 160
   Thr Val Thr Ser Leu Glu Gln Ala Phe Asp Gly Ala Asp Leu Val Val
   165 170 175
   Asn Ala Thr Gly Leu Gly Ala Lys Ser Ile Ala Gly Ile Asp Asp Gln
   180 185 190
   Ala Ala Glu Pro Ile Arg Gly Gln Thr Val Leu Val Lys Ser Pro Cys
   195 200 205
   Lys Arg Cys Thr Met Asp Ser Ser Asp Pro Ala Ser Pro Ala Tyr Ile
   210 215 220
   Ile Pro Arg Pro Gly Gly Glu Val Ile Cys Gly Gly Thr Tyr Gly Val
   225 230 235 240
   Gly Asp Trp Asp Leu Ser Val Asn Pro Glu Thr Val Gln Arg Ile Leu
   245 250 255
   Lys His Cys Leu Arg Leu Asp Pro Thr Ile Ser Ser Asp Gly Thr Ile
   260 265 270
   Glu Gly Ile Glu Val Leu Arg His Asn Val Gly Leu Arg Pro Ala Arg
   275 280 285
   Arg Gly Gly Pro Arg Val Glu Ala Glu Arg Ile Val Leu Pro Leu Asp
       290                 295                 300
   Arg Thr Lys Ser Pro Leu Ser Leu Gly Arg Gly Ser Ala Arg Ala Ala
   305                 310                 315                 320
   Lys Glu Lys Glu Val Thr Leu Val His Ala Tyr Gly Phe Ser Ser Ala
                   325                 330                 335
   Gly Tyr Gln Gln Ser Trp Gly Ala Ala Glu Asp Val Ala Gln Leu Val
               340                 345                 350
   Asp Glu Ala Phe Gln Arg Tyr His Gly Ala Ala Arg Glu Ser Lys Leu
           355                 360                 365
<210> 3
   <211> 1005
   <212> DNA
   <213> Caenorhabditis elegans
<220>
   <221> CDS
   <222> (1)..(1002)
   <223> coding for DAAO
<400> 3
   atg gca aac ata att ccg aag att gca att atc ggc gaa gga gtc att      48
   Met Ala Asn Ile Ile Pro Lys Ile Ala Ile Ile Gly Glu Gly Val Ile
   1               5                   10                  15
   gga tgt act tca gca ctt caa ata tca aaa gct ata cca aat gcg aaa      96
   Gly Cys Thr Ser Ala Leu Gln Ile Ser Lys Ala Ile Pro Asn Ala Lys
               20                  25                  30
   ata act gtg ctc cac gat aaa cca ttt aaa aaa tcg tgc agt gca gga     144
   Ile Thr Val Leu His Asp Lys Pro Phe Lys Lys Ser Cys Ser Ala Gly
           35                  40                  45
   cca gca gga tta ttt aga atc gat tat gag gag aat act gaa tac gga     192
   Pro Ala Gly Leu Phe Arg Ile Asp Tyr Glu Glu Asn Thr Glu Tyr Gly
       50                  55                  60
   cgt gct tct ttc gcc tgg ttc tca cat ctc tat cgc act aca aaa gga     240
   Arg Ala Ser Phe Ala Trp Phe Ser His Leu Tyr Arg Thr Thr Lys Gly
   65                  70                  75                  80
   tcc gaa acc ggc gtg aaa tta gtt tct gga cat att caa tcc gac aac         288
   Ser Glu Thr Gly Val Lys Leu Val Ser Gly His Ile Gln Ser Asp Asn
                   85                  90                  95
   ttg gag tca ttg aag caa caa caa aga gcc tat ggc gat att gtg tac         336
   Leu Glu Ser Leu Lys Gln Gln Gln Arg Ala Tyr Gly Asp Ile Val Tyr
               100                 105                 110
   aac ttt aga ttc ttg gat gat aga gaa cgg ctg gac att ttt ccc gaa         384
   Asn Phe Arg Phe Leu Asp Asp Arg Glu Arg Leu Asp Ile Phe Pro Glu
           115                 120                 125
   cca tca aag cac tgc att cac tac acc gcc tac gca tca gaa ggt aac         432
   Pro Ser Lys His Cys Ile His Tyr Thr Ala Tyr Ala Ser Glu Gly Asn
       130                 135                 140
   aag tac gtg cct tat ttg aag aat ttg ctg ctt gag caa aaa atc gag         480
   Lys Tyr Val Pro Tyr Leu Lys Asn Leu Leu Leu Glu Gln Lys Ile Glu
   145                 150                 155                 160
   ttc aag caa caa gaa gtg acg agt ttg gac gca gtc gcc gac gct ggt         528
   Phe Lys Gln Gln Glu Val Thr Ser Leu Asp Ala Val Ala Asp Ala Gly
                   165                 170                 175
   tac gat gtt att gta aac tgc gca ggc ttg tac ggt gga aag ttg gct         576
   Tyr Asp Val Ile Val Asn Cys Ala Gly Leu Tyr Gly Gly Lys Leu Ala
               180                 185                 190
   ggt gat gac gat act tgc tac ccc att aga gga gtc att ttg gaa gtt         624
   Gly Asp Asp Asp Thr Cys Tyr Pro Ile Arg Gly Val Ile Leu Glu Val
           195                 200                 205
   gat gca cca tgg cac aag cac ttc aat tat cga gac ttt act act ttc         672
   Asp Ala Pro Trp His Lys His Phe Asn Tyr Arg Asp Phe Thr Thr Phe
       210                 215                 220
   aca att cca aaa gag cac agc gtg gtg gtt ggg tcc acc aag cag gac         720
   Thr Ile Pro Lys Glu His Ser Val Val Val Gly Ser Thr Lys Gln Asp
   225                 230                 235                 240
   aat cga tgg gat ttg gag atc acc gac gag gat aga aat gat att ttg         768
   Asn Arg Trp Asp Leu Glu Ile Thr Asp Glu Asp Arg Asn Asp Ile Leu
                   245                 250                 255
   aaa cga tac att gct tta cat cct gga atg aga gag cca aag att atc      816
   Lys Arg Tyr Ile Ala Leu His Pro Gly Met Arg Glu Pro Lys Ile Ile
                   260                 265                 270
   aaa gaa tgg tca gca ctt cgc ccg gga cgt aag cat gtc aga att gaa      864
   Lys Glu Trp Ser Ala Leu Arg Pro Gly Arg Lys His Val Arg Ile Glu
               275                 280                 285
   gcg cag aag agg aca tct gtt gga aac tca aaa gat tat atg gtt gtg      912
   Ala Gln Lys Arg Thr Ser Val Gly Asn Ser Lys Asp Tyr Met Val Val
           290                 295                 300
   cat cac tat ggt cac ggg agc aac gga ttc acg ttg ggt tgg gga aca      960
   His His Tyr Gly His Gly Ser Asn Gly Phe Thr Leu Gly Trp Gly Thr
   305                 310                 315                 320
   gca att gaa gca act aaa ctt gtt aag act gca cta gga tta taa         1005
   Ala Ile Glu Ala Thr Lys Leu Val Lys Thr Ala Leu Gly Leu
                   325                 330
<210> 4
   <211> 334
   <212> PRT
   <213> Caenorhabditis elegans
<400> 4
   Met Ala Asn Ile Ile Pro Lys Ile Ala Ile Ile Gly Glu Gly Val Ile
   1               5                   10                  15
   Gly Cys Thr Ser Ala Leu Gln Ile Ser Lys Ala Ile Pro Asn Ala Lys
               20                  25                  30
   Ile Thr Val Leu His Asp Lys Pro Phe Lys Lys Ser Cys Ser Ala Gly
           35                  40                  45
   Pro Ala Gly Leu Phe Arg Ile Asp Tyr Glu Glu Asn Thr Glu Tyr Gly
       50                  55                  60
   Arg Ala Ser Phe Ala Trp Phe Ser His Leu Tyr Arg Thr Thr Lys Gly
   65                  70                  75                  80
   Ser Glu Thr Gly Val Lys Leu Val Ser Gly His Ile Gln Ser Asp Asn
                   85                  90                  95
   Leu Glu Ser Leu Lys Gln Gln Gln Arg Ala Tyr Gly Asp Ile Val Tyr
               100                 105                 110
   Asn Phe Arg Phe Leu Asp Asp Arg Glu Arg Leu Asp Ile Phe Pro Glu
           115                 120                 125
   Pro Ser Lys His Cys Ile His Tyr Thr Ala Tyr Ala Ser Glu Gly Asn
       130                 135                 140
   Lys Tyr Val Pro Tyr Leu Lys Asn Leu Leu Leu Glu Gln Lys Ile Glu
   145                 150                 155                 160
   Phe Lys Gln Gln Glu Val Thr Ser Leu Asp Ala Val Ala Asp Ala Gly
                   165                 170                 175
   Tyr Asp Val Ile Val Asn Cys Ala Gly Leu Tyr Gly Gly Lys Leu Ala
               180                 185                 190
   Gly Asp Asp Asp Thr Cys Tyr Pro Ile Arg Gly Val Ile Leu Glu Val
           195                 200                 205
   Asp Ala Pro Trp His Lys His Phe Asn Tyr Arg Asp Phe Thr Thr Phe
       210                 215                 220
   Thr Ile Pro Lys Glu His Ser Val Val Val Gly Ser Thr Lys Gln Asp
   225                 230                 235                 240
   Asn Arg Trp Asp Leu Glu Ile Thr Asp Glu Asp Arg Asn Asp Ile Leu
                   245                 250                 255
   Lys Arg Tyr Ile Ala Leu His Pro Gly Met Arg Glu Pro Lys Ile Ile
               260                 265                 270
   Lys Glu Trp Ser Ala Leu Arg Pro Gly Arg Lys His Val Arg Ile Glu
           275                 280                 285
   Ala Gln Lys Arg Thr Ser Val Gly Asn Ser Lys Asp Tyr Met Val Val
       290                 295                 300
   His His Tyr Gly His Gly Ser Asn Gly Phe Thr Leu Gly Trp Gly Thr
   305                 310                 315                 320
   Ala Ile Glu Ala Thr Lys Leu Val Lys Thr Ala Leu Gly Leu
                   325                 330
<210> 5
   <211> 1186
   <212> DNA
   <213> Nectria haematococca
<220>
   <221> CDS
   <222> (42)..(1124)
   <223> coding for DAAO
<400> 5
   agcgacttga atttagcgaa aagaacttgt caaccacaat c atg tcc aac aca atc      56
                                                 Met Ser Asn Thr Ile
                                                 1               5
   gtc gtc gtt ggt gcc ggt gtc att ggc ttg acg tcg gcc ttg ttg ctc       104
   Val Val Val Gly Ala Gly Val Ile Gly Leu Thr Ser Ala Leu Leu Leu
                   10                  15                  20
   tcc aag aac aag ggc aac aag atc acc gtc gtg gcc aag cac atg ccc       152
   Ser Lys Asn Lys Gly Asn Lys Ile Thr Val Val Ala Lys His Met Pro
               25                  30                  35
   ggc gac tat gac gtt gaa tac gcc tcg cct ttt gct ggt gcc aac cac       200
   Gly Asp Tyr Asp Val Glu Tyr Ala Ser Pro Phe Ala Gly Ala Asn His
           40                  45                  50
   tcc ccc atg gcg acg gaa gag agc agc gaa tgg gaa cgt cgc act tgg       248
   Ser Pro Met Ala Thr Glu Glu Ser Ser Glu Trp Glu Arg Arg Thr Trp
       55                  60                  65
   tac gag ttt aag aga ctg gtc gag gag gtc cct gag gcc ggt gtt cat       296
   Tyr Glu Phe Lys Arg Leu Val Glu Glu Val Pro Glu Ala Gly Val His
   70                  75                  80                  85
   ttc cag aag tct cgc atc cag agg cgc aat gtg gac act gaa aag gcg       344
   Phe Gln Lys Ser Arg Ile Gln Arg Arg Asn Val Asp Thr Glu Lys Ala
                   90                  95                  100
   cag agg tct ggt ttc cca gac gcc ctc ttc tcg aaa gaa ccc tgg ttc       392
   Gln Arg Ser Gly Phe Pro Asp Ala Leu Phe Ser Lys Glu Pro Trp Phe
               105                 110                 115
   aag aac atg ttt gag gac ttc cgt gag cag cac cct agc gag gtc atc       440
   Lys Asn Met Phe Glu Asp Phe Arg Glu Gln His Pro Ser Glu Val Ile
   120 125 130
   ccc ggt tac gac tct ggc tgc gag ttc aca tcg gtg tgc atc aac acg 488
   Pro Gly Tyr Asp Ser Gly Cys Glu Phe Thr Ser Val Cys Ile Asn Thr
   135 140 145
   gcc atc tac ctc ccc tgg ctc ctc ggc cag tgc atc aag aat ggc gtc 536
   Ala Ile Tyr Leu Pro Trp Leu Leu Gly Gln Cys Ile Lys Asn Gly Val
   150 155 160 165
   atc gtc aag cgc gcc atc ctc aac gac att agc gag gcc aag aag ctg 584
   Ile Val Lys Arg Ala Ile Leu Asn Asp Ile Ser Glu Ala Lys Lys Leu
   170 175 180
   agc cac gcg ggc aag acg ccc aat atc atc gtc aac gcc acg ggt ctc 632
   Ser His Ala Gly Lys Thr Pro Asn Ile Ile Val Asn Ala Thr Gly Leu
   185 190 195
   ggc tcc tac aag ctg ggc ggt gtc gag gac aag acc atg gcg cct gcg 680
   Gly Ser Tyr Lys Leu Gly Gly Val Glu Asp Lys Thr Met Ala Pro Ala
   200 205 210
   cgg gga cag att gtg gtt gtg cgc aac gag agc agc ccc atg ctc ctc 728
   Arg Gly Gln Ile Val Val Val Arg Asn Glu Ser Ser Pro Met Leu Leu
   215 220 225
   act tca ggt gtc gag gac ggc ggt gct gat gtc atg tac ttg atg cag 776
   Thr Ser Gly Val Glu Asp Gly Gly Ala Asp Val Met Tyr Leu Met Gln
   230 235 240 245
   cga gca gct ggc ggt ggc acc atc ctg ggc ggt acc tac gac gtt ggc 824
   Arg Ala Ala Gly Gly Gly Thr Ile Leu Gly Gly Thr Tyr Asp Val Gly
   250 255 260
   aac tgg gag tct cag cca gac ccc aac atc gcg aat cgc atc atg cag 872
   Asn Trp Glu Ser Gln Pro Asp Pro Asn Ile Ala Asn Arg Ile Met Gln
   265 270 275
   cgc atc gtc gag gtg cgg ccc gag att gcc aac ggc aag ggc gtc aag 920
   Arg Ile Val Glu Val Arg Pro Glu Ile Ala Asn Gly Lys Gly Val Lys
   280 285 290
   ggg ctg agc gtg atc cga cac gcc gtc ggc atg cgg ccg tgg cga aag 968
   Gly Leu Ser Val Ile Arg His Ala Val Gly Met Arg Pro Trp Arg Lys
   295 300 305
   gac gga gtc agg atc gag gag gag aag ctg gat gat gag act tgg atc      1016
   Asp Gly Val Arg Ile Glu Glu Glu Lys Leu Asp Asp Glu Thr Trp Ile
   310                 315                 320                 325
   gtg cac aac tac gga cac tct gga tgg ggt tac cag ggt tcg tat ggt      1064
   Val His Asn Tyr Gly His Ser Gly Trp Gly Tyr Gln Gly Ser Tyr Gly
                   330                 335                 340
   tgt gct gag aat gta gtc cag ttg gtt gac aag gtc ggc aag gcg gcc      1112
   Cys Ala Glu Asn Val Val Gln Leu Val Asp Lys Val Gly Lys Ala Ala
               345                 350                 355
   aag tct aag ctg tagttgaaaa ggcctgaatg agtaatagta attggatatt          1164
   Lys Ser Lys Leu
           360
   ggaaataccg tatttgccct cg                                             1186
<210> 6
   <211> 361
   <212> PRT
   <213> Nectria haematococca
<400> 6
   Met Ser Asn Thr Ile Val Val Val Gly Ala Gly Val Ile Gly Leu Thr
   1               5                   10                  15
   Ser Ala Leu Leu Leu Ser Lys Asn Lys Gly Asn Lys Ile Thr Val Val
               20                  25                  30
   Ala Lys His Met Pro Gly Asp Tyr Asp Val Glu Tyr Ala Ser Pro Phe
           35                  40                  45
   Ala Gly Ala Asn His Ser Pro Met Ala Thr Glu Glu Ser Ser Glu Trp
       50                  55                  60
   Glu Arg Arg Thr Trp Tyr Glu Phe Lys Arg Leu Val Glu Glu Val Pro
   65                  70                  75                  80
   Glu Ala Gly Val His Phe Gln Lys Ser Arg Ile Gln Arg Arg Asn Val
                   85                  90                  95
   Asp Thr Glu Lys Ala Gln Arg Ser Gly Phe Pro Asp Ala Leu Phe Ser
               100                 105                 110
   Lys Glu Pro Trp Phe Lys Asn Met Phe Glu Asp Phe Arg Glu Gln His
   115 120 125
   Pro Ser Glu Val Ile Pro Gly Tyr Asp Ser Gly Cys Glu Phe Thr Ser
   130 135 140
   Val Cys Ile Asn Thr Ala Ile Tyr Leu Pro Trp Leu Leu Gly Gln Cys
   145 150 155 160
   Ile Lys Asn Gly Val Ile Val Lys Arg Ala Ile Leu Asn Asp Ile Ser
   165 170 175
   Glu Ala Lys Lys Leu Ser His Ala Gly Lys Thr Pro Asn Ile Ile Val
   180 185 190
   Asn Ala Thr Gly Leu Gly Ser Tyr Lys Leu Gly Gly Val Glu Asp Lys
   195 200 205
   Thr Met Ala Pro Ala Arg Gly Gln Ile Val Val Val Arg Asn Glu Ser
   210 215 220
   Ser Pro Met Leu Leu Thr Ser Gly Val Glu Asp Gly Gly Ala Asp Val
   225 230 235 240
   Met Tyr Leu Met Gln Arg Ala Ala Gly Gly Gly Thr Ile Leu Gly Gly
   245 250 255
   Thr Tyr Asp Val Gly Asn Trp Glu Ser Gln Pro Asp Pro Asn Ile Ala
   260 265 270
   Asn Arg Ile Met Gln Arg Ile Val Glu Val Arg Pro Glu Ile Ala Asn
   275 280 285
   Gly Lys Gly Val Lys Gly Leu Ser Val Ile Arg His Ala Val Gly Met
   290 295 300
   Arg Pro Trp Arg Lys Asp Gly Val Arg Ile Glu Glu Glu Lys Leu Asp
   305 310 315 320
   Asp Glu Thr Trp Ile Val His Asn Tyr Gly His Ser Gly Trp Gly Tyr
   325 330 335
   Gln Gly Ser Tyr Gly Cys Ala Glu Asn Val Val Gln Leu Val Asp Lys
   340 345 350
   Val Gly Lys Ala Ala Lys Ser Lys Leu
   355 360
<210> 7
   <211> 1071
   <212> DNA
   <213> Trigonopsis variabilis
<220>
   <221> CDS
   <222> (1)..(1068)
   <223>
<400> 7
   atg gct aaa atc gtt gtt att ggt gcc ggt gtt gcc ggt tta act aca 48
   Met Ala Lys Ile Val Val Ile Gly Ala Gly Val Ala Gly Leu Thr Thr
   1 5 10 15
   gct ctt caa ctt ctt cgt aaa gga cat gag gtt aca att gtg tcc gag 96
   Ala Leu Gln Leu Leu Arg Lys Gly His Glu Val Thr Ile Val Ser Glu
   20 25 30
   ttt acg ccc ggt gat ctt agt atc gga tat acc tcg cct tgg gca ggt 144
   Phe Thr Pro Gly Asp Leu Ser Ile Gly Tyr Thr Ser Pro Trp Ala Gly
   35 40 45
   gcc aac tgg ctc aca ttt tac gat gga ggc aag tta gcc gac tac gat 192
   Ala Asn Trp Leu Thr Phe Tyr Asp Gly Gly Lys Leu Ala Asp Tyr Asp
   50 55 60
   gcc gtc tct tat cct atc ttg cga gag ctg gct cga agc agc ccc gag 240
   Ala Val Ser Tyr Pro Ile Leu Arg Glu Leu Ala Arg Ser Ser Pro Glu
   65 70 75 80
   gct gga att cga ctc atc agc caa cgc tcc cat gtt ctc aag cgt gat 288
   Ala Gly Ile Arg Leu Ile Ser Gln Arg Ser His Val Leu Lys Arg Asp
   85 90 95
   ctt cct aaa ctg gaa gtt gcc atg tcg gcc atc tgt caa cgc aat ccc 336
   Leu Pro Lys Leu Glu Val Ala Met Ser Ala Ile Cys Gln Arg Asn Pro
   100 105 110
   tgg ttc aaa aac aca gtc gat tct ttc gag att atc gag gac agg tcc      384
   Trp Phe Lys Asn Thr Val Asp Ser Phe Glu Ile Ile Glu Asp Arg Ser
           115                 120                 125
   agg att gtc cac gat gat gtg gct tat cta gtc gaa ttt cgt tcc gtt      432
   Arg Ile Val His Asp Asp Val Ala Tyr Leu Val Glu Phe Arg Ser Val
       130                 135                 140
   tgt atc cac acc gga gtc tac ttg aac tgg ctg atg tcc caa tgc tta      480
   Cys Ile His Thr Gly Val Tyr Leu Asn Trp Leu Met Ser Gln Cys Leu
   145                 150                 155                 160
   tcg ctc ggc gcc acg gtg gtt aaa cgt cga gtg aac cat atc aag gat      528
   Ser Leu Gly Ala Thr Val Val Lys Arg Arg Val Asn His Ile Lys Asp
                   165                 170                 175
   gcc aat tta cta cac tcc tca gga tca cgc ccc gac gtg att gtc aac      576
   Ala Asn Leu Leu His Ser Ser Gly Ser Arg Pro Asp Val Ile Val Asn
               180                 185                 190
   tgt agt ggt ctc ttt gcc cgg ttc ttg gga ggc gtc gag gac aag aag      624
   Cys Ser Gly Leu Phe Ala Arg Phe Leu Gly Gly Val Glu Asp Lys Lys
           195                 200                 205
   atg tac cct att cga gga caa gtc gtc ctt gtt cga aac tct ctt cct      672
   Met Tyr Pro Ile Arg Gly Gln Val Val Leu Val Arg Asn Ser Leu Pro
       210                 215                 220
   ttt atg gcc tcc ttt tcc agc act cct gaa aaa gaa aat gaa gac gaa      720
   Phe Met Ala Ser Phe Ser Ser Thr Pro Glu Lys Glu Asn Glu Asp Glu
   225                 230                 235                 240
   gct cta tat atc atg acc cga ttc gat ggt act tct atc att ggc ggt      768
   Ala Leu Tyr Ile Met Thr Arg Phe Asp Gly Thr Ser Ile Ile Gly Gly
                   245                 250                 255
   tgt ttc caa ccc aac aac tgg tca tcc gaa ccc gat cct tct ctc acc      816
   Cys Phe Gln Pro Asn Asn Trp Ser Ser Glu Pro Asp Pro Ser Leu Thr
               260                 265                 270
   cat cga atc ctg tct aga gcc ctc gac cga ttc ccg gaa ctg acc aaa      864
   His Arg Ile Leu Ser Arg Ala Leu Asp Arg Phe Pro Glu Leu Thr Lys
           275                 280                 285
   gat ggc cct ctt gac att gtg cgc gaa tgc gtt ggc cac cgt cct ggt      912
   Asp Gly Pro Leu Asp Ile Val Arg Glu Cys Val Gly His Arg Pro Gly
       290                 295                 300
   aga gag ggc ggt ccc cga gta gaa tta gag aag atc ccc ggc gtt ggc      960
   Arg Glu Gly Gly Pro Arg Val Glu Leu Glu Lys Ile Pro Gly Val Gly
   305                 310                 315                 320
   ttt gtt gtc cat aac tat ggt gcc gcc ggt gct ggt tac caa tcc tct     1008
   Phe Val Val His Asn Tyr Gly Ala Ala Gly Ala Gly Tyr Gln Ser Ser
                   325                 330                 335
   tac ggc atg gct gat gaa gct gtt tct tac gtc gaa aga gct ctt act     1056
   Tyr Gly Met Ala Asp Glu Ala Val Ser Tyr Val Glu Arg Ala Leu Thr
               340                 345                 350
   cgt cca aac ctt tag                                                 1071
   Arg Pro Asn Leu
           355
<210> 8
   <211> 356
   <212> PRT
   <213> Trigonopsis variabilis
<400> 8
   Met Ala Lys Ile Val Val Ile Gly Ala Gly Val Ala Gly Leu Thr Thr
   1               5                   10                  15
   Ala Leu Gln Leu Leu Arg Lys Gly His Glu Val Thr Ile Val Ser Glu
               20                  25                  30
   Phe Thr Pro Gly Asp Leu Ser Ile Gly Tyr Thr Ser Pro Trp Ala Gly
           35                  40                  45
   Ala Asn Trp Leu Thr Phe Tyr Asp Gly Gly Lys Leu Ala Asp Tyr Asp
       50                  55                  60
   Ala Val Ser Tyr Pro Ile Leu Arg Glu Leu Ala Arg Ser Ser Pro Glu
   65                  70                  75                  80
   Ala Gly Ile Arg Leu Ile Ser Gln Arg Ser His Val Leu Lys Arg Asp
                   85                  90                  95
   Leu Pro Lys Leu Glu Val Ala Met Ser Ala Ile Cys Gln Arg Asn Pro
   100 105 110
   Trp Phe Lys Asn Thr Val Asp Ser Phe Glu Ile Ile Glu Asp Arg Ser
   115 120 125
   Arg Ile Val His Asp Asp Val Ala Tyr Leu Val Glu Phe Arg Ser Val
   130 135 140
   Cys Ile His Thr Gly Val Tyr Leu Asn Trp Leu Met Ser Gln Cys Leu
   145 150 155 160
   Ser Leu Gly Ala Thr Val Val Lys Arg Arg Val Asn His Ile Lys Asp
   165 170 175
   Ala Asn Leu Leu His Ser Ser Gly Ser Arg Pro Asp Val Ile Val Asn
   180 185 190
   Cys Ser Gly Leu Phe Ala Arg Phe Leu Gly Gly Val Glu Asp Lys Lys
   195 200 205
   Met Tyr Pro Ile Arg Gly Gln Val Val Leu Val Arg Asn Ser Leu Pro
   210 215 220
   Phe Met Ala Ser Phe Ser Ser Thr Pro Glu Lys Glu Asn Glu Asp Glu
   225 230 235 240
   Ala Leu Tyr Ile Met Thr Arg Phe Asp Gly Thr Ser Ile Ile Gly Gly
   245 250 255
   Cys Phe Gln Pro Asn Asn Trp Ser Ser Glu Pro Asp Pro Ser Leu Thr
   260 265 270
   His Arg Ile Leu Ser Arg Ala Leu Asp Arg Phe Pro Glu Leu Thr Lys
   275 280 285
   Asp Gly Pro Leu Asp Ile Val Arg Glu Cys Val Gly His Arg Pro Gly
   290 295 300
   Arg Glu Gly Gly Pro Arg Val Glu Leu Glu Lys Ile Pro Gly Val Gly
   305 310 315 320
   Phe Val Val His Asn Tyr Gly Ala Ala Gly Ala Gly Tyr Gln Ser Ser
   325 330 335
   Tyr Gly Met Ala Asp Glu Ala Val Ser Tyr Val Glu Arg Ala Leu Thr
               340                 345                 350
   Arg Pro Asn Leu
           355
<210> 9
   <211> 1047
   <212> DNA
   <213> Schizosaccharomyces pombe
<220>
   <221> CDS
   <222> (22)..(1041)
   <223> coding for DAAO
<400> 9
   atgactaagg aaaataagcc a aga gat att gtc atc gtt ggc gct ggc gtt        51
                           Arg Asp Ile Val Ile Val Gly Ala Gly Val
                           1               5                   10
   att gga ttg acc act gct tgg att ctt tca gac ttg ggt ctt gct cct        99
   Ile Gly Leu Thr Thr Ala Trp Ile Leu Ser Asp Leu Gly Leu Ala Pro
                   15                  20                  25
   cgt att aag gtg att gcc aag tat acg cct gaa gat cgt tct gta gaa       147
   Arg Ile Lys Val Ile Ala Lys Tyr Thr Pro Glu Asp Arg Ser Val Glu
               30                  35                  40
   tac act tcc cct tgg gct ggc gca aat ttc tgt agc att tct gct act       195
   Tyr Thr Ser Pro Trp Ala Gly Ala Asn Phe Cys Ser Ile Ser Ala Thr
           45                  50                  55
   gat gac aat gct ttg cgc tgg gat aaa atc act tac cat cgt ttc gcc       243
   Asp Asp Asn Ala Leu Arg Trp Asp Lys Ile Thr Tyr His Arg Phe Ala
       60                  65                  70
   tac ttg gcg aaa act cgt cct gaa gca gga atc cgt ttt gct gat ctt       291
   Tyr Leu Ala Lys Thr Arg Pro Glu Ala Gly Ile Arg Phe Ala Asp Leu
   75                  80                  85                  90
   cga gaa ttg tgg gag tac gag ccg aaa cac gac aaa atc aga tcc tgg       339
   Arg Glu Leu Trp Glu Tyr Glu Pro Lys His Asp Lys Ile Arg Ser Trp
                   95                  100                 105
   aat acc tat gtc aga gat ttc aaa gtt atc cct gaa aaa gat ctt cca       387
   Asn Thr Tyr Val Arg Asp Phe Lys Val Ile Pro Glu Lys Asp Leu Pro
               110                 115                 120
   gga gaa tgt atc tac gga cat aag gcc acc acc ttt tta atc aac gct       435
   Gly Glu Cys Ile Tyr Gly His Lys Ala Thr Thr Phe Leu Ile Asn Ala
           125                 130                 135
   cct cat tac ttg aat tat atg tac aag ctg ctc att gaa gct ggc gtc       483
   Pro His Tyr Leu Asn Tyr Met Tyr Lys Leu Leu Ile Glu Ala Gly Val
       140                 145                 150
   gaa ttt gaa aag aaa gaa ttg agt cac atc aaa gag act gtc gaa gaa       531
   Glu Phe Glu Lys Lys Glu Leu Ser His Ile Lys Glu Thr Val Glu Glu
   155                 160                 165                 170
   act cca gaa gct tca gta gta ttt aat tgc act ggt ctc tgg gct tcc       579
   Thr Pro Glu Ala Ser Val Val Phe Asn Cys Thr Gly Leu Trp Ala Ser
                   175                 180                 185
   aaa ttg ggt ggc gtt gaa gac ccg gac gtt tat ccg act cgt gga cat       627
   Lys Leu Gly Gly Val Glu Asp Pro Asp Val Tyr Pro Thr Arg Gly His
               190                 195                 200
   gtt gtt ttg gtt aag gct cct cat gta aca gaa act cgc att ttg aat       675
   Val Val Leu Val Lys Ala Pro His Val Thr Glu Thr Arg Ile Leu Asn
           205                 210                 215
   ggc aag aac tct gat acc tat att att cct cgt ccc tta aat ggt gga       723
   Gly Lys Asn Ser Asp Thr Tyr Ile Ile Pro Arg Pro Leu Asn Gly Gly
       220                 225                 230
   gtc att tgc ggc ggt ttc atg caa cca gga aac tgg gat cgt gaa att       771
   Val Ile Cys Gly Gly Phe Met Gln Pro Gly Asn Trp Asp Arg Glu Ile
   235                 240                 245                 250
   cac cct gaa gac act ttg gat atc ctt aag aga aca tcg gct ttg atg       819
   His Pro Glu Asp Thr Leu Asp Ile Leu Lys Arg Thr Ser Ala Leu Met
                   255                 260                 265
   cca gaa ttg ttc cac ggc aag ggt ccg gag ggt gct gaa att att caa       867
   Pro Glu Leu Phe His Gly Lys Gly Pro Glu Gly Ala Glu Ile Ile Gln
               270                 275                 280
   gaa tgt gtc gga ttc cgt cct tct cga aag ggt ggt gcc cgc gta gag 915
   Glu Cys Val Gly Phe Arg Pro Ser Arg Lys Gly Gly Ala Arg Val Glu
   285 290 295
   ctt gat gtt gtt ccc ggc acc tca gtc ccc ctt gtt cat gat tac ggt 963
   Leu Asp Val Val Pro Gly Thr Ser Val Pro Leu Val His Asp Tyr Gly
   300 305 310
   gct tct ggc aca gga tac caa gct ggt tat ggt atg gct ctt gac tct 1011
   Ala Ser Gly Thr Gly Tyr Gln Ala Gly Tyr Gly Met Ala Leu Asp Ser
   315 320 325 330
   gtc atg ttg gct ctt cct aaa atc aaa ttg gcttag 1047
   Val Met Leu Ala Leu Pro Lys Ile Lys Leu
   335 340
<210> 10
   <211> 340
   <212> PRT
   <213> Schizosaccharomyces pombe
<400> 10
   Arg Asp Ile Val Ile Val Gly Ala Gly Val Ile Gly Leu Thr Thr Ala
   1 5 10 15
   Trp Ile Leu Ser Asp Leu Gly Leu Ala Pro Arg Ile Lys Val Ile Ala
   20 25 30
   Lys Tyr Thr Pro Glu Asp Arg Ser Val Glu Tyr Thr Ser Pro Trp Ala
   35 40 45
   Gly Ala Asn Phe Cys Ser Ile Ser Ala Thr Asp Asp Asn Ala Leu Arg
   50 55 60
   Trp Asp Lys Ile Thr Tyr His Arg Phe Ala Tyr Leu Ala Lys Thr Arg
   65 70 75 80
   Pro Glu Ala Gly Ile Arg Phe Ala Asp Leu Arg Glu Leu Trp Glu Tyr
   85 90 95
   Glu Pro Lys His Asp Lys Ile Arg Ser Trp Asn Thr Tyr Val Arg Asp
   100 105 110
   Phe Lys Val Ile Pro Glu Lys Asp Leu Pro Gly Glu Cys Ile Tyr Gly
           115                 120                 125
   His Lys Ala Thr Thr Phe Leu Ile Asn Ala Pro His Tyr Leu Asn Tyr
       130                 135                 140
   Met Tyr Lys Leu Leu Ile Glu Ala Gly Val Glu Phe Glu Lys Lys Glu
   145                 150                 155                 160
   Leu Ser His Ile Lys Glu Thr Val Glu Glu Thr Pro Glu Ala Ser Val
                   165                 170                 175
   Val Phe Asn Cys Thr Gly Leu Trp Ala Ser Lys Leu Gly Gly Val Glu
               180                 185                 190
   Asp Pro Asp Val Tyr Pro Thr Arg Gly His Val Val Leu Val Lys Ala
           195                 200                 205
   Pro His Val Thr Glu Thr Arg Ile Leu Asn Gly Lys Asn Ser Asp Thr
       210                 215                 220
   Tyr Ile Ile Pro Arg Pro Leu Asn Gly Gly Val Ile Cys Gly Gly Phe
   225                 230                 235                 240
   Met Gln Pro Gly Asn Trp Asp Arg Glu Ile His Pro Glu Asp Thr Leu
                   245                 250                 255
   Asp Ile Leu Lys Arg Thr Ser Ala Leu Met Pro Glu Leu Phe His Gly
               260                 265                 270
   Lys Gly Pro Glu Gly Ala Glu Ile Ile Gln Glu Cys Val Gly Phe Arg
           275                 280                 285
   Pro Ser Arg Lys Gly Gly Ala Arg Val Glu Leu Asp Val Val Pro Gly
       290                 295                 300
   Thr Ser Val Pro Leu Val His Asp Tyr Gly Ala Ser Gly Thr Gly Tyr
   305                 310                 315                 320
   Gln Ala Gly Tyr Gly Met Ala Leu Asp Ser Val Met Leu Ala Leu Pro
                   325                 330                 335
   Lys Ile Lys Leu
               340
<210> 11
   <211> 963
   <212> DNA
   <213> Streptomyces coelicolor
<220>
   <221> CDS
   <222> (31)..(957)
   <223> coding for DAAO
<220>
   <221> misc_feature
   <222> (880)..(936)
   <223> DAAO signature
<400> 11
   gtggaaaccg aactggatga cgagcgggat ggc gaa gtc gtc gtg gtc ggc ggc      54
                                    Gly Glu Val Val Val Val Gly Gly
                                    1               5
   ggg gtg atc ggg ctg acg acg gcc gtc gtc ctc gcc gag cgg ggc aga      102
   Gly Val Ile Gly Leu Thr Thr Ala Val Val Leu Ala Glu Arg Gly Arg
       10                  15                  20
   cgg gtg cgg ctg tgg acc cgg gag ccc gcg gag cgg acc acc tcg gtg      150
   Arg Val Arg Leu Trp Thr Arg Glu Pro Ala Glu Arg Thr Thr Ser Val
   25                  30                  35                  40
   gta gcg ggc ggg ctg tgg tgg ccg tac cgg atc gag ccg gtc gcg ctg      198
   Val Ala Gly Gly Leu Trp Trp Pro Tyr Arg Ile Glu Pro Val Ala Leu
                   45                  50                  55
   gcc cag gcc tgg gcg ctg cgt tcc ctg gac gtg tac gag gag ctg gcg      246
   Ala Gln Ala Trp Ala Leu Arg Ser Leu Asp Val Tyr Glu Glu Leu Ala
               60                  65                  70
   gca cgg ccc ggg cag acc ggc gta cgc atg ctc gaa ggg gtg ctc ggc      294
   Ala Arg Pro Gly Gln Thr Gly Val Arg Met Leu Glu Gly Val Leu Gly
           75                  80                  85
   gag acc ggc ctg gac gag gtg gac ggg tgg gcc gcg gcc cgg ctg ccg      342
   Glu Thr Gly Leu Asp Glu Val Asp Gly Trp Ala Ala Ala Arg Leu Pro
       90                  95                  100
   ggg ctg cgc gcg gcg agc gcc gcc gag tac gcc ggg acg ggg ctg tgg      390
   Gly Leu Arg Ala Ala Ser Ala Ala Glu Tyr Ala Gly Thr Gly Leu Trp
   105                 110                 115                 120
   gcg cgg ctg ccg ctc atc gac atg tcg acc cat ctg ccg tgg ctg cgg      438
   Ala Arg Leu Pro Leu Ile Asp Met Ser Thr His Leu Pro Trp Leu Arg
                   125                 130                 135
   gag cgg ctg ctg gcc gcg ggc ggc acg gtg gag gac cgc gcg gtg acc      486
   Glu Arg Leu Leu Ala Ala Gly Gly Thr Val Glu Asp Arg Ala Val Thr
               140                 145                 150
   gat ctg gcc gag gcg gac gcg ccg gtg gtg gtc aac tgc acc ggc ctg      534
   Asp Leu Ala Glu Ala Asp Ala Pro Val Val Val Asn Cys Thr Gly Leu
           155                 160                 165
   ggc gcc cgg gag ctg gtg ccg gac ccg gcg gta cgg ccg gtg cgc gga      582
   Gly Ala Arg Glu Leu Val Pro Asp Pro Ala Val Arg Pro Val Arg Gly
       170                 175                 180
   cag ctg gtc gtc gtg gag aac ccc ggc atc cac aac tgg ctg gtc gcg      630
   Gln Leu Val Val Val Glu Asn Pro Gly Ile His Asn Trp Leu Val Ala
   185                 190                 195                 200
   gcc gac gcg gac tcc ggg gag acg acg tac ttc ctt ccg cag ccg gga      678
   Ala Asp Ala Asp Ser Gly Glu Thr Thr Tyr Phe Leu Pro Gln Pro Gly
                   205                 210                 215
   cgg ctc ctg ctg ggc ggc acg gct gag gag gac gcc tgg tcg acc gag      726
   Arg Leu Leu Leu Gly Gly Thr Ala Glu Glu Asp Ala Trp Ser Thr Glu
               220                 225                 230
   ccg gac ccg gag gtc gcg gcg gcc atc gtg cga cgg tgc gcg gcc ctg      774
   Pro Asp Pro Glu Val Ala Ala Ala Ile Val Arg Arg Cys Ala Ala Leu
           235                 240                 245
   cgt ccc gag atc gcc gga gcg cgg gtg ctc gcg cac ctg gtg ggg ctg      822
   Arg Pro Glu Ile Ala Gly Ala Arg Val Leu Ala His Leu Val Gly Leu
       250                 255                 260
   cgg ccg gcc cgg gac gcg gtc cgg ctg gag cgc ggg acg ctg ccg gac      870
   Arg Pro Ala Arg Asp Ala Val Arg Leu Glu Arg Gly Thr Leu Pro Asp
   265                 270                 275                 280
   ggg cgc cgg ctg gtg cac aac tac ggt cac ggc ggc gcg ggc gtc acc      918
   Gly Arg Arg Leu Val His Asn Tyr Gly His Gly Gly Ala Gly Val Thr
   285 290 295
   gtg gcc tgg ggc tgc gct cag gag gcg gcc cgg ctc gcc tcctga 963
   Val Ala Trp Gly Cys Ala Gln Glu Ala Ala Arg Leu Ala
   300 305
<210> 12
   <211> 309
   <212> PRT
   <213> Streptomyces coelicolor
<220>
   <221> misc_feature
   <222> (880)..(936)
   <223> DAAO signature
<400> 12
   Gly Glu Val Val Val Val Gly Gly Gly Val Ile Gly Leu Thr Thr Ala
   1 5 10 15
   Val Val Leu Ala Glu Arg Gly Arg Arg Val Arg Leu Trp Thr Arg Glu
   20 25 30
   Pro Ala Glu Arg Thr Thr Ser Val Val Ala Gly Gly Leu Trp Trp Pro
   35 40 45
   Tyr Arg Ile Glu Pro Val Ala Leu Ala Gln Ala Trp Ala Leu Arg Ser
   50 55 60
   Leu Asp Val Tyr Glu Glu Leu Ala Ala Arg Pro Gly Gln Thr Gly Val
   65 70 75 80
   Arg Met Leu Glu Gly Val Leu Gly Glu Thr Gly Leu Asp Glu Val Asp
   85 90 95
   Gly Trp Ala Ala Ala Arg Leu Pro Gly Leu Arg Ala Ala Ser Ala Ala
   100 105 110
   Glu Tyr Ala Gly Thr Gly Leu Trp Ala Arg Leu Pro Leu Ile Asp Met
   115 120 125
   Ser Thr His Leu Pro Trp Leu Arg Glu Arg Leu Leu Ala Ala Gly Gly
   130 135 140
   Thr Val Glu Asp Arg Ala Val Thr Asp Leu Ala Glu Ala Asp Ala Pro
   145 150 155 160
   Val Val Val Asn Cys Thr Gly Leu Gly Ala Arg Glu Leu Val Pro Asp
   165 170 175
   Pro Ala Val Arg Pro Val Arg Gly Gln Leu Val Val Val Glu Asn Pro
   180 185 190
   Gly Ile His Asn Trp Leu Val Ala Ala Asp Ala Asp Ser Gly Glu Thr
   195 200 205
   Thr Tyr Phe Leu Pro Gln Pro Gly Arg Leu Leu Leu Gly Gly Thr Ala
   210 215 220
   Glu Glu Asp Ala Trp Ser Thr Glu Pro Asp Pro Glu Val Ala Ala Ala
   225 230 235 240
   Ile Val Arg Arg Cys Ala Ala Leu Arg Pro Glu Ile Ala Gly Ala Arg
   245 250 255
   Val Leu Ala His Leu Val Gly Leu Arg Pro Ala Arg Asp Ala Val Arg
   260 265 270
   Leu Glu Arg Gly Thr Leu Pro Asp Gly Arg Arg Leu Val His Asn Tyr
   275 280 285
   Gly His Gly Gly Ala Gly Val Thr Val Ala Trp Gly Cys Ala Gln Glu
   290 295 300
   Ala Ala Arg Leu Ala
   305
<210> 13
   <211> 1038
   <212> DNA
   <213> Candida boidinii
<220>
   <221> CDS
   <222> (1)..(1035)
   <223> coding for DAAO
<400> 13
   atg ggt gat caa att gtt gtt ctt ggt tcc ggt att att ggt tta tat     48
   Met Gly Asp Gln Ile Val Val Leu Gly Ser Gly Ile Ile Gly Leu Tyr
   1               5                   10                  15
   act aca tac tgt tta atc tat gag gct gga tgt gct cca gct aaa att     96
   Thr Thr Tyr Cys Leu Ile Tyr Glu Ala Gly Cys Ala Pro Ala Lys Ile
               20                  25                  30
   act att gtt gct gaa ttt tta cca ggt gat caa tct aca tta tat aca     144
   Thr Ile Val Ala Glu Phe Leu Pro Gly Asp Gln Ser Thr Leu Tyr Thr
           35                  40                  45
   tct cca tgg gca ggt ggt aat ttt tct tgt att tca cca gct gat gat     192
   Ser Pro Trp Ala Gly Gly Asn Phe Ser Cys Ile Ser Pro Ala Asp Asp
       50                  55                  60
   aca aca ttg gct tat gat aaa ttc aca tat ctt aat tta ttc aag att     240
   Thr Thr Leu Ala Tyr Asp Lys Phe Thr Tyr Leu Asn Leu Phe Lys Ile
   65                  70                  75                  80
   cac aaa aaa tta ggt gga cca gaa tgt gga tta gat aat aag cca agt     288
   His Lys Lys Leu Gly Gly Pro Glu Cys Gly Leu Asp Asn Lys Pro Ser
                   85                  90                  95
   act gaa tat tgg gat ttt tat cct ggt gat gaa aaa gtc aat tct tta     336
   Thr Glu Tyr Trp Asp Phe Tyr Pro Gly Asp Glu Lys Val Asn Ser Leu
               100                 105                 110
   aaa caa tat ctt aaa gat ttt aaa gtt att cca aaa tca gaa tta cca     384
   Lys Gln Tyr Leu Lys Asp Phe Lys Val Ile Pro Lys Ser Glu Leu Pro
           115                 120                 125
   gaa ggt gtt gaa tat ggt att agt tat act aca tgg aat ttc aac tgt     432
   Glu Gly Val Glu Tyr Gly Ile Ser Tyr Thr Thr Trp Asn Phe Asn Cys
       130                 135                 140
   cct gtt ttc tta caa aat atg gct aat ttt tta aat aaa aga aat gtt     480
   Pro Val Phe Leu Gln Asn Met Ala Asn Phe Leu Asn Lys Arg Asn Val
   145                 150                 155                 160
   acc att att aga aaa cat tta aca cat att tct caa gct tat tta aca     528
   Thr Ile Ile Arg Lys His Leu Thr His Ile Ser Gln Ala Tyr Leu Thr
                   165                 170                 175
   gtt aat aca aaa gtt gtt ttc aac tgt aca ggt att ggt gct gct gat      576
   Val Asn Thr Lys Val Val Phe Asn Cys Thr Gly Ile Gly Ala Ala Asp
               180                 185                 190
   tta ggt ggt gtt aaa gat gaa aaa gtt tat cca act aga gga caa gtt      624
   Leu Gly Gly Val Lys Asp Glu Lys Val Tyr Pro Thr Arg Gly Gln Val
           195                 200                 205
   gtt gtt gtt aga gct cca cat att caa gaa aat aaa atg aga tgg ggt      672
   Val Val Val Arg Ala Pro His Ile Gln Glu Asn Lys Met Arg Trp Gly
       210                 215                 220
   aaa gac tat gct act tat att att cca aga cca tat tct aat ggt gaa      720
   Lys Asp Tyr Ala Thr Tyr Ile Ile Pro Arg Pro Tyr Ser Asn Gly Glu
   225                 230                 235                 240
   tta gtc tta ggt ggt ttc tta caa aag gat aat tgg aca ggt aat act      768
   Leu Val Leu Gly Gly Phe Leu Gln Lys Asp Asn Trp Thr Gly Asn Thr
                   245                 250                 255
   ttt ggt ttt gaa act gat gat att gtt agt aga act aca tct tta tta      816
   Phe Gly Phe Glu Thr Asp Asp Ile Val Ser Arg Thr Thr Ser Leu Leu
               260                 265                 270
   cca aag att tta gat gaa cca ctt cat att att aga gtt gca gct ggt      864
   Pro Lys Ile Leu Asp Glu Pro Leu His Ile Ile Arg Val Ala Ala Gly
           275                 280                 285
   tta aga cca agt aga cat ggt ggt cca aga att gaa gct gaa gtt tgt      912
   Leu Arg Pro Ser Arg His Gly Gly Pro Arg Ile Glu Ala Glu Val Cys
       290                 295                 300
   gaa gaa ggt aaa tta act att cat aat tat ggt gct tct gga tat ggt      960
   Glu Glu Gly Lys Leu Thr Ile His Asn Tyr Gly Ala Ser Gly Tyr Gly
   305                 310                 315                 320
   tat caa gct ggt tat ggt atg tct tat gaa gct gtc aaa ctt tta gtt     1008
   Tyr Gln Ala Gly Tyr Gly Met Ser Tyr Glu Ala Val Lys Leu Leu Val
                   325                 330                 335
   gat aac caa aaa gtt aaa gct aaa ctt tag                             1038
   Asp Asn Gln Lys Val Lys Ala Lys Leu
               340                 345
<210> 14
   <211> 345
   <212> PRT
   <213> Candida boidinii
<400> 14
   Met Gly Asp Gln Ile Val Val Leu Gly Ser Gly Ile Ile Gly Leu Tyr
   1               5                   10                  15
   Thr Thr Tyr Cys Leu Ile Tyr Glu Ala Gly Cys Ala Pro Ala Lys Ile
               20                  25                  30
   Thr Ile Val Ala Glu Phe Leu Pro Gly Asp Gln Ser Thr Leu Tyr Thr
           35                  40                  45
   Ser Pro Trp Ala Gly Gly Asn Phe Ser Cys Ile Ser Pro Ala Asp Asp
       50                  55                  60
   Thr Thr Leu Ala Tyr Asp Lys Phe Thr Tyr Leu Asn Leu Phe Lys Ile
   65                  70                  75                  80
   His Lys Lys Leu Gly Gly Pro Glu Cys Gly Leu Asp Asn Lys Pro Ser
                   85                  90                  95
   Thr Glu Tyr Trp Asp Phe Tyr Pro Gly Asp Glu Lys Val Asn Ser Leu
               100                 105                 110
   Lys Gln Tyr Leu Lys Asp Phe Lys Val Ile Pro Lys Ser Glu Leu Pro
           115                 120                 125
   Glu Gly Val Glu Tyr Gly Ile Ser Tyr Thr Thr Trp Asn Phe Asn Cys
       130                 135                 140
   Pro Val Phe Leu Gln Asn Met Ala Asn Phe Leu Asn Lys Arg Asn Val
   145                 150                 155                 160
   Thr Ile Ile Arg Lys His Leu Thr His Ile Ser Gln Ala Tyr Leu Thr
                   165                 170                 175
   Val Asn Thr Lys Val Val Phe Asn Cys Thr Gly Ile Gly Ala Ala Asp
               180                 185                 190
   Leu Gly Gly Val Lys Asp Glu Lys Val Tyr Pro Thr Arg Gly Gln Val
           195                 200                 205
   Val Val Val Arg Ala Pro His Ile Gln Glu Asn Lys Met Arg Trp Gly
   210 215 220
   Lys Asp Tyr Ala Thr Tyr Ile Ile Pro Arg Pro Tyr Ser Asn Gly Glu
   225 230 235 240
   Leu Val Leu Gly Gly Phe Leu Gln Lys Asp Asn Trp Thr Gly Asn Thr
   245 250 255
   Phe Gly Phe Glu Thr Asp Asp Ile Val Ser Arg Thr Thr Ser Leu Leu
   260 265 270
   Pro Lys Ile Leu Asp Glu Pro Leu His Ile Ile Arg Val Ala Ala Gly
   275 280 285
   Leu Arg Pro Ser Arg His Gly Gly Pro Arg Ile Glu Ala Glu Val Cys
   290 295 300
   Glu Glu Gly Lys Leu Thr Ile His Asn Tyr Gly Ala Ser Gly Tyr Gly
   305 310 315 320
   Tyr Gln Ala Gly Tyr Gly Met Ser Tyr Glu Ala Val Lys Leu Leu Val
   325 330 335
   Asp Asn Gln Lys Val Lys Ala Lys Leu
   340 345
<210> 15
   <211> 12466
   <212> DNA
   <213> vector daaoSceITetON
<220>
   <221> misc_feature
   <222> (38)..(183)
   <223> Agrobacterium right border
<220>
   <221> misc_feature
   <222> (445)..(462)
   <223> recognition / cleavage site for I-SceI endonuclease
<220>
   <221> terminator
   <222> (196)..(400)
   <223> complementary: 35S terminator
<220>
   <221> misc_feature
   <222> (515)..(1222)
   <223> complementary: coding for I-SceI endonuclease
<220>
   <221> promoter
   <222> (1270)..(1660)
   <223> complementary: coding for pTOP10P teracyclin regulatable promoter
<220>
   <221> terminator
   <222> (1735)..(1990)
   <223> complementary: NOS. terminator
<220>
   <221> misc_feature
   <222> (2067)..(3173)
   <223> complementary: coding for Rhodotorula gracilis D-amino acid oxida se
<220>
   <221> promoter
   <222> (3217)..(5028)
   <223> complementary: Arabidopsis thaliana nitrilase I promoter
<220>
   <221> terminator
   <222> (5118)..(5343)
   <223> complementary: OCS terminator
<220>
   <221> misc_feature
   <222> (5418)..(6425)
   <223> complementary: coding for tetracyclin repressor rtTA
<220>
   <221> promoter
   <222> (6479)..(7341)
   <223> complementary: coding for Pisum sativum ptxA promoter
<220>
   <221> terminator
   <222> (7345)..(7549)
   <223> complementary: coding for 23S terminator (functioning as homology sequence)
<220>
   <221> misc_feature
   <222> (7618)..(7834)
   <223> Agrobacterium left border
<400> 15
   aatattcaaa caaacacata cagcgcgact tatcatggac atacaaatgg acgaacggat     60
   aaaccttttc acgccctttt aaatatccga ttattctaat aaacgctctt ttctcttagg    120
   tttacccgcc aatatatcct gtcaaacact gatagtttaa actgaaggcg ggaaacgaca    180
   atcagatctg gtacccggtc actggatttt ggttttagga attagaaatt ttattgatag    240
   aagtatttta caaatacaaa tacatactaa gggtttctta tatgctcaac acatgagcga    300
   aaccctataa gaaccctaat tcccttatct gggaactact cacacattat tctggagaaa    360
   aatagagaga gatagatttg tagagagaga ctggtgattt ttgcgccggg taccccaaac    420
   tgtctcacga cgttttgaac ccagattacc ctgttatccc tagtcgagcg gccgccagtg    480
   tgatggatat ctgcagaatt cgccctttta gatcttattt caggaaagtt tcggaggaga    540
   tagtgttcgg cagtttgtac atcatctgcg ggatcaggta cggtttgatc aggttgtaga    600
   agatcaggta agacatagaa tcgatgtaga tgatcggttt gtttttgttg atttttacgt    660
   aacagttcag ttggaatttg ttacgcagac ccttaaccag gtattctact tcttcgaaag    720
   tgaaagactg ggtgttcagt acgatcgatt tgttggtaga gtttttgttg taatcccatt    780
   taccaccatc atccatgaac cagtatgcca gagacatcgg ggtcaggtag ttttcaacca    840
   ggttgttcgg gatggttttt ttgttgttaa cgatgaacag gctagccagt ttgttgaaag    900
   cttggtgttt gaaagtctgg gcgccccagg tgattaccag gttacccagg tggttaacac    960
   gttctttttt gtgcggcggg gacagtaccc actgatcgta cagcagacat acgtggtcca   1020
   tgtatgcttt gtttttccac tcgaactgca tacagtaggt tttaccttca tcacgagaac   1080
   ggatgtaagc atcacccagg atcagaccga tacctgcttc gaactgttcg atgttcagtt   1140
   cgatcagctg ggatttgtat tctttcagca gtttagagtt cggacccagg ttcattacct   1200
   ggtttttttt gatgtttttc atatggtcga ctaaagggcg aattccagca cactggcggc   1260
   cgttactagc ccgggctcga gcaaatgtct agaaaggcct tatatacgta aagggtcttg   1320
   cgaagactag atcactctat ctcgagttta ccactcccta tcagtgatag agaaaagtga   1380
   aagtcgagtt taccactccc tatcagtgat agagaaaagt gaaagtcgag tttaccactc   1440
   cctatcagtg atagagaaaa gtgaaagtcg agtttaccac tccctatccg tgatagagaa   1500
   aagtgaaagt cgagtttacc actccctatc agtgatagag aaaagtgaaa gtcgagttta   1560
   ccactcccta tcagtgatag agaaaatgaa agtcgagttt accactccct atcagtgata   1620
   gagaaaagtg aaagtcgagc tcggtaccga gctcgaattc agcacactgg cggccgttac   1680
   tagtggatca attcactggc cgtcgtttta caacgactca gagcttgaca ggaggcccga   1740
   tctagtaaca tagatgacac cgcgcgcgat aatttatcct agtttgcgcg ctatattttg   1800
   ttttctatcg cgtattaaat gtataattgc gggactctaa tcataaaaac ccatctcata   1860
   aataacgtca tgcattacat gttaattatt acatgcttaa cgtaattcaa cagaaattat   1920
   atgataatca tcgcaagacc ggcaacagga ttcaatctta agaaacttta ttgccaaatg   1980
   tttgaacgat cggggatcat ccgggtctgt ggcgggaact ccacgaaaat atccgaacgc   2040
   agcaagatct agagcttggg tcccgcctac aacttcgact cccgcgccgc gccgtggtac   2100
   cgctggaacg cctcgtcgac gagctgcgcg acatcctccg ccgcgcccca actctgctgg   2160
   tatcccgcac tcgagaagcc atacgcatgc acaagcgtga cctccttctc cttcgccgct   2220
   cgtgcgctgc ccctgccgag cgagaggggc gactttgtcc ggtcgagagg caggacgatc   2280
   cgttctgcct caacgcgggg tccgcctcgt cgtgcaggtc gcaagccgac gttgtggcgg   2340
   aggacctcga tgccttcgat cgttccgtcg ctcgagatgg tcgggtcgag gcgcaagcag   2400
   tgcttgagga tccgctggac cgtctctggg ttgacagaca agtcccagtc tcccacgccg   2460
   tacgtcccgc cgcagatgac ttcgccacct ggtcggggaa tgatgtaggc gggagaagcg   2520
   gggtcggacg agtccatcgt gcatcgcttg catggggact tgacgaggac ggtttgcccg   2580
   cggattggct cggcggcttg gtcgtcgatg cccgcaatcg acttggcgcc aagtcccgta   2640
   gcgttgacca ccaaatccgc accgtcgaac gcctgctcaa gcgacgtaac ggtccgtctc   2700
   tcaaacgtcg cgccgagctt ctgcagctct cttgcaaggt actggcagta ctttggtgcg   2760
   tggacggaga gggtgtcgta ggttacgccg atagcgccag gtggacattc ggaagatggg   2820
   agggggcggt aatttggcgt gatgtccttg taccagtgcc cgagcaagcc gtcttcgttc   2880
   tgcgcgaacc gcctcgtccc cttgagccac atggcatggc ccgtcgggac caactcgacc   2940
   cacttcttga aagtcgattc ttcccatttt gcttgtcgag gaccgtctgt aagcgtcatg   3000
   aaaggcgtcc aattcgcgcc agcccatggt gaagcgaaag tctggctcga gacgtcctcc   3060
   ggcaagtcgc gcgcgagaat atgcacgctg tagcccttcc gagcgaggat gagggcgctg   3120
   ctcagaccga taacgcctga tccgaggaca acgacgcgct tctgcgagtg catgggccct   3180
   cgactagagt cgagatccga tatcgcccgg gctcgagtct ttgtttttta ctttggttca   3240
   tgacactcag agacttgaga gaagcaatat atagactttt ttttgttttt tttttgtggt   3300
   cacgtttatt ttcctattgg agacggtaac gaagatcgaa cctgtggtgg aaatgaaaca   3360
   aggtgggact agcccacgtg gtttcttttc tctgcattga tttgtttttg ttttttttgt   3420
   aaagttcaca tcaaacctac taataattga gaagaaaaat aaaatctatt gattgattaa   3480
   accagccgat gctttatgtc tgaatataaa aaagaagtga aaaccccgtt taagaattac   3540
   aacggtggtt tacaaagtat ttggacacaa taaatccaaa cgaaataaaa caaaatggag   3600
   aactaccaaa taaaaaacaa ataaaaaact taaaagaatt tattccattt tttttcccgt   3660
   agaatttatt cttttatgga ttccttaaat ccatatttga tgcattttga ttcctcataa   3720
   taggtaataa tatatactat gttatagata tgtttctaat tcgtattaac ctaccttttt   3780
   ttggtcgtac gattctacct aataatattg aacggaattg atgttttgga ccacttagaa   3840
   agtatttttt ttttggtttg tcttagctgt atttcattaa atataaattt aaataagaaa   3900
   tgtcataaat aaaatttgac gtatagattt tttaaatcca ttttatgtta tttaatattt   3960
   gaaatgtgag tttggctcct atttaatctt aggatgggtt aatactaagt tttccttaat   4020
   gaattatctc agagaaactg gattaaataa actaaaaaat agatcaatgt gttttggtcc   4080
   ggtcaaatat ctttggattt actattattg gcgaaaagaa agtctcatat agtaaatcat   4140
   attcctacaa gagaaatcaa aatttttgaa ttaacatgga ttgtatagtt tcttatataa   4200
   ccaattagtt cgcatcaaga aaaccaaacc ccaattaata atcaaacggg cttggtagga   4260
   atatttcatt gcagctttca gataaaagaa aaaaacacac actcaagtct tttatttcat   4320
   ctttcttact tgcaggaact caaattccac tttgccactt ttctttacaa ataaacacaa   4380
   attgtcaatg aaacgaaata gtctttttat gcaaacactg tttgtctttt ttcgatcacg   4440
   tttctgattg tgacagccat ccatatatat agggaatgta aaacaacaac atgtgaagtc   4500
   acatatacgt aatggtttag catagcttct attttcgttg tcaatattag tcattccaaa   4560
   acatttttaa gaaaaataaa ttaatatatg tatattcttg gaactaatgt atgtggaaat   4620
   acagtaactt aattattaaa cattctaaat gcaaatatgc aaagaaaaaa aagaaaagaa   4680
   cacaactgaa atcaaagcca gattcataat aattggctac atggttgtag aatgtagggt 4740
   aacacaacat ccagaattga acactcaaat tggatgatag atggataatc tttagataca 4800
   agagaattgg ttctcttcca ttattaacga aaataaagaa aaaaagttta gcataaaagt 4860
   ttgaaactca acataacatt ttgaacttga ctccttcata ggagtgacat gaactgacga 4920
   atcacaaccg attacttgtt tgagtcatct tccgctttct ccaccttcga aatgaatgtg 4980
   accggtttct tcgggtgctc atttacggtc aagtgtaaaa catctggtct cgaggtacct 5040
   ggtagggata acagggtaat ctgggttcaa aacgtcgtga gacagtttgg tgcaggtcga 5100
   aattcgagct cggtaccaat tcccatcttg aaagaaatat agtttaaata tttattgata 5160
   aaataacaag tcaggtatta tagtccaagc aaaaacataa atttattgat gcaagtttaa 5220
   attcagaaat atttcaataa ctgattatat cagctggtac attgccgtag atgaaagact 5280
   gagtgcgata ttatgtgtaa tacataaatt gatgatatag ctagcttagc tcatcggggg 5340
   atcttgcgcc gggtaccgag ctcggtagca attcccgagg ctgtagccga cgatggtgcg 5400
   ccaggagagt tgttgatcta cccaccgtac tcgtcaattc caagggcatc ggtaaacatc 5460
   tgctcaaact cgaagtcggc catatccaga gcgccgtagg gggcggagtc gtggggggta 5520
   aatcccggac ccggggaatc cccgtccccc aacatgtcca gatcgaaatc gtctagcgcg 5580
   tcggcatgcg ccatcgccac gtcctcgccg tctaagtgga gctcgtcccc caggctgaca 5640
   tcggtcgggg gggccgtcga cagtctgcgc gtgtgtcccg cggggagaaa ggacaggcgc 5700
   ggagccgcca gccccgcctc ttcgggggcg tcgtcgtccg ggagatcgag caggccctcg 5760
   atggtagacc cgtaattgtt tttcgtacgc gcgcggctgt acgcggaccc actttcacat 5820
   ttaagttgtt tttctaatcc gcatatgatc aattcaaggc cgaataagaa ggctggctct 5880
   gcaccttggt gatcaaataa ttcgatagct tgtcgtaata atggcggcat actatcagta 5940
   gtaggtgttt ccctttcttc tttagcgact tgatgctctt gatcttccaa tacgcaacct 6000
   aaagtaaaat gccccacagc gctgagtgca tataacgcgt tctctagtga aaaaccttgt 6060
   tggcataaaa aggctaattg attttcgaga gtttcatact gtttttctgt aggccgtgta 6120
   tctgaatgta cttttgctcc attgcgatga cttagtaaag cacatctaaa acttttagcg 6180
   ttattgcgta aaaaatcttg ccagctttcc ccttttaaag ggcaaaagtg agtatggtgc 6240
   ctatctaaca tctcaatggc taaggcgtcg agcaaagccc gcttattttt tacatgccaa 6300
   tacagtgtag gctgctctac accaagcttc tgggcgagtt tacgggttgt taaaccttcg 6360
   attccgacct cattaagcag ctctaatgcg ctgttaatca ctttactttt atctaatcta 6420
   gacatggtcg atcgactcta gactagtgga tccgatatcg cccgggctcg actctagagt 6480
   ttcgaagatt ttagtgtaat gtgtgtgctc actactatga agctttgcac ttaaaaaaat 6540
   agaatgagtg atgaggttta tatggtgaaa aaaactatga aattttgata ttttgatata 6600
   tctttctcgt gagtcatatt cacggaccat gttgcagcaa attggaatta aactattcat 6660
   tttttatgtt aaatcattga ttgattttta gtgggcctcg ttacatattc aagagttaga 6720
   atgaattcaa acaaactagg ccagaaaaaa ggatgtgggg ccattttttt gtgtcttaag 6780
   aatttgttta tttttttcat ggataagggg aatcaatgga aaaagtttga tgtactagag 6840
   gacatttttt taacatgtag tgacaagtag tgctattatt cgacccgtga tgaaaggggc 6900
   aatcttaatc tttttttcat aaatctgcac atgtgatgct ttaattatgc tttagacttt 6960
   gtgctaaact attggtaatt tctttttgta atcgaatcaa gtatctttta aactatgtat 7020
   gaaatgtgtc atcctaaaaa caacattttg ctagttttag actttgatgt ttatatgctt 7080
   aatggaagaa gcaatatgtt gatgtttatt gggtaaaaga aagggacttg attgagtatg 7140
   taattgacaa ctatgatttt atattggatt tgatattcct aacattaatt taagtgtgtg 7200
   ggtttcaaag catgttatgc tagtgattct tgtgtttgat gcttgaaaaa tctacattca 7260
   tccttgaatg gagggacaaa ctttgaatga cttttgaata ggtgtaaaat ccaatcctcc 7320
   ctcagcttca caaaaaattg cggacggtca ctggattttg gttttaggaa ttagaaattt 7380
   tattgataga agtattttac aaatacaaat acatactaag ggtttcttat atgctcaaca 7440
   catgagcgaa accctataag aaccctaatt cccttatctg ggaactactc acacattatt 7500
   ctggagaaaa atagagagag atagatttgt agagagagac tggtgatttt ccgggggatc 7560
   ctctagagtc gaggtaccga gctcgaattc actggccgtc gttttacaac gactcagtac 7620
   tgcttggtaa taattgtcat tagattgttt ttatgcatag atgcactcga aatcagccaa 7680
   ttttagacaa gtatcaaacg gatgttaatt cagtacatta aagacgtccg caatgtgtta 7740
   ttaagttgtc taagcgtcaa tttgtttaca ccacaatata tcctgccacc agccagccaa 7800
   cagctccccg accggcagct cggcacaaaa tcaccacgcg tctaaaaagg tgatgtgtat 7860
   ttgagtaaaa cagcttgcgt catgcggtcg ctgcgtatat gatgcgatga gtaaataaac 7920
   aaatacgcaa ggggaacgca tgaaggttat cgctgtactt aaccagaaag gcgggtcagg 7980
   caagacgacc atcgcaaccc atctagcccg cgccctgcaa ctcgccgggg ccgatgttct 8040
   gttagtcgat tccgatcccc agggcagtgc ccgcgattgg gcggccgtgc gggaagatca 8100
   accgctaacc gttgtcggca tcgaccgccc gacgattgac cgcgacgtga aggccatcgg 8160
   ccggcgcgac ttcgtagtga tcgacggagc gccccaggcg gcggacttgg ctgtgtccgc 8220
   gatcaaggca gccgacttcg tgctgattcc ggtgcagcca agcccttacg acatatgggc 8280
   caccgccgac ctggtggagc tggttaagca gcgcattgag gtcacggatg gaaggctaca 8340
   agcggccttt gtcgtgtcgc gggcgatcaa aggcacgcgc atcggcggtg aggttgccga 8400
   ggcgctggcc gggtacgagc tgcccattct tgagtcccgt atcacgcagc gcgtgagcta 8460
   cccaggcact gccgccgccg gcacaaccgt tcttgaatca gaacccgagg gcgacgctgc 8520
   ccgcgaggtc caggcgctgg ccgctgaaat taaatcaaaa ctcatttgag ttaatgaggt 8580
   aaagagaaaa tgagcaaaag cacaaacacg ctaagtgccg gccgtccgag cgcacgcagc 8640
   agcaaggctg caacgttggc cagcctggca gacacgccag ccatgaagcg ggtcaacttt 8700
   cagttgccgg cggaggatca caccaagctg aagatgtacg cggtacgcca aggcaagacc 8760
   attaccgagc tgctatctga atacatcgcg cagctaccag agtaaatgag caaatgaata 8820
   aatgagtaga tgaattttag cggctaaagg aggcggcatg gaaaatcaag aacaaccagg 8880
   caccgacgcc gtggaatgcc ccatgtgtgg aggaacgggc ggttggccag gcgtaagcgg 8940
   ctgggttgtc tgccggccct gcaatggcac tggaaccccc aagcccgagg aatcggcgtg 9000
   agcggtcgca aaccatccgg cccggtacaa atcggcgcgg cgctgggtga tgacctggtg 9060
   gagaagttga aggccgcgca ggccgcccag cggcaacgca tcgaggcaga agacgccccg 9120
   gtgaatcgtg gcaagggggc gctgatcgaa tccgcaaaga atcccggcaa ccgccggcag 9180
   ccggtgcgcc gtcgattagg aagccgccca agggcgacga gcaaccagat tttttcgttc 9240
   cgatgctcta tgacgtgggc acccgcgata gtcgcagcat catggacgtg gccgttttcc 9300
   gtctgtcgaa gcgtgaccga cgagctggcg aggtgatccg ctacgagctt ccagacgggc 9360
   acgtagaggt ttccgcaggg ccggccggca tggccagtgt gtgggattac gacctggtac 9420
   tgatggcggt ttcccatcta accgaatcca tgaaccgata ccgggaaggg aagggagaca 9480
   agcccggccg cgtgttccgt ccacacgttg cggacgtact caagttctgc cggcgagccg 9540
   atggcggaaa gcagaaagac gacctggtag aaacctgcat tcggttaaac accacgcacg 9600
   ttgccatgca gcgtacgaag aaggccaaga acggccgcct ggtgacggta tccgagggtg 9660
   aagccttgat tagccgctac aagatcgtaa agagcgaaac cgggcggccg gagtacatcg 9720
   agatcgagct agctgattgg atgtaccgcg agatcacaga aggcaagaac ccggacgtgc 9780
   tgacggttca ccccgattac tttttgatcg atcccggcat cggccgtttt ctctaccgcc 9840
   tggcacgccg cgccgcaggc aaggcagaag ccagatggtt gttcaagacg atctacgaac 9900
   gcagtggcag cgccggagag ttcaagaagt tctgtttcac cgtgcgcaag ctgatcgggt 9960
   caaatgacct gccggagtac gatttgaagg aggaggcggg gcaggctggc ccgatcctag 10020
   tcatgcgcta ccgcaacctg atcgagggcg aagcatccgc cggttcctaa tgtacggagc 10080
   agatgctagg gcaaattgcc ctagcagggg aaaaaggtcg aaaaggtctc tttcctgtgg   10140
   atagcacgta cattgggaac ccaaagccgt acattgggaa ccggaacccg tacattggga   10200
   acccaaagcc gtacattggg aaccggtcac acatgtaagt gactgatata aaagagaaaa   10260
   aaggcgattt ttccgcctaa aactctttaa aacttattaa aactcttaaa acccgcctgg   10320
   cctgtgcata actgtctggc cagcgcacag ccgaagagct gcaaaaagcg cctacccttc   10380
   ggtcgctgcg ctccctacgc cccgccgctt cgcgtcggcc tatcgcggcc tatgcggtgt   10440
   gaaataccgc acagatgcgt aaggagaaaa taccgcatca ggcgctcttc cgcttcctcg   10500
   ctcactgact cgctgcgctc ggtcgttcgg ctgcggcgag cggtatcagc tcactcaaag   10560
   gcggtaatac ggttatccac agaatcaggg gataacgcag gaaagaacat gtgagcaaaa   10620
   ggccagcaaa aggccaggaa ccgtaaaaag gccgcgttgc tggcgttttt ccataggctc   10680
   cgcccccctg acgagcatca caaaaatcga cgctcaagtc agaggtggcg aaacccgaca   10740
   ggactataaa gataccaggc gtttccccct ggaagctccc tcgtgcgctc tcctgttccg   10800
   accctgccgc ttaccggata cctgtccgcc tttctccctt cgggaagcgt ggcgctttct   10860
   catagctcac gctgtaggta tctcagttcg gtgtaggtcg ttcgctccaa gctgggctgt   10920
   gtgcacgaac cccccgttca gcccgaccgc tgcgccttat ccggtaacta tcgtcttgag   10980
   tccaacccgg taagacacga cttatcgcca ctggcagcag ccactggtaa caggattagc   11040
   agagcgaggt atgtaggcgg tgctacagag ttcttgaagt ggtggcctaa ctacggctac   11100
   actagaagga cagtatttgg tatctgcgct ctgctgaagc cagttacctt cggaaaaaga   11160
   gttggtagct cttgatccgg caaacaaacc accgctggta gcggtggttt ttttgtttgc   11220
   aagcagcaga ttacgcgcag aaaaaaagga tctcaagaag atcctttgat cttttctacg   11280
   gggtctgacg ctcagtggaa cgaaaactca cgttaaggga ttttggtcat gcatgatata   11340
   tctcccaatt tgtgtagggc ttattatgca cgcttaaaaa taataaaagc agacttgacc   11400
   tgatagtttg gctgtgagca attatgtgct tagtgcatct aacgcttgag ttaagccgcg   11460
   ccgcgaagcg gcgtcggctt gaacgaattt ctagctagac attatttgcc gactaccttg   11520
   gtgatctcgc ctttcacgta gtggacaaat tcttccaact gatctgcgcg cgaggccaag   11580
   cgatcttctt cttgtccaag ataagcctgt ctagcttcaa gtatgacggg ctgatactgg   11640
   gccggcaggc gctccattgc ccagtcggca gcgacatcct tcggcgcgat tttgccggtt   11700
   actgcgctgt accaaatgcg ggacaacgta agcactacat ttcgctcatc gccagcccag   11760
   tcgggcggcg agttccatag cgttaaggtt tcatttagcg cctcaaatag atcctgttca   11820
   ggaaccggat caaagagttc ctccgccgct ggacctacca aggcaacgct atgttctctt   11880
   gcttttgtca gcaagatagc cagatcaatg tcgatcgtgg ctggctcgaa gatacctgca   11940
   agaatgtcat tgcgctgcca ttctccaaat tgcagttcgc gcttagctgg ataacgccac   12000
   ggaatgatgt cgtcgtgcac aacaatggtg acttctacag cgcggagaat ctcgctctct   12060
   ccaggggaag ccgaagtttc caaaaggtcg ttgatcaaag ctcgccgcgt tgtttcatca   12120
   agccttacgg tcaccgtaac cagcaaatca atatcactgt gtggcttcag gccgccatcc   12180
   actgcggagc cgtacaaatg tacggccagc aacgtcggtt cgagatggcg ctcgatgacg   12240
   ccaactacct ctgatagttg agtcgatact tcggcgatca ccgcttcccc catgatgttt   12300
   aactttgttt tagggcgact gccctgctgc gtaacatcgt tgctgctcca taacatcaaa   12360
   catcgaccca cggcgtaacg cgcttgctgc ttggatgccc gaggcataga ctgtacccca   12420
   aaaaaacagt cataacaagc catgaaaacc gccactgcgt tccatg                   12466
<210> 16
   <211> 12539
   <212> DNA
   <213> daaoNit-PRecombination
<220>
   <221> misc_feature
   <222> (38)..(183)
   <223> Agrobacterium right border
<220>
   <221> terminator
   <222> (196)..(400)
   <223> complementary: 35S terminator
<220>
   <221> misc_feature
   <222> (445)..(462)
   <223> cleavage / recognition site for I-SceI endonuclease
<220>
   <221> terminator
   <222> (589)..(844)
   <223> complementary: nos terminator
<220>
   <221> misc_feature
   <222> (921)..(2027)
   <223> complementary: coding for Rhodotorula gracilis D-amino acid oxida se
<220>
   <221> promoter
   <222> (2071)..(3882)
   <223> complementary: A-thaliana nitrilase I promoter
<220>
   <221> terminator
   <222> (3972)..(4176)
   <223> complementary: 35S terminator
<220>
   <221> misc_feature
   <222> (4251)..(6248)
   <223> complementary: coding for beta-glucuronidase
<220>
   <221> promoter
   <222> (6302)..(7619)
   <223> complementar. coding for sTPT promoter
<220>
   <221> misc_feature
   <222> (7691)..(7907)
   <223> Agrobacterium left border
<400> 16
   aatattcaaa caaacacata cagcgcgact tatcatggac atacaaatgg acgaacggat      60
   aaaccttttc acgccctttt aaatatccga ttattctaat aaacgctctt ttctcttagg     120
   tttacccgcc aatatatcct gtcaaacact gatagtttaa actgaaggcg ggaaacgaca     180
   atcagatctg gtacccggtc actggatttt ggttttagga attagaaatt ttattgatag     240
   aagtatttta caaatacaaa tacatactaa gggtttctta tatgctcaac acatgagcga     300
   aaccctataa gaaccctaat tcccttatct gggaactact cacacattat tctggagaaa     360
   aatagagaga gatagatttg tagagagaga ctggtgattt ttgcgccggg taccccaaac     420
   tgtctcacga cgttttgaac ccagattacc ctgttatccc tagtcgagcg gccgccagtg     480
   tgatggatat ctgcagaatt cgccctttta gatcagcaca ctggcggccg ttactagtgg     540
   atcaattcac tggccgtcgt tttacaacga ctcagagctt gacaggaggc ccgatctagt     600
   aacatagatg acaccgcgcg cgataattta tcctagtttg cgcgctatat tttgttttct     660
   atcgcgtatt aaatgtataa ttgcgggact ctaatcataa aaacccatct cataaataac     720
   gtcatgcatt acatgttaat tattacatgc ttaacgtaat tcaacagaaa ttatatgata     780
   atcatcgcaa gaccggcaac aggattcaat cttaagaaac tttattgcca aatgtttgaa     840
   cgatcgggga tcatccgggt ctgtggcggg aactccacga aaatatccga acgcagcaag     900
   atctagagct tgggtcccgc ctacaacttc gactcccgcg ccgcgccgtg gtaccgctgg     960
   aacgcctcgt cgacgagctg cgcgacatcc tccgccgcgc cccaactctg ctggtatccc    1020
   gcactcgaga agccatacgc atgcacaagc gtgacctcct tctccttcgc cgctcgtgcg    1080
   ctgcccctgc cgagcgagag ggcgactttt gtccggtcga gaggcaggac gatccgttct    1140
   gcctcaacgc ggggtccgcc tcgtcgtgca ggtcgcaagc cgacgttgtg gcggaggacc    1200
   tcgatgcctt cgatcgttcc gtcgctcgag atggtcgggt cgaggcgcaa gcagtgcttg    1260
   aggatccgct ggaccgtctc tgggttgaca gacaagtccc agtctcccac gccgtacgtc    1320
   ccgccgcaga tgacttcgcc acctggtcgg ggaatgatgt aggcgggaga agcggggtcg    1380
   gacgagtcca tcgtgcatcg cttgcatggg gacttgacga ggacggtttg cccgcggatt    1440
   ggctcggcgg cttggtcgtc gatgcccgca atcgacttgg cgccaagtcc cgtagcgttg    1500
   accaccaaat ccgcaccgtc gaacgcctgc tcaagcgacg taacggtccg tctctcaaac    1560
   gtcgcgccga gcttctgcag ctctcttgca aggtactggc agtactttgg tgcgtggacg    1620
   gagagggtgt cgtaggttac gccgatagcg ccaggtggac attcggaaga tgggaggggg    1680
   cggtaatttg gcgtgatgtc cttgtaccag tgcccgagca agccgtcttc gttctgcgcg    1740
   aaccgcctcg tccccttgag ccacatggca tggcccgtcg ggaccaactc gacccacttc    1800
   ttgaaagtcg attcttccca ttttgcttgt cgaggaccgt ctgtaagcgt catgaaaggc    1860
   gtccaattcg cgccagccca tggtgaagcg aaagtctggc tcgagacgtc ctccggcaag    1920
   tcgcgcgcga gaatatgcac gctgtagccc ttccgagcga ggatgagggc gctgctcaga    1980
   ccgataacgc ctgatccgag gacaacgacg cgcttctgcg agtgcatggg ccctcgacta    2040
   gagtcgagat ccgatatcgc ccgggctcga gtctttgttt tttactttgg ttcatgacac 2100
   tcagagactt gagagaagca atatatagac ttttttttgt tttttttttg tggtcacgtt 2160
   tattttccta ttggagacgg taacgaagat cgaacctgtg gtggaaatga aacaaggtgg 2220
   gactagccca cgtggtttct tttctctgca ttgatttgtt tttgtttttt ttgtaaagtt 2280
   cacatcaaac ctactaataa ttgagaagaa aaataaaatc tattgattga ttaaaccagc 2340
   cgatgcttta tgtctgaata taaaaaagaa gtgaaaaccc cgtttaagaa ttacaacggt 2400
   ggtttacaaa gtatttggac acaataaatc caaacgaaat aaaacaaaat ggagaactac 2460
   caaataaaaa acaaataaaa aacttaaaag aatttattcc attttttttc ccgtagaatt 2520
   tattctttta tggattcctt aaatccatat ttgatgcatt ttgattcctc ataataggta 2580
   ataatatata ctatgttata gatatgtttc taattcgtat taacctacct ttttttggtc 2640
   gtacgattct acctaataat attgaacgga attgatgttt tggaccactt agaaagtatt 2700
   ttttttttgg tttgtcttag ctgtatttca ttaaatataa atttaaataa gaaatgtcat 2760
   aaataaaatt tgacgtatag attttttaaa tccattttat gttatttaat atttgaaatg 2820
   tgagtttggc tcctatttaa tcttaggatg ggttaatact aagttttcct taatgaatta 2880
   tctcagagaa actggattaa ataaactaaa aaatagatca atgtgttttg gtccggtcaa 2940
   atatctttgg atttactatt attggcgaaa agaaagtctc atatagtaaa tcatattcct 3000
   acaagagaaa tcaaaatttt tgaattaaca tggattgtat agtttcttat ataaccaatt 3060
   agttcgcatc aagaaaacca aaccccaatt aataatcaaa cgggcttggt aggaatattt 3120
   cattgcagct ttcagataaa agaaaaaaac acacactcaa gtcttttatt tcatctttct 3180
   tacttgcagg aactcaaatt ccactttgcc acttttcttt acaaataaac acaaattgtc 3240
   aatgaaacga aatagtcttt ttatgcaaac actgtttgtc ttttttcgat cacgtttctg 3300
   attgtgacag ccatccatat atatagggaa tgtaaaacaa caacatgtga agtcacatat 3360
   acgtaatggt ttagcatagc ttctattttc gttgtcaata ttagtcattc caaaacattt 3420
   ttaagaaaaa taaattaata tatgtatatt cttggaacta atgtatgtgg aaatacagta 3480
   acttaattat taaacattct aaatgcaaat atgcaaagaa aaaaaagaaa agaacacaac 3540
   tgaaatcaaa gccagattca taataattgg ctacatggtt gtagaatgta gggtaacaca 3600
   acatccagaa ttgaacactc aaattggatg atagatggat aatctttaga tacaagagaa 3660
   ttggttctct tccattatta acgaaaataa agaaaaaaag tttagcataa aagtttgaaa 3720
   ctcaacataa cattttgaac ttgactcctt cataggagtg acatgaactg acgaatcaca 3780
   accgattact tgtttgagtc atcttccgct ttctccacct tcgaaatgaa tgtgaccggt 3840
   ttcttcgggt gctcatttac ggtcaagtgt aaaacatctg gtctcgaggt acctggtagg 3900
   gataacaggg taatctgggt tcaaaacgtc gtgagacagt ttggtgcagg tcgaaattcg 3960
   agctcggtac ccggtcactg gattttggtt ttaggaatta gaaattttat tgatagaagt 4020
   attttacaaa tacaaataca tactaagggt ttcttatatg ctcaacacat gagcgaaacc 4080
   ctataagaac cctaattccc ttatctggga actactcaca cattattctg gagaaaaata 4140
   gagagagata gatttgtaga gagagactgg tgatttttgc gccgggtacc gagctcggta 4200
   gcaattcccg aggctgtagc cgacgatggt gcgccaggag agttgttgat tcattgtttg 4260
   cctccctgct gcggtttttc accgaagttc atgccagtcc agcgtttttg cagcagaaaa 4320
   gccgccgact tcggtttgcg gtcgcgagtg aagatccctt tcttgttacc gccaacgcgc 4380
   aatatgcctt gcgaggtcgc aaaatcggcg aaattccata cctgttcacc gacgacggcg 4440
   ctgacgcgat caaagacgcg gtgatacata tccagccatg cacactgata ctcttcactc 4500
   cacatgtcgg tgtacattga gtgcagcccg gctaacgtat ccacgccgta ttcggtgatg 4560
   ataatcggct gatgcagttt ctcctgccag gccagaagtt ctttttccag taccttctct 4620
   gccgtttcca aatcgccgct ttggacatac catccgtaat aacggttcag gcacagcaca 4680
   tcaaagagat cgctgatggt atcggtgtga gcgtcgcaga acattacatt gacgcaggtg 4740
   atcggacgcg tcgggtcgag tttacgcgtt gcttccgcca gtggcgaaat attcccgtgc 4800
   acttgcggac gggtatccgg ttcgttggca atactccaca tcaccacgct tgggtggttt 4860
   ttgtcacgcg ctatcagctc tttaatcgcc tgtaagtgcg cttgctgagt ttccccgttg 4920
   actgcctctt cgctgtacag ttctttcggc ttgttgcccg cttcgaaacc aatgcctaaa 4980
   gagaggttaa agccgacagc agcagtttca tcaatcacca cgatgccatg ttcatctgcc 5040
   cagtcgagca tctcttcagc gtaagggtaa tgcgaggtac ggtaggagtt ggccccaatc 5100
   cagtccatta atgcgtggtc gtgcaccatc agcacgttat cgaatccttt gccacgtaag 5160
   tccgcatctt catgacgacc aaagccagta aagtagaacg gtttgtggtt aatcaggaac 5220
   tgttcgccct tcactgccac tgaccggatg ccgacgcgaa gcgggtagat atcacactct 5280
   gtctggcttt tggctgtgac gcacagttca tagagataac cttcacccgg ttgccagagg 5340
   tgcggattca ccacttgcaa agtcccgcta gtgccttgtc cagttgcaac cacctgttga 5400
   tccgcatcac gcagttcaac gctgacatca ccattggcca ccacctgcca gtcaacagac 5460
   gcgtggttac agtcttgcgc gacatgcgtc accacggtga tatcgtccac ccaggtgttc 5520
   ggcgtggtgt agagcattac gctgcgatgg attccggcat agttaaagaa atcatggaag 5580
   taagactgct ttttcttgcc gttttcgtcg gtaatcacca ttcccggcgg gatagtctgc 5640
   cagttcagtt cgttgttcac acaaacggtg atacctgcac atcaacaaat tttggtcata 5700
   tattagaaaa gttataaatt aaaatataca cacttataaa ctacagaaaa gcaattgcta 5760
   tatactacat tcttttattt tgaaaaaaat atttgaaata ttatattact actaattaat 5820
   gataattatt atatatatat caaaggtaga agcagaaact tacgtacact tttcccggca 5880
   ataacatacg gcgtgacatc ggcttcaaat ggcgtatagc cgccctgatg ctccatcact 5940
   tcctgattat tgacccacac tttgccgtaa tgagtgaccg catcgaaacg cagcacgata 6000
   cgctggcctg cccaaccttt cggtataaag acttcgcgct gataccagac gttgcccgca 6060
   taattacgaa tatctgcatc ggcgaactga tcgttaaaac tgcctggcac agcaattgcc 6120
   cggctttctt gtaacgcgct ttcccaccaa cgctgaccaa ttccacagtt ttcgcgatcc 6180
   agactgaatg cccacaggcc gtcgagtttt ttgatttcac gggttggggt ttctacagga 6240
   cgtaccatgg tcgatcgact ctagactagt ggatccgata tcgcccgggc tcgactctag 6300
   atgaaatcga aattcagagt tttgatagtg agagcaaaga gggacggact tatgaggatt 6360
   tcgagtattt caagagatgg tacttgttga tcggacggct acgatgatct cgatttggtt 6420
   aatccagtat ctcgcggtgt atggagttat ggtagggtta atggtcaatt tcatctaacg 6480
   gtagagaatg atgtaattag ataagaatct tgagatactg gtttagattg gatgagtgta 6540
   gggtccatct tatcttgata agtggatggt ttttagagac acagtgaata ttagccaatc 6600
   gaagttccat atcaccatca tcatctgtat aattttgttt ttttggaaga taataatgat 6660
   tgaaattttg gtagatttta tttttcatta tttaccttgt atgttgagtg gtcttcaaat 6720
   tattgaacgt gacagattca caagaaagta gattttttat aaatgaaatt ttacttattt 6780
   taaaggtatc tctatttaat ttcttttgtt tatggttgtc tgtcagcatt tgacttgcag 6840
   tttcatgctc atagtcatat acgttattct aggctttttt gaatatctta ttactttttt 6900
   cgtaatacaa ttttataatt ttatcaaagt tatacaacta taactaaaat tagggttttc 6960
   tacaaaacaa aaaaatcttc taattttttt tgttgtagcc agtttactcg taagttacaa 7020
   aaaaatacaa atgaacccac atgtattatg cgtttaacta ggattaccat gtactttcat 7080
   gtactcaatt caccctatac tctttttttt tttttttcta gttccaccca atctataaaa 7140
   ttctgtccat ttgaccaaat tcaattaatt tctgtaattg cgatttaaaa ttaatattac 7200
   atgttcacta tttctcgatt tgagggaacc cgagtttaaa tatgataaaa atgttgaccc 7260
   atcactacaa atatgttata gtttatactt aatagtggtg tttttgggga taattgatga 7320
   attaagtaaa catgattctt cttatgaagt tgattgagtg attattgtat gtaaacctat 7380
   gtgattgatg ttattggttg attgagtgat tattgtatta gtatgtaagc aaagatgatt 7440
   gttcttatga ggtaatttgt tactcattca tccttttgca tatgagaaat tgtgttagcg 7500
   tacgcaaaac aatagagaac ataaaagata tgtgtattta tttaaggtga cttttgttaa 7560
   tgatattgta gtatctatac atttatatat aacttgttga atttgagtat aagctatcag 7620
   gatccggggg atcctctaga gtcgaggtac cgagctcgaa ttcactggcc gtcgttttac 7680
   aacgactcag tactgcttgg taataattgt cattagattg tttttatgca tagatgcact 7740
   cgaaatcagc caattttaga caagtatcaa acggatgtta attcagtaca ttaaagacgt 7800
   ccgcaatgtg ttattaagtt gtctaagcgt caatttgttt acaccacaat atatcctgcc 7860
   accagccagc caacagctcc ccgaccggca gctcggcaca aaatcaccac gcgtctaaaa 7920
   aggtgatgtg tatttgagta aaacagcttg cgtcatgcgg tcgctgcgta tatgatgcga 7980
   tgagtaaata aacaaatacg caaggggaac gcatgaaggt tatcgctgta cttaaccaga 8040
   aaggcgggtc aggcaagacg accatcgcaa cccatctagc ccgcgccctg caactcgccg 8100
   gggccgatgt tctgttagtc gattccgatc cccagggcag tgcccgcgat tgggcggccg 8160
   tgcgggaaga tcaaccgcta accgttgtcg gcatcgaccg cccgacgatt gaccgcgacg 8220
   tgaaggccat cggccggcgc gacttcgtag tgatcgacgg agcgccccag gcggcggact 8280
   tggctgtgtc cgcgatcaag gcagccgact tcgtgctgat tccggtgcag ccaagccctt 8340
   acgacatatg ggccaccgcc gacctggtgg agctggttaa gcagcgcatt gaggtcacgg 8400
   atggaaggct acaagcggcc tttgtcgtgt cgcgggcgat caaaggcacg cgcatcggcg 8460
   gtgaggttgc cgaggcgctg gccgggtacg agctgcccat tcttgagtcc cgtatcacgc 8520
   agcgcgtgag ctacccaggc actgccgccg ccggcacaac cgttcttgaa tcagaacccg 8580
   agggcgacgc tgcccgcgag gtccaggcgc tggccgctga aattaaatca aaactcattt 8640
   gagttaatga ggtaaagaga aaatgagcaa aagcacaaac acgctaagtg ccggccgtcc 8700
   gagcgcacgc agcagcaagg ctgcaacgtt ggccagcctg gcagacacgc cagccatgaa 8760
   gcgggtcaac tttcagttgc cggcggagga tcacaccaag ctgaagatgt acgcggtacg 8820
   ccaaggcaag accattaccg agctgctatc tgaatacatc gcgcagctac cagagtaaat 8880
   gagcaaatga ataaatgagt agatgaattt tagcggctaa aggaggcggc atggaaaatc 8940
   aagaacaacc aggcaccgac gccgtggaat gccccatgtg tggaggaacg ggcggttggc 9000
   caggcgtaag cggctgggtt gtctgccggc cctgcaatgg cactggaacc cccaagcccg 9060
   aggaatcggc gtgagcggtc gcaaaccatc cggcccggta caaatcggcg cggcgctggg 9120
   tgatgacctg gtggagaagt tgaaggccgc gcaggccgcc cagcggcaac gcatcgaggc 9180
   agaagacgcc ccggtgaatc gtggcaaggg gccgctgatc gaatccgcaa agaatcccgg 9240
   caaccgccgg cagccggtgc gccgtcgatt aggaagccgc ccaagggcga cgagcaacca 9300
   gattttttcg ttccgatgct ctatgacgtg ggcacccgcg atagtcgcag catcatggac 9360
   gtggccgttt tccgtctgtc gaagcgtgac cgacgagctg gcgaggtgat ccgctacgag 9420
   cttccagacg ggcacgtaga ggtttccgca gggccggccg gcatggccag tgtgtgggat 9480
   tacgacctgg tactgatggc ggtttcccat ctaaccgaat ccatgaaccg ataccgggaa 9540
   gggaagggag acaagcccgg ccgcgtgttc cgtccacacg ttgcggacgt actcaagttc 9600
   tgccggcgag ccgatggcgg aaagcagaaa gacgacctgg tagaaacctg cattcggtta 9660
   aacaccacgc acgttgccat gcagcgtacg aagaaggcca agaacggccg cctggtgacg 9720
   gtatccgagg gtgaagcctt gattagccgc tacaagatcg taaagagcga aaccgggcgg 9780
   ccggagtaca tcgagatcga gctagctgat tggatgtacc gcgagatcac agaaggcaag 9840
   aacccggacg tgctgacggt tcaccccgat tactttttga tcgatcccgg catcggccgt 9900
   tttctctacc gcctggcacg ccgcgccgca ggcaaggcag aagccagatg gttgttcaag 9960
   acgatctacg aacgcagtgg cagcgccgga gagttcaaga agttctgttt caccgtgcgc 10020
   aagctgatcg ggtcaaatga cctgccggag tacgatttga aggaggaggc ggggcaggct 10080
   ggcccgatcc tagtcatgcg ctaccgcaac ctgatcgagg gcgaagcatc cgccggttcc 10140
   taatgtacgg agcagatgct agggcaaatt gccctagcag gggaaaaagg tcgaaaaggt  10200
   ctctttcctg tggatagcac gtacattggg aacccaaagc cgtacattgg gaaccggaac  10260
   ccgtacattg ggaacccaaa gccgtacatt gggaaccggt cacacatgta agtgactgat  10320
   ataaaagaga aaaaaggcga tttttccgcc taaaactctt taaaacttat taaaactctt  10380
   aaaacccgcc tggcctgtgc ataactgtct ggccagcgca cagccgaaga gctgcaaaaa  10440
   gcgcctaccc ttcggtcgct gcgctcccta cgccccgccg cttcgcgtcg gcctatcgcg  10500
   gcctatgcgg tgtgaaatac cgcacagatg cgtaaggaga aaataccgca tcaggcgctc  10560
   ttccgcttcc tcgctcactg actcgctgcg ctcggtcgtt cggctgcggc gagcggtatc  10620
   agctcactca aaggcggtaa tacggttatc cacagaatca ggggataacg caggaaagaa  10680
   catgtgagca aaaggccagc aaaaggccag gaaccgtaaa aaggccgcgt tgctggcgtt  10740
   tttccatagg ctccgccccc ctgacgagca tcacaaaaat cgacgctcaa gtcagaggtg  10800
   gcgaaacccg acaggactat aaagatacca ggcgtttccc cctggaagct ccctcgtgcg  10860
   ctctcctgtt ccgaccctgc cgcttaccgg atacctgtcc gcctttctcc cttcgggaag  10920
   cgtggcgctt tctcatagct cacgctgtag gtatctcagt tcggtgtagg tcgttcgctc  10980
   caagctgggc tgtgtgcacg aaccccccgt tcagcccgac cgctgcgcct tatccggtaa  11040
   ctatcgtctt gagtccaacc cggtaagaca cgacttatcg ccactggcag cagccactgg  11100
   taacaggatt agcagagcga ggtatgtagg cggtgctaca gagttcttga agtggtggcc  11160
   taactacggc tacactagaa ggacagtatt tggtatctgc gctctgctga agccagttac  11220
   cttcggaaaa agagttggta gctcttgatc cggcaaacaa accaccgctg gtagcggtgg  11280
   tttttttgtt tgcaagcagc agattacgcg cagaaaaaaa ggatctcaag aagatccttt  11340
   gatcttttct acggggtctg acgctcagtg gaacgaaaac tcacgttaag ggattttggt  11400
   catgcatgat atatctccca atttgtgtag ggcttattat gcacgcttaa aaataataaa  11460
   agcagacttg acctgatagt ttggctgtga gcaattatgt gcttagtgca tctaacgctt  11520
   gagttaagcc gcgccgcgaa gcggcgtcgg cttgaacgaa tttctagcta gacattattt  11580
   gccgactacc ttggtgatct cgcctttcac gtagtggaca aattcttcca actgatctgc  11640
   gcgcgaggcc aagcgatctt cttcttgtcc aagataagcc tgtctagctt caagtatgac  11700
   gggctgatac tgggccggca ggcgctccat tgcccagtcg gcagcgacat ccttcggcgc  11760
   gattttgccg gttactgcgc tgtaccaaat gcgggacaac gtaagcacta catttcgctc  11820
   atcgccagcc cagtcgggcg gcgagttcca tagcgttaag gtttcattta gcgcctcaaa  11880
   tagatcctgt tcaggaaccg gatcaaagag ttcctccgcc gctggaccta ccaaggcaac  11940
   gctatgttct cttgcttttg tcagcaagat agccagatca atgtcgatcg tggctggctc  12000
   gaagatacct gcaagaatgt cattgcgctg ccattctcca aattgcagtt cgcgcttagc  12060
   tggataacgc cacggaatga tgtcgtcgtg cacaacaatg gtgacttcta cagcgcggag  12120
   aatctcgctc tctccagggg aagccgaagt ttccaaaagg tcgttgatca aagctcgccg  12180
   cgttgtttca tcaagcctta cggtcaccgt aaccagcaaa tcaatatcac tgtgtggctt  12240
   caggccgcca tccactgcgg agccgtacaa atgtacggcc agcaacgtcg gttcgagatg  12300
   gcgctcgatg acgccaacta cctctgatag ttgagtcgat acttcggcga tcaccgcttc  12360
   ccccatgatg tttaactttg ttttagggcg actgccctgc tgcgtaacat cgttgctgct  12420
   ccataacatc aaacatcgac ccacggcgta acgcgcttgc tgcttggatg cccgaggcat  12480
   agactgtacc ccaaaaaaac agtcataaca agccatgaaa accgccactg cgttccatg   12539